# EUROPEAN PATENT SPECIFICATION

(11) **EP 2 062 980 B1**
(45) Date of publication and mention of the grant of the patent: **31.08.2011**
(21) Application number: 09003559.3
(22) Date of filing: 28.06.2006
(51) Int. Cl.: C12Q 1/68, C12N 15/11

(54) **Methods and sequences to preferentially suppress expression of mutated huntingtin gene.**
Verfahren und Nukleotid-Sequenzen, die bevorzugt die Expression eines mutierten Huntingtin-Genes unterdrücken
Méthodes et séquences pour supprimer préférentiellement l'expression du gène huntingtin muté.

(30) Priority: 28.06.2005 US 695078 P
(43) Date of publication of application: 27.05.2009
(62) Divisional of application: 06785990.0
(73) Proprietor: MEDTRONIC, INC., Minneapolis, MN 55432-5604 (US)
(72) Inventor: Van Bilsen, Paul H. j., 6223 HV Maastrich XX (NL); Jaspers, Leonie, 6218 TS Maastrich XX (NL)
(74) Representative: Grünecker, Kinkeldey, Stockmair & Schwanhäusser Anwaltssozietät

(56) References cited:
- WO-A1-2005/027980
- EUN A J ET AL: "Molecular beacons: a new approach to plant virus detection." PHYTOPATHOLOGY MAR 2000, vol. 90, no. 3, March 2000 (2000-03), pages 269-275, XP002522349 ISSN: 0031-949X
- DATABASE SNP [Online] 13 July 2000 (2000-07-13), XP002522351 retrieved from NCBI Database accession no. ss1556778
- DATABASE SNP [Online] 20 August 2004 (2004-08-20), XP002522352 retrieved from NCBI Database accession no. ss23893221
- DATABASE SNP [Online] 6 September 2000 (2000-09-06), XP002522353 retrieved from NCBI Database accession no. ss1303522
- VAN BILSEN P H J ET AL: "Identification and allele-specific silencing of the mutant huntingtin allele in Huntington's disease patient-derived fibroblasts." HUMAN GENE THERAPY JUL 2008, vol. 19, no. 7, July 2008 (2008-07), pages 710-719, XP002522350 ISSN: 1557-7422

### SEQUENCE LISTING

<110> Medtronic, Inc.
<120> Methods and Sequences to preferentially Suppress Expression of Mutated Huntingtin
<130> EP55724IFzapu
<140> not yet assigned
   <141> herewith
<150> 06 785 990.0
   <151> 2006-06-28
<150> PCT/uS2006/025638
   <151> 2006-06-28
<150> 60/695078
   <151> 2005-06-28
<160> 150
<170> PatentIn version 3.4
<210> 1
   <211> 19
   <212> DNA
   <213> Homo sapiens
<400> 1
   aggccttcat agcgaacct 19
<210> 2
   <211> 19
   <212> DNA
   <213> Homo sapiens
<400> 2
   aggccttcac agcgaacct 19
<210> 3
   <211> 19
   <212> DNA
   <213> Homo sapiens
<400> 3
   aaatgtgctg ttaggctta 19
<210> 4
   <211> 19
   <212> DNA
   <213> Homo sapiens
<400> 4
   aaatgtgctc ttaggctta 19
<210> 5
   <211> 19
   <212> DNA
   <213> Homo sapiens
<400> 5
   caataaagga agccttgcc 19
<210> 6
   <211> 19
   <212> DNA
   <213> Homo sapiens
<400> 6
   caataaaggc agccttgcc 19
<210> 7
   <211> 19
   <212> DNA
   <213> Homo sapiens
<400> 7
   agggtttctc cgctcagcc 19
<210> 8
   <211> 19
   <212> DNA
   <213> Homo sapiens
<400> 8
   agggtttctt cgctcagcc 19
<210> 9
   <211> 19
   <212> DNA
   <213> Homo sapiens
<400> 9
   gagcaggaga acgacacct 19
<210> 10
   <211> 19
   <212> DNA
   <213> Homo sapiens
<400> 10
   gagcaggagc acgacacct 19
<210> 11
   <211> 19
   <212> DNA
   <213> Homo sapiens
<400> 11
   taagaggaac aaataaagc 19
<210> 12
   <211> 19
   <212> DNA
   <213> Homo sapiens
<400> 12
   taagaggaat aaataaagc 19
<210> 13
   <211> 19
   <212> DNA
   <213> Homo sapiens
<400> 13
   gggacagtac ttcaacgct 19
<210> 14
   <211> 19
   <212> DNA
   <213> Homo sapiens
<400> 14
   gggacagtaa ttcaacgct 19
<210> 15
   <211> 19
   <212> DNA
   <213> Homo sapiens
<400> 15
   tccctcatct actgtgtgc 19
<210> 16
   <211> 19
   <212> DNA
   <213> Homo sapiens
<400> 16
   tccctcatcc actgtgtgc 19
<210> 17
   <211> 19
   <212> DNA
   <213> Homo sapiens
<400> 17
   ccgcatcaac acactaggc 19
<210> 18
   <211> 19
   <212> DNA
   <213> Homo sapiens
<400> 18
   ccgcatcaat acactaggc 19
<210> 19
   <211> 19
   <212> DNA
   <213> Homo sapiens
<400> 19
   ctggtgacgc agcccctcg 19
<210> 20
   <211> 19
   <212> DNA
   <213> Homo sapiens
<400> 20
   ctggtgacgt agcccctcg 19
<210> 21
   <211> 19
   <212> DNA
   <213> Homo sapiens
<400> 21
   aagcccatat caccggctg 19
<210> 22
   <211> 19
   <212> DNA
   <213> Homo sapiens
<400> 22
   aagcccatac caccggctg 19
<210> 23
   <211> 19
   <212> DNA
   <213> Homo sapiens
<400> 23
   ggccttcata gcgaacctg 19
<210> 24
   <211> 19
   <212> DNA
   <213> Homo sapiens
<400> 24
   gccttcatag cgaacctga 19
<210> 25
   <211> 19
   <212> DNA
   <213> Homo sapiens
<400> 25
   aaggccttca tagcgaacc 19
<210> 26
   <211> 19
   <212> DNA
   <213> Homo sapiens
<400> 26
   aaaggccttc atagcgaac 19
<210> 27
   <211> 19
   <212> DNA
   <213> Homo sapiens
<400> 27
   ggccttcaca gcgaacctg 19
<210> 28
   <211> 19
   <212> DNA
   <213> Homo sapiens
<400> 28
   gccttcacag cgaacctga 19
<210> 29
   <211> 19
   <212> DNA
   <213> Homo sapiens
<400> 29
   aaggccttca cagcgaacc 19
<210> 30
   <211> 19
   <212> DNA
   <213> Homo sapiens
<400> 30
   aaaggccttc acagcgaac 19
<210> 31
   <211> 19
   <212> DNA
   <213> Homo sapiens
<400> 31
   taaatgtgct gttaggctt 19
<210> 32
   <211> 19
   <212> DNA
   <213> Homo sapiens
<400> 32
   ctaaatgtgc tgttaggct 19
<210> 33
   <211> 19
   <212> DNA
   <213> Homo sapiens
<400> 33
   aatgtgctgt taggcttac 19
<210> 34
   <211> 19
   <212> DNA
   <213> Homo sapiens
<400> 34
   atgtgctgtt aggcttact 19
<210> 35
   <211> 19
   <212> DNA
   <213> Homo sapiens
<400> 35
   taaatgtgct cttaggctt 19
<210> 36
   <211> 19
   <212> DNA
   <213> Homo sapiens
<400> 36
   ctaaatgtgc tcttaggct 19
<210> 37
   <211> 19
   <212> DNA
   <213> Homo sapiens
<400> 37
   aatgtgctct taggcttac 19
<210> 38
   <211> 19
   <212> DNA
   <213> Homo sapiens
<400> 38
   atgtgctctt aggcttact 19
<210> 39
   <211> 19
   <212> DNA
   <213> Homo sapiens
<400> 39
   gcaataaagg aagccttgc 19
<210> 40
   <211> 19
   <212> DNA
   <213> Homo sapiens
<400> 40
   cgcaataaag gaagccttg 19
<210> 41
   <211> 19
   <212> DNA
   <213> Homo sapiens
<400> 41
   aataaaggaa gccttgcct 19
<210> 42
   <211> 19
   <212> DNA
   <213> Homo sapiens
<400> 42
   ataaaggaag ccttgcctt 19
<210> 43
   <211> 19
   <212> DNA
   <213> Homo sapiens
<400> 43
   gcaataaagg cagccttgc 19
<210> 44
   <211> 19
   <212> DNA
   <213> Homo sapiens
<400> 44
   cgcaataaag gcagccttg 19
<210> 45
   <211> 19
   <212> DNA
   <213> Homo sapiens
<400> 45
   aataaaggca gccttgcct 19
<210> 46
   <211> 19
   <212> DNA
   <213> Homo sapiens
<400> 46
   ataaaggcag ccttgcctt 19
<210> 47
   <211> 19
   <212> DNA
   <213> Homo sapiens
<400> 47
   gagggtttct ccgctcagc 19
<210> 48
   <211> 19
   <212> DNA
   <213> Homo sapiens
<400> 48
   ggagggtttc tccgctcag 19
<210> 49
   <211> 19
   <212> DNA
   <213> Homo sapiens
<400> 49
   gggtttctcc gctcagcct 19
<210> 50
   <211> 19
   <212> DNA
   <213> Homo sapiens
<400> 50
   ggtttctccg ctcagcctt 19
<210> 51
   <211> 19
   <212> DNA
   <213> Homo sapiens
<400> 51
   gagggtttct tcgctcagc 19
<210> 52
   <211> 19
   <212> DNA
   <213> Homo sapiens
<400> 52
   ggagggtttc ttcgctcag 19
<210> 53
   <211> 19
   <212> DNA
   <213> Homo sapiens
<400> 53
   gggtttcttc gctcagcct 19
<210> 54
   <211> 19
   <212> DNA
   <213> Homo sapiens
<400> 54
   ggtttcttcg ctcagcctt 19
<210> 55
   <211> 19
   <212> DNA
   <213> Homo sapiens
<400> 55
   ggagcaggag aacgacacc 19
<210> 56
   <211> 19
   <212> DNA
   <213> Homo sapiens
<400> 56
   cggagcagga gaacgacac 19
<210> 57
   <211> 19
   <212> DNA
   <213> Homo sapiens
<400> 57
   agcaggagaa cgacacctc 19
<210> 58
   <211> 19
   <212> DNA
   <213> Homo sapiens
<400> 58
   gcaggagaac gacacctcg 19
<210> 59
   <211> 19
   <212> DNA
   <213> Homo sapiens
<400> 59
   ggagcaggag cacgacacc 19
<210> 60
   <211> 19
   <212> DNA
   <213> Homo sapiens
<400> 60
   cggagcagga gcacgacac 19
<210> 61
   <211> 19
   <212> DNA
   <213> Homo sapiens
<400> 61
   agcaggagca cgacacctc 19
<210> 62
   <211> 19
   <212> DNA
   <213> Homo sapiens
<400> 62
   gcaggagcac gacacctcg 19
<210> 63
   <211> 19
   <212> DNA
   <213> Homo sapiens
<400> 63
   ttaagaggaa caaataaag 19
<210> 64
   <211> 19
   <212> DNA
   <213> Homo sapiens
<400> 64
   attaagagga acaaataaa 19
<210> 65
   <211> 19
   <212> DNA
   <213> Homo sapiens
<400> 65
   aagaggaaca aataaagct 19
<210> 66
   <211> 19
   <212> DNA
   <213> Homo sapiens
<400> 66
   agaggaacaa ataaagctg 19
<210> 67
   <211> 19
   <212> DNA
   <213> Homo sapiens
<400> 67
   ttaagaggaa taaataaag 19
<210> 68
   <211> 19
   <212> DNA
   <213> Homo sapiens
<400> 68
   attaagagga ataaataaa 19
<210> 69
   <211> 19
   <212> DNA
   <213> Homo sapiens
<400> 69
   aagaggaata aataaagct 19
<210> 70
   <211> 19
   <212> DNA
   <213> Homo sapiens
<400> 70
   agaggaataa ataaagctg 19
<210> 71
   <211> 19
   <212> DNA
   <213> Homo sapiens
<400> 71
   ggacagtact tcaacgcta 19
<210> 72
   <211> 19
   <212> DNA
   <213> Homo sapiens
<400> 72
   gacagtactt caacgctag 19
<210> 73
   <211> 19
   <212> DNA
   <213> Homo sapiens
<400> 73
   ggggacagta cttcaacgc 19
<210> 74
   <211> 19
   <212> DNA
   <213> Homo sapiens
<400> 74
   tggggacagt acttcaacg 19
<210> 75
   <211> 19
   <212> DNA
   <213> Homo sapiens
<400> 75
   ggacagtaat tcaacgcta 19
<210> 76
   <211> 19
   <212> DNA
   <213> Homo sapiens
<400> 76
   gacagtaatt caacgctag 19
<210> 77
   <211> 19
   <212> DNA
   <213> Homo sapiens
<400> 77
   ggggacagta attcaacgc 19
<210> 78
   <211> 19
   <212> DNA
   <213> Homo sapiens
<400> 78
   tggggacagt aattcaacg 19
<210> 79
   <211> 19
   <212> DNA
   <213> Homo sapiens
<400> 79
   ctccctcatc tactgtgtg 19
<210> 80
   <211> 19
   <212> DNA
   <213> Homo sapiens
<400> 80
   gctccctcat ctactgtgt 19
<210> 81
   <211> 19
   <212> DNA
   <213> Homo sapiens
<400> 81
   ccctcatcta ctgtgtgca 19
<210> 82
   <211> 19
   <212> DNA
   <213> Homo sapiens
<400> 82
   cctcatctac tgtgtgcac 19
<210> 83
   <211> 19
   <212> DNA
   <213> Homo sapiens
<400> 83
   ctccctcatc cactgtgtg 19
<210> 84
   <211> 19
   <212> DNA
   <213> Homo sapiens
<400> 84
   gctccctcat ccactgtgt 19
<210> 85
   <211> 19
   <212> DNA
   <213> Homo sapiens
<400> 85
   ccctcatcca ctgtgtgca 19
<210> 86
   <211> 19
   <212> DNA
   <213> Homo sapiens
<400> 86
   cctcatccac tgtgtgcac 19
<210> 87
   <211> 19
   <212> DNA
   <213> Homo sapiens
<400> 87
   accgcatcaa cacactagg 19
<210> 88
   <211> 19
   <212> DNA
   <213> Homo sapiens
<400> 88
   taccgcatca acacactag 19
<210> 89
   <211> 19
   <212> DNA
   <213> Homo sapiens
<400> 89
   cgcatcaaca cactaggct 19
<210> 90
   <211> 19
   <212> DNA
   <213> Homo sapiens
<400> 90
   gcatcaacac actaggctg 19
<210> 91
   <211> 19
   <212> DNA
   <213> Homo sapiens
<400> 91
   accgcatcaa tacactagg 19
<210> 92
   <211> 19
   <212> DNA
   <213> Homo sapiens
<400> 92
   taccgcatca atacactag 19
<210> 93
   <211> 19
   <212> DNA
   <213> Homo sapiens
<400> 93
   cgcatcaata cactaggct 19
<210> 94
   <211> 19
   <212> DNA
   <213> Homo sapiens
<400> 94
   gcatcaatac actaggctg 19
<210> 95
   <211> 19
   <212> DNA
   <213> Homo sapiens
<400> 95
   cctggtgacg cagcccctc 19
<210> 96
   <211> 19
   <212> DNA
   <213> Homo sapiens
<400> 96
   tcctggtgac gcagcccct 19
<210> 97
   <211> 19
   <212> DNA
   <213> Homo sapiens
<400> 97
   tggtgacgca gcccctcgt 19
<210> 98
   <211> 19
   <212> DNA
   <213> Homo sapiens
<400> 98
   ggtgacgcag cccctcgtg 19
<210> 99
   <211> 19
   <212> DNA
   <213> Homo sapiens
<400> 99
   cctggtgacg tagcccctc 19
<210> 100
   <211> 19
   <212> DNA
   <213> Homo sapiens
<400> 100
   tcctggtgac gtagcccct 19
<210> 101
   <211> 19
   <212> DNA
   <213> Homo sapiens
<400> 101
   tggtgacgta gcccctcgt 19
<210> 102
   <211> 19
   <212> DNA
   <213> Homo sapiens
<400> 102
   ggtgacgtag cccctcgtg 19
<210> 103
   <211> 19
   <212> DNA
   <213> Homo sapiens
<400> 103
   gaagcccata tcaccggct 19
<210> 104
   <211> 19
   <212> DNA
   <213> Homo sapiens
<400> 104
   ggaagcccat atcaccggc 19
<210> 105
   <211> 19
   <212> DNA
   <213> Homo sapiens
<400> 105
   agcccatatc accggctgc 19
<210> 106
   <211> 19
   <212> DNA
   <213> Homo sapiens
<400> 106
   gcccatatca ccggctgct 19
<210> 107
   <211> 19
   <212> DNA
   <213> Homo sapiens
<400> 107
   gaagcccata ccaccggct 19
<210> 108
   <211> 19
   <212> DNA
   <213> Homo sapiens
<400> 108
   ggaagcccat accaccggc 19
<210> 109
   <211> 19
   <212> DNA
   <213> Homo sapiens
<400> 109
   agcccatacc accggctgc 19
<210> 110
   <211> 19
   <212> DNA
   <213> Homo sapiens
<400> 110
   gcccatacca ccggctgct 19
<210> 111
   <211> 19
   <212> DNA
   <213> Homo sapiens
<400> 111
   tagcgactaa acacatcaa 19
<210> 112
   <211> 53
   <212> DNA
   <213> Homo sapiens
<400> 112
   aggccttcat agcgaacctt tcaagagaag gttcgctatg aaggcctttt ttg 53
<210> 113
   <211> 54
   <212> DNA
   <213> Homo sapiens
<400> 113
   tagcgactaa acacatcaat tcaagagatt gatgtgttta gtcgctattt tttg 54
<210> 114
   <211> 19
   <212> DNA
   <213> Homo sapiens
<400> 114
   aaatgtgctg tgaggctta 19
<210> 115
   <211> 19
   <212> DNA
   <213> Homo sapiens
<400> 115
   gctactaaat gtgctgtta 19
<210> 116
   <211> 19
   <212> DNA
   <213> Homo sapiens
<400> 116
   aaatgtgctc tgaggctta 19
<210> 117
   <211> 19
   <212> DNA
   <213> Homo sapiens
<400> 117
   gctactaaat gtgctctta 19
<210> 118
   <211> 19
   <212> DNA
   <213> Homo sapiens
<400> 118
   gtttggaggg tttctccgc 19
<210> 119
   <211> 19
   <212> DNA
   <213> Homo sapiens
<400> 119
   agggtttctc ctctcagcc 19
<210> 120
   <211> 19
   <212> DNA
   <213> Homo sapiens
<400> 120
   agggtttctc cactcagcc 19
<210> 121
   <211> 19
   <212> DNA
   <213> Homo sapiens
<400> 121
   gtttggaggg tttcttcgc 19
<210> 122
   <211> 19
   <212> DNA
   <213> Homo sapiens
<400> 122
   agggtttctt ctctcagcc 19
<210> 123
   <211> 19
   <212> DNA
   <213> Homo sapiens
<400> 123
   agggtttctt cactcagcc 19
<210> 124
   <211> 19
   <212> DNA
   <213> Homo sapiens
<400> 124
   ggacagtaca tcaacgcta 19
<210> 125
   <211> 19
   <212> DNA
   <213> Homo sapiens
<400> 125
   gagatgggga cagtacttc 19
<210> 126
   <211> 19
   <212> DNA
   <213> Homo sapiens
<400> 126
   ggggacagta cttaaacgc 19
<210> 127
   <211> 19
   <212> DNA
   <213> Homo sapiens
<400> 127
   ggacagtaag tcaacgcta 19
<210> 128
   <211> 19
   <212> DNA
   <213> Homo sapiens
<400> 128
   gagatgggga cagtaattc 19
<210> 129
   <211> 19
   <212> DNA
   <213> Homo sapiens
<400> 129
   ggggacagta attaaacgc 19
<210> 130
   <211> 19
   <212> DNA
   <213> Homo sapiens
<400> 130
   gcctgctccc tcatctact 19
<210> 131
   <211> 19
   <212> DNA
   <213> Homo sapiens
<400> 131
   tccctcatct actgggtgc 19
<210> 132
   <211> 19
   <212> DNA
   <213> Homo sapiens
<400> 132
   tccctcatct acggtgtgc 19
<210> 133
   <211> 19
   <212> DNA
   <213> Homo sapiens
<400> 133
   gcctgctccc tcatccact 19
<210> 134
   <211> 19
   <212> DNA
   <213> Homo sapiens
<400> 134
   tccctcatcc actgggtgc 19
<210> 135
   <211> 19
   <212> DNA
   <213> Homo sapiens
<400> 135
   tccctcatcc acggtgtgc 19
<210> 136
   <211> 21
   <212> DNA
   <213> Homo sapiens
<400> 136
   gagaugggga caguacuuct t 21
<210> 137
   <211> 21
   <212> DNA
   <213> Homo sapiens
<400> 137
   gaaguacugu ccccaucuct t 21
<210> 138
   <211> 21
   <212> DNA
   <213> Homo sapiens
<400> 138
   ggacaguaag ucaacgcuat t 21
<210> 139
   <211> 21
   <212> DNA
   <213> Homo sapiens
<400> 139
   uagcguugac uuacuguccc t 21
<210> 140
   <211> 19
   <212> DNA
   <213> Homo sapiens
<400> 140
   actcctccac ctttgacgc 19
<210> 141
   <211> 21
   <212> DNA
   <213> Homo sapiens
<400> 141
   gttgctgtag ccaaattcgt t 21
<210> 142
   <211> 40
   <212> DNA
   <213> Artificial
<220>
   <223> molecular beacon
<400> 142
   cgcgatcgat ggggacagta cttcaacgct agagatcgcg 40
<210> 143
   <211> 26
   <212> DNA
   <213> Homo sapiens
<400> 143
   agatattgtt ctttctcgta ttcagg 26
<210> 144
   <211> 21
   <212> DNA
   <213> Homo sapiens
<400> 144
   tgctcactca tttccacctt c 21
<210> 145
   <211> 35
   <212> DNA
   <213> Artificial
<220>
   <223> molecular beacon
<400> 145
   agtgcgtggg acagtaattc aacgctagac gcact 35
<210> 146
   <211> 27
   <212> DNA
   <213> Homo sapiens
<400> 146
   ccttctctcc gtatttaatc tcctgta 27
<210> 147
   <211> 27
   <212> DNA
   <213> Homo sapiens
<400> 147
   tcatttccac cttcagctgt tttgtaa 27
<210> 148
   <211> 62
   <212> DNA
   <213> Homo sapiens
<400> 148
   ccggtccgga agtatcgctt ggaaagttct cttccaagcg atacttccgg aaaaaaactt 60
   aa 62
<210> 149
   <211> 62
   <212> DNA
   <213> Homo sapiens
<400> 149
   ccggatcgct gatttgtgta gttaagttct ctaactacac aaatcagcga taaaaaactt 60
   aa 62
<210> 150
   <211> 13482
   <212> DNA
   <213> Homo sapiens
<400> 150
   gctgccggga cgggtccaag atggacggcc gctcaggttc tgcttttacc tgcggcccag   60
   agccccattc attgccccgg tgctgagcgg cgccgcgagt cggcccgagg cctccgggga  120
   ctgccgtgcc gggcgggaga ccgccatggc gaccctggaa aagctgatga aggccttcga  180
   gtccctcaag tccttccagc agcagcagca gcagcagcag cagcagcagc agcagcagca  240
   gcagcagcag cagcagcagc aacagccgcc accgccgccg ccgccgccgc cgcctcctca  300
   gcttcctcag ccgccgccgc aggcacagcc gctgctgcct cagccgcagc cgcccccgcc  360
   gccgcccccg ccgccacccg gcccggctgt ggctgaggag ccgctgcacc gaccaaagaa  420
   agaactttca gctaccaaga aagaccgtgt gaatcattgt ctgacaatat gtgaaaacat  480
   agtggcacag tctgtcagaa attctccaga atttcagaaa cttctgggca tcgctatgga  540
   actttttctg ctgtgcagtg atgacgcaga gtcagatgtc aggatggtgg ctgacgaatg  600
   cctcaacaaa gttatcaaag ctttgatgga ttctaatctt ccaaggttac agctcgagct  660
   ctataaggaa attaaaaaga atggtgcccc tcggagtttg cgtgctgccc tgtggaggtt  720
   tgctgagctg gctcacctgg ttcggcctca gaaatgcagg ccttacctgg tgaaccttct  780
   gccgtgcctg actcgaacaa gcaagagacc cgaagaatca gtccaggaga ccttggctgc  840
   agctgttccc aaaattatgg cttcttttgg caattttgca aatgacaatg aaattaaggt  900
   tttgttaaag gccttcatag cgaacctgaa gtcaagctcc cccaccattc ggcggacagc  960
   ggctggatca gcagtgagca tctgccagca ctcaagaagg acacaatatt tctatagttg 1020
   gctactaaat gtgctcttag gcttactcgt tcctgtcgag gatgaacact ccactctgct 1080
   gattcttggc gtgctgctca ccctgaggta tttggtgccc ttgctgcagc agcaggtcaa 1140
   ggacacaagc ctgaaaggca gcttcggagt gacaaggaaa gaaatggaag tctctccttc 1200
   tgcagagcag cttgtccagg tttatgaact gacgttacat catacacagc accaagacca 1260
   caatgttgtg accggagccc tggagctgtt gcagcagctc ttcagaacgc ctccacccga 1320
   gcttctgcaa accctgaccg cagtcggggg cattgggcag ctcaccgctg ctaaggagga 1380
   gtctggtggc cgaagccgta gtgggagtat tgtggaactt atagctggag ggggttcctc 1440
   atgcagccct gtcctttcaa gaaaacaaaa aggcaaagtg ctcttaggag aagaagaagc 1500
   cttggaggat gactctgaat cgagatcgga tgtcagcagc tctgccttaa cagcctcagt 1560
   gaaggatgag atcagtggag agctggctgc ttcttcaggg gtttccactc cagggtcagc 1620
   aggtcatgac atcatcacag aacagccacg gtcacagcac acactgcagg cggactcagt 1680
   ggatctggcc agctgtgact tgacaagctc tgccactgat ggggatgagg aggatatctt 1740
   gagccacagc tccagccagg tcagcgccgt cccatctgac cctgccatgg acctgaatga 1800
   tgggacccag gcctcgtcgc ccatcagcga cagctcccag accaccaccg aagggcctga 1860
   ttcagctgtt accccttcag acagttctga aattgtgtta gacggtaccg acaaccagta 1920
   tttgggcctg cagattggac agccccagga tgaagatgag gaagccacag gtattcttcc 1980
   tgatgaagcc tcggaggcct tcaggaactc ttccatggcc cttcaacagg cacatttatt 2040
   gaaaaacatg agtcactgca ggcagccttc tgacagcagt gttgataaat ttgtgttgag 2100
   agatgaagct actgaaccgg gtgatcaaga aaacaagcct tgccgcatca aaggtgacat 2160
   tggacagtcc actgatgatg actctgcacc tcttgtccat tgtgtccgcc ttttatctgc 2220
   ttcgtttttg ctaacagggg gaaaaaatgt gctggttccg gacagggatg tgagggtcag 2280
   cgtgaaggcc ctggccctca gctgtgtggg agcagctgtg gccctccacc cggaatcttt 2340
   cttcagcaaa ctctataaag ttcctcttga caccacggaa taccctgagg aacagtatgt 2400
   ctcagacatc ttgaactaca tcgatcatgg agacccacag gttcgaggag ccactgccat 2460
   tctctgtggg accctcatct gctccatcct cagcaggtcc cgcttccacg tgggagattg 2520
   gatgggcacc attagaaccc tcacaggaaa tacattttct ttggcggatt gcattccttt 2580
   gctgcggaaa acactgaagg atgagtcttc tgttacttgc aagttagctt gtacagctgt 2640
   gaggaactgt gtcatgagtc tctgcagcag cagctacagt gagttaggac tgcagctgat 2700
   catcgatgtg ctgactctga ggaacagttc ctattggctg gtgaggacag agcttctgga 2760
   aacccttgca gagattgact tcaggctggt gagctttttg gaggcaaaag cagaaaactt 2820
   acacagaggg gctcatcatt atacagggct tttaaaactg caagaacgag tgctcaataa 2880
   tgttgtcatc catttgcttg gagatgaaga ccccagggtg cgacatgttg ccgcagcatc 2940
   actaattagg cttgtcccaa agctgtttta taaatgtgac caaggacaag ctgatccagt 3000
   agtggccgtg gcaagagatc aaagcagtgt ttacctgaaa cttctcatgc atgagacgca 3060
   gcctccatct catttctccg tcagcacaat aaccagaata tatagaggct ataacctact 3120
   accaagcata acagacgtca ctatggaaaa taacctttca agagttattg cagcagtttc 3180
   tcatgaacta atcacatcaa ccaccagagc actcacattt ggatgctgtg aagctttgtg 3240
   tcttctttcc actgccttcc cagtttgcat ttggagttta ggttggcact gtggagtgcc 3300
   tccactgagt gcctcagatg agtctaggaa gagctgtacc gttgggatgg ccacaatgat 3360
   tctgaccctg ctctcgtcag cttggttccc attggatctc tcagcccatc aagatgcttt 3420
   gattttggcc ggaaacttgc ttgcagccag tgctcccaaa tctctgagaa gttcatgggc 3480
   ctctgaagaa gaagccaacc cagcagccac caagcaagag gaggtctggc cagccctggg 3540
   ggaccgggcc ctggtgccca tggtggagca gctcttctct cacctgctga aggtgattaa 3600
   catttgtgcc cacgtcctgg atgacgtggc tcctggaccc gcaataaagg cagccttgcc 3660
   ttctctaaca aacccccctt ctctaagtcc catccgacga aaggggaagg agaaagaacc 3720
   aggagaacaa gcatctgtac cgttgagtcc caagaaaggc agtgaggcca gtgcagcttc 3780
   tagacaatct gatacctcag gtcctgttac aacaagtaaa tcctcatcac tggggagttt 3840
   ctatcatctt ccttcatacc tcaaactgca tgatgtcctg aaagctacac acgctaacta 3900
   caaggtcacg ctggatcttc agaacagcac ggaaaagttt ggagggtttc tccgctcagc 3960
   cttggatgtt ctttctcaga tactagagct ggccacactg caggacattg ggaagtgtgt 4020
   tgaagagatc ctaggatacc tgaaatcctg ctttagtcga gaaccaatga tggcaactgt 4080
   ttgtgttcaa caattgttga agactctctt tggcacaaac ttggcctccc agtttgatgg 4140
   cttatcttcc aaccccagca agtcacaagg ccgagcacag cgccttggct cctccagtgt 4200
   gaggccaggc ttgtaccact actgcttcat ggccccgtac acccacttca cccaggccct 4260
   cgctgacgcc agcctgagga acatggtgca ggcggagcag gagaacgaca cctcgggatg 4320
   gtttgatgtc ctccagaaag tgtctaccca gttgaagaca aacctcacga gtgtcacaaa 4380
   gaaccgtgca gataagaatg ctattcataa tcacattcgt ttgtttgaac ctcttgttat 4440
   aaaagcttta aaacagtaca cgactacaac atgtgtgcag ttacagaagc aggttttaga 4500
   tttgctggcg cagctggttc agttacgggt taattactgt cttctggatt cagatcaggt 4560
   gtttattggc tttgtattga aacagtttga atacattgaa gtgggccagt tcagggaatc 4620
   agaggcaatc attccaaaca tctttttctt cttggtatta ctatcttatg aacgctatca 4680
   ttcaaaacag atcattggaa ttcctaaaat cattcagctc tgtgatggca tcatggccag 4740
   tggaaggaag gctgtgacac atgccatacc ggctctgcag cccatagtcc acgacctctt 4800
   tgtattaaga ggaacaaata aagctgatgc aggaaaagag cttgaaaccc aaaaagaggt 4860
   ggtggtgtca atgttactga gactcatcca gtaccatcag gtgttggaga tgttcattct 4920
   tgtcctgcag cagtgccaca aggagaatga agacaagtgg aagcgactgt ctcgacagat 4980
   agctgacatc atcctcccaa tgttagccaa acagcagatg cacattgact ctcatgaagc 5040
   ccttggagtg ttaaatacat tatttgagat tttggcccct tcctccctcc gtccggtaga 5100
   catgctttta cggagtatgt tcgtcactcc aaacacaatg gcgtccgtga gcactgttca 5160
   actgtggata tcgggaattc tggccatttt gagggttctg atttcccagt caactgaaga 5220
   tattgttctt tctcgtattc aggagctctc cttctctccg tatttaatct cctgtacagt 5280
   aattaatagg ttaagagatg gggacagtac ttcaacgcta gaagaacaca gtgaagggaa 5340
   acaaataaag aatttgccag aagaaacatt ttcaaggttt ctattacaac tggttggtat 5400
   tcttttagaa gacattgtta caaaacagct gaaggtggaa atgagtgagc agcaacatac 5460
   tttctattgc caggaactag gcacactgct aatgtgtctg atccacatct tcaagtctgg 5520
   aatgttccgg agaatcacag cagctgccac taggctgttc cgcagtgatg gctgtggcgg 5580
   cagtttctac accctggaca gcttgaactt gcgggctcgt tccatgatca ccacccaccc 5640
   ggccctggtg ctgctctggt gtcagatact gctgcttgtc aaccacaccg actaccgctg 5700
   gtgggcagaa gtgcagcaga ccccgaaaag acacagtctg tccagcacaa agttacttag 5760
   tccccagatg tctggagaag aggaggattc tgacttggca gccaaacttg gaatgtgcaa 5820
   tagagaaata gtacgaagag gggctctcat tctcttctgt gattatgtct gtcagaacct 5880
   ccatgactcc gagcacttaa cgtggctcat tgtaaatcac attcaagatc tgatcagcct 5940
   ttcccacgag cctccagtac aggacttcat cagtgccgtt catcggaact ctgctgccag 6000
   cggcctgttc atccaggcaa ttcagtctcg ttgtgaaaac ctttcaactc caaccatgct 6060
   gaagaaaact cttcagtgct tggaggggat ccatctcagc cagtcgggag ctgtgctcac 6120
   gctgtatgtg gacaggcttc tgtgcacccc tttccgtgtg ctggctcgca tggtcgacat 6180
   ccttgcttgt cgccgggtag aaatgcttct ggctgcaaat ttacagagca gcatggccca 6240
   gttgccaatg gaagaactca acagaatcca ggaatacctt cagagcagcg ggctcgctca 6300
   gagacaccaa aggctctatt ccctgctgga caggtttcgt ctctccacca tgcaagactc 6360
   acttagtccc tctcctccag tctcttccca cccgctggac ggggatgggc acgtgtcact 6420
   ggaaacagtg agtccggaca aagactggta cgttcatctt gtcaaatccc agtgttggac 6480
   caggtcagat tctgcactgc tggaaggtgc agagctggtg aatcggattc ctgctgaaga 6540
   tatgaatgcc ttcatgatga actcggagtt caacctaagc ctgctagctc catgcttaag 6600
   cctagggatg agtgaaattt ctggtggcca gaagagtgcc ctttttgaag cagcccgtga 6660
   ggtgactctg gcccgtgtga gcggcaccgt gcagcagctc cctgctgtcc atcatgtctt 6720
   ccagcccgag ctgcctgcag agccggcggc ctactggagc aagttgaatg atctgtttgg 6780
   ggatgctgca ctgtatcagt ccctgcccac tctggcccgg gccctggcac agtacctggt 6840
   ggtggtctcc aaactgccca gtcatttgca ccttcctcct gagaaagaga aggacattgt 6900
   gaaattcgtg gtggcaaccc ttgaggccct gtcctggcat ttgatccatg agcagatccc 6960
   gctgagtctg gatctccagg cagggctgga ctgctgctgc ctggccctgc agctgcctgg 7020
   cctctggagc gtggtctcct ccacagagtt tgtgacccac gcctgctccc tcatctactg 7080
   tgtgcacttc atcctggagg ccgttgcagt gcagcctgga gagcagcttc ttagtccaga 7140
   aagaaggaca aataccccaa aagccatcag cgaggaggag gaggaagtag atccaaacac 7200
   acagaatcct aagtatatca ctgcagcctg tgagatggtg gcagaaatgg tggagtctct 7260
   gcagtcggtg ttggccttgg gtcataaaag gaatagcggc gtgccggcgt ttctcacgcc 7320
   attgctcagg aacatcatca tcagcctggc ccgcctgccc cttgtcaaca gctacacacg 7380
   tgtgccccca ctggtgtgga agcttggatg gtcacccaaa ccgggagggg attttggcac 7440
   agcattccct gagatccccg tggagttcct ccaggaaaag gaagtcttta aggagttcat 7500
   ctaccgcatc aacacactag gctggaccag tcgtactcag tttgaagaaa cttgggccac 7560
   cctccttggt gtcctggtga cgcagcccct cgtgatggag caggaggaga gcccaccaga 7620
   agaagacaca gagaggaccc agatcaacgt cctggccgtg caggccatca cctcactggt 7680
   gctcagtgca atgactgtgc ctgtggccgg caacccagct gtaagctgct tggagcagca 7740
   gccccggaac aagcctctga aagctctcga caccaggttt gggaggaagc tgagcattat 7800
   cagagggatt gtggagcaag agattcaagc aatggtttca aagagagaga atattgccac 7860
   ccatcattta tatcaggcat gggatcctgt cccttctctg tctccggcta ctacaggtgc 7920
   cctcatcagc cacgagaagc tgctgctaca gatcaacccc gagcgggagc tggggagcat 7980
   gagctacaaa ctcggccagg tgtccataca ctccgtgtgg ctggggaaca gcatcacacc 8040
   cctgagggag gaggaatggg acgaggaaga ggaggaggag gccgacgccc ctgcaccttc 8100
   gtcaccaccc acgtctccag tcaactccag gaaacaccgg gctggagttg acatccactc 8160
   ctgttcgcag tttttgcttg agttgtacag ccgctggatc ctgccgtcca gctcagccag 8220
   gaggaccccg gccatcctga tcagtgaggt ggtcagatcc cttctagtgg tctcagactt 8280
   gttcaccgag cgcaaccagt ttgagctgat gtatgtgacg ctgacagaac tgcgaagggt 8340
   gcacccttca gaagacgaga tcctcgctca gtacctggtg cctgccacct gcaaggcagc 8400
   tgccgtcctt gggatggaca aggccgtggc ggagcctgtc agccgcctgc tggagagcac 8460
   gctcaggagc agccacctgc ccagcagggt tggagccctg cacggcgtcc tctatgtgct 8520
   ggagtgcgac ctgctggacg acactgccaa gcagctcatc ccggtcatca gcgactatct 8580
   cctctccaac ctgaaaggga tcgcccactg cgtgaacatt cacagccagc agcacgtact 8640
   ggtcatgtgt gccactgcgt tttacctcat tgagaactat cctctggacg tagggccgga 8700
   attttcagca tcaataatac agatgtgtgg ggtgatgctg tctggaagtg aggagtccac 8760
   cccctccatc atttaccact gtgccctcag aggcctggag cgcctcctgc tctctgagca 8820
   gctctcccgc ctggatgcag aatcgctggt caagctgagt gtggacagag tgaacgtgca 8880
   cagcccgcac cgggccatgg cggctctggg cctgatgctc acctgcatgt acacaggaaa 8940
   ggagaaagtc agtccgggta gaacttcaga ccctaatcct gcagcccccg acagcgagtc 9000
   agtgattgtt gctatggagc gggtatctgt tctttttgat aggatcagga aaggctttcc 9060
   ttgtgaagcc agagtggtgg ccaggatcct gccccagttt ctagacgact tcttcccacc 9120
   ccaggacatc atgaacaaag tcatcggaga gtttctgtcc aaccagcagc cataccccca 9180
   gttcatggcc accgtggtgt ataaggtgtt tcagactctg cacagcaccg ggcagtcgtc 9240
   catggtccgg gactgggtca tgctgtccct ctccaacttc acgcagaggg ccccggtcgc 9300
   catggccacg tggagcctct cctgcttctt tgtcagcgcg tccaccagcc cgtgggtcgc 9360
   ggcgatcctc ccacatgtca tcagcaggat gggcaagctg gagcaggtgg acgtgaacct 9420
   tttctgcctg gtcgccacag acttctacag acaccagata gaggaggagc tcgaccgcag 9480
   ggccttccag tctgtgcttg aggtggttgc agccccagga agcccatatc accggctgct 9540
   gacttgttta cgaaatgtcc acaaggtcac cacctgctga gcgccatggt gggagagact 9600
   gtgaggcggc agctggggcc ggagcctttg gaagtctgtg cccttgtgcc ctgcctccac 9660
   cgagccagct tggtccctat gggcttccgc acatgccgcg ggcggccagg caacgtgcgt 9720
   gtctctgcca tgtggcagaa gtgctctttg tggcagtggc caggcaggga gtgtctgcag 9780
   tcctggtggg gctgagcctg aggccttcca gaaagcagga gcagctgtgc tgcaccccat 9840
   gtgggtgacc aggtcctttc tcctgatagt cacctgctgg ttgttgccag gttgcagctg 9900
   ctcttgcatc tgggccagaa gtcctccctc ctgcaggctg gctgttggcc cctctgctgt 9960
   cctgcagtag aaggtgccgt gagcaggctt tgggaacact ggcctgggtc tccctggtgg 10020
   ggtgtgcatg ccacgccccg tgtctggatg cacagatgcc atggcctgtg ctgggccagt 10080
   ggctgggggt gctagacacc cggcaccatt ctcccttctc tcttttcttc tcaggattta 10140
   aaatttaatt atatcagtaa agagattaat tttaacgtaa ctctttctat gcccgtgtaa 10200
   agtatgtgaa tcgcaaggcc tgtgctgcat gcgacagcgt ccggggtggt ggacagggcc 10260
   cccggccacg ctccctctcc tgtagccact ggcatagccc tcctgagcac ccgctgacat 10320
   ttccgttgta catgttcctg tttatgcatt cacaaggtga ctgggatgta gagaggcgtt 10380
   agtgggcagg tggccacagc aggactgagg acaggccccc attatcctag gggtgcgctc 10440
   acctgcagcc cctcctcctc gggcacagac gactgtcgtt ctccacccac cagtcaggga 10500
   cagcagcctc cctgtcactc agctgagaag gccagccctc cctggctgtg agcagcctcc 10560
   actgtgtcca gagacatggg cctcccactc ctgttccttg ctagccctgg ggtggcgtct 10620
   gcctaggagc tggctggcag gtgttgggac ctgctgctcc atggatgcat gccctaagag 10680
   tgtcactgag ctgtgttttg tctgagcctc tctcggtcaa cagcaaagct tggtgtcttg 10740
   gcactgttag tgacagagcc cagcatccct tctgcccccg ttccagctga catcttgcac 10800
   ggtgacccct tttagtcagg agagtgcaga tctgtgctca tcggagactg ccccacggcc 10860
   ctgtcagagc cgccactcct atccccaggc caggtccctg gaccagcctc ctgtttgcag 10920
   gcccagagga gccaagtcat taaaatggaa gtggattctg gatggccggg ctgctgctga 10980
   tgtaggagct ggatttggga gctctgcttg ccgactggct gtgagacgag gcaggggctc 11040
   tgcttcctca gccctagagg cgagccaggc aaggttggcg actgtcatgt ggcttggttt 11100
   ggtcatgccc gtcgatgttt tgggtattga atgtggtaag tggaggaaat gttggaactc 11160
   tgtgcaggtg ctgccttgag acccccaagc ttccacctgt ccctctccta tgtggcagct 11220
   ggggagcagc tgagatgtgg acttgtatgc tgcccacata cgtgaggggg agctgaaagg 11280
   gagcccctcc tctgagcagc ctctgccagg cctgtatgag gcttttccca ccagctccca 11340
   acagaggcct cccccagcca ggaccacctc gtcctcgtgg cggggcagca ggagcggtag 11400
   aaaggggtcc gatgtttgag gaggccctta agggaagcta ctgaattata acacgtaaga 11460
   aaatcaccat tccgtattgg ttgggggctc ctgtttctca tcctagcttt ttcctggaaa 11520
   gcccgctaga aggtttggga acgaggggaa agttctcaga actgttggct gctccccacc 11580
   cgcctcccgc ctcccccgca ggttatgtca gcagctctga gacagcagta tcacaggcca 11640
   gatgttgttc ctggctagat gtttacattt gtaagaaata acactgtgaa tgtaaaacag 11700
   agccattccc ttggaatgca tatcgctggg ctcaacatag agtttgtctt cctcttgttt 11760
   acgacgtgat ctaaaccagt ccttagcaag gggctcagaa caccccgctc tggcagtagg 11820
   tgtcccccac ccccaaagac ctgcctgtgt gctccggaga tgaatatgag ctcattagta 11880
   aaaatgactt cacccacgca tatacataaa gtatccatgc atgtgcatat agacacatct 11940
   ataattttac acacacacct ctcaagacgg agatgcatgg cctctaagag tgcccgtgtc 12000
   ggttcttcct ggaagttgac tttccttaga cccgccaggt caagttagcc gcgtgacgga 12060
   catccaggcg tgggacgtgg tcagggcagg gctcattcat tgcccactag gatcccactg 12120
   gcgaagatgg tctccatatc agctctctgc agaagggagg aagactttat catgttccta 12180
   aaaatctgtg gcaagcaccc atcgtattat ccaaattttg ttgcaaatgt gattaatttg 12240
   gttgtcaagt tttgggggtg ggctgtgggg agattgcttt tgttttcctg ctggtaatat 12300
   cgggaaagat tttaatgaaa ccagggtaga attgtttggc aatgcactga agcgtgtttc 12360
   tttcccaaaa tgtgcctccc ttccgctgcg ggcccagctg agtctatgta ggtgatgttt 12420
   ccagctgcca agtgctcttt gttactgtcc accctcattt ctgccagcgc atgtgtcctt 12480
   tcaaggggaa aatgtgaagc tgaaccccct ccagacaccc agaatgtagc atctgagaag 12540
   gccctgtgcc ctaaaggaca cccctcgccc ccatcttcat ggagggggtc atttcagagc 12600
   cctcggagcc aatgaacagc tcctcctctt ggagctgaga tgagccccac gtggagctcg 12660
   ggacggatag tagacagcaa taactcggtg tgtggccgcc tggcaggtgg aacttcctcc 12720
   cgttgcgggg tggagtgagg ttagttctgt gtgtctggtg ggtggagtca ggcttctctt 12780
   gctacctgtg agcatccttc ccagcagaca tcctcatcgg gctttgtccc tcccccgctt 12840
   cctccctctg cggggaggac ccgggaccac agctgctggc cagggtagac ttggagctgt 12900
   cctccagagg ggtcacgtgt aggagtgaga agaaggaaga tcttgagagc tgctgaggga 12960
   ccttggagag ctcaggatgg ctcagacgag gacactcgct tgccgggcct gggcctcctg 13020
   ggaaggaggg agctgctcag aatgccgcat gacaactgaa ggcaacctgg aaggttcagg 13080
   ggccgctctt cccccatgtg cctgtcacgc tctggtgcag tcaaaggaac gccttcccct 13140
   cagttgtttc taagagcaga gtctcccgct gcaatctggg tggtaactgc cagccttgga 13200
   ggatcgtggc caacgtggac ctgcctacgg agggtgggct ctgacccaag tggggcctcc 13260
   ttgtccaggt ctcactgctt tgcaccgtgg tcagagggac tgtcagctga gcttgagctc 13320
   ccctggagcc agcagggctg tgatgggcga gtcccggagc cccacccaga cctgaatgct 13380
   tctgagagca aagggaagga ctgacgagag atgtatattt aattttttaa ctgctgcaaa 13440
   cattgtacat ccaaattaaa ggaaaaaaat ggaaaccatc aa 13482

## Description

### FIELD OF THE INVENTION

The present invention relates to methods and sequences to preferentially suppress the expression of the mutated huntingtin ("htt") protein over expression of the normal htt protein.

### BACKGROUND OF THE INVENTION

Huntington's disease ("HD") is a degenerative brain disorder. It slowly destroys an affected individual's ability to walk, think, talk and reason. Symptoms include changes in cognitive ability, such as impaired short-term memory and a decreased ability to concentrate; changes in mood, such as the development of mood swings, depression and irritability; and changes in coordination and physical movement such as clumsiness, involuntary movements and twitching. These symptoms gradually worsen until HD patients die, approximately 15-20 years after the onset of the disease.

While the biochemical cause of HD is not yet fully understood, it is now known that HD is inherited. Organisms typically inherit two copies of each gene, one from each parent. An individual copy of a gene inherited from one parent is called an allele. With regard to the inheritance of HD, every individual who inherits a HD-causing allele from either parent will develop the disease.

One breakthrough in research regarding HO has been the identification of the mutated gene that causes HD. Based on this breakthrough, researchers and physicians now can predict which individuals will develop HD. Specifically, researchers and physicians can predict which individuals will develop HO by counting the number of "CAG repeats" that exist within an individual's HD genes. If a person has 35 or less CAG repeats in each of their HD gene alleles, that person will not develop HD. If a person has more than 35 CAG repeats in either of their HD gene alleles, that person will develop the disease. The more CAG repeats a person has over 35 in either gene allele, the earlier the person will develop the symptoms of HD.

The HD gene encodes for a protein called "huntingtin" ("htt"). The exact function of htt is not known. However, it is known that the increased number of CAG repeats in HD- causing gene alleles leads to htt proteins with expanded polyglutamine sequences. Expanded polyglutamine sequences in proteins confer novel toxic properties resulting from a tendency of the protein to misfold and form aggregates within brain cells. Thus, suppressing the production of htt in brain cells may provide an avenue to prevent the accumulation of toxic htt in brain cells. This prevention can provide an avenue to further study the physiological mechanisms underlying HD and may also prevent or alleviate the symptoms or occurrence of HD.

While suppressing the production of htt could provide a method to prevent or treat the symptoms of HD, because its functions are not fully understood, fully blocking the expression of this protein may not be desirable. Indeed, animal studies have shown that htt is critical at least during development because animals lacking the protein altogether do not survive. Thus, it would be desirable to preferentially suppress the expression of toxic or mutated htt encoded for by a mutated HD allele while allowing expression of the normal HD allele that encodes for a normal htt protein.

Recent developments in genetic technologies have made the selective suppression of certain alleles and proteins, such as the mutated HD gene allele and the mutated htt protein, possible. Some background in the art is required to understand the potential impact of these technologies. Generally, for a protein to exert an effect, the cell that will use the protein must create it. To create a protein the cell first makes a copy of the protein's gene sequence in the nucleus of the cell. This copy of the gene sequence that encodes for the protein (called messenger RNA ("mRNA")) leaves the nucleus and is trafficked to a region of the cell containing ribosomes. Ribosomes read the sequence of the mRNA and create the protein for which it encodes. This process of new protein synthesis is known as translation. A variety of factors affect the rate and efficiency of protein translation. Among the most significant of these factors is the intrinsic stability of the mRNA itself. If the mRNA is degraded quickly within the cell (such as before it reaches a ribosome), it is unable to serve as a template for new protein translation, thus reducing the cell's ability to create the protein for which it encoded.

Based on the foregoing, the technology of RNA interference ("RNAi") has emerged. RNA interference is, in fact, a naturally occurring mechanism for suppressing gene expression and subsequent protein translation. RNA interference suppresses protein translation by either degrading the mRNA before it can be translated or by binding the mRNA and directly preventing its translation. Generally, RNAi has been used to suppress the expression of both gene alleles that lead to the production of a given protein. However, in some cases, such as those involved in the present invention, it is desirable to suppress the expression of only one allele.

Recently, several single nucleotide polymorphisms ("SNPs") have been identified that can be used to distinguish between the two HD gene alleles in human cells. SNPs are single nucleotide changes in the nucleotide sequence of a given gene. Thus, using RNAi, it is now possible to derive therapeutic agents to specifically suppress the expression of a mutated HD gene allele carrying a SNP. This personal approach can lead to individualized or "directed" therapeutics for HD patients, and provides an avenue to suppress the expression and actions of mutated toxic htt in an attempt to prevent or alleviate the symptoms of HD.

### SUMMARY OF THE INVENTION

The present invention describes a kit for screening individuals for the heterozygous presence of one or more single nucleotide polymorphisms within said individual's Huntington's genes, the kit comprising two pairs of molecular beacons selected from the group consisting of:
(a) a molecular beacon that detects a single polymorphism found in SEQ ID NO: 7, wherein said molecular beacon comprises at least a portion of a reverse complement of SEQ ID NO: 7 and a molecular beacon that detects a single polymorphism found in SEQ ID NO: 8, wherein said molecular beacon comprises at least a portion of a reverse complement of SEQ ID NO: 8;
(b) a molecular beacon that detects a single polymorphism found in SEQ ID NO: 15, wherein said molecular beacon comprises at least a portion of a reverse complement of SEQ ID NO: 15 and a molecular beacon that detects a single polymorphism found in SEQ ID NO: 16, wherein said molecular beacon comprises at least a portion of a reverse complement of SEQ ID NO: 16;
   and optionally, including at least one pair of molecular beacons selected from the group (c) and (d), wherein (c) and (d) are as follows:
(c) a molecular beacon that detects a single polymorphism found in SEQ ID NO: 3, wherein said molecular beacon comprises at least a portion of a reverse complement of SEQ ID NO: 3; and a molecular beacon that detects a single polymorphism found in SEQ ID NO: 4, wherein said molecular beacon comprises at least a portion of a reverse complement of SEQ ID NO: 4;
(d) a molecular beacon that detects a single polymorphism found in SEQ ID NO: 13, wherein said molecular beacon comprises at least a portion of a reverse complement of SEQ ID NO: 13 and a molecular beacon that detects a single polymorphism found in SEQ ID NO: 14, wherein said molecular beacon comprises at least a portion of a reverse complement of SEQ ID NO: 14,
wherein the presence or the detection of the SNP allows for deciding on allele-specific Huntington's disease treatment of the individual.
In a further embodiment, the present invention relates to the above kit for screening that further comprises nucleic acid molecules that preferentially suppress the expression of amino acid sequences encoding for mutated huntingtin ("htt") over suppressing the expression of amino acid sequences encoding for normal htt by targeting an area of a Huntington's disease gene that is heterozygous for the presence of one or more single nucleotide polymorphisms.
In a further embodiment, the present invention relates to the above kit for screening individuals for the heterozygous presence of one or more SNPs with the individual's Huntingtin genes, wherein said one or more SNPs are selected from the group consisting of SNP4, and SNP8.
In a further embodiment, the present invention relates to the above kit for screening which comprises:
(a) a molecular beacon that detects a single polymorphism found in SEQ ID NO: 3, wherein said molecular beacon comprises at least a portion of a reverse complement of SEQ ID NO: 3;
(b) a molecular beacon that detects a single polymorphism found in SEQ ID NO: 4, wherein said molecular beacon comprises at least a portion of a reverse complement of SEQ ID NO: 4;
(c) a molecular beacon that detects a single polymorphism found in SEQ ID NO: 7, wherein said molecular beacon comprises at least a portion of a reverse complement of SEQ ID NO: 7;
(d) a molecular beacon that detects a single polymorphism found in SEQ ID NO: 8, wherein said molecular beacon comprises at least a portion of a reverse complement of SEQ ID NO: 8;
(e) a molecular beacon that detects a single polymorphism found in SEQ ID NO: 13, wherein said molecular beacon comprises at least a portion of a reverse complement of SEQ ID NO: 13;
(f) a molecular beacon that detects a single polymorphism found in SEQ ID NO: 14, wherein said molecular beacon comprises at least a portion of a reverse complement of SEQ ID NO: 14;
(g) a molecular beacon that detects a single polymorphism found in SEQ ID NO: 15, wherein said molecular beacon comprises at least a portion of a reverse complement of SEQ ID NO: 15; and
(h) a molecular beacon that detects a single polymorphism found in SEQ ID NO: 16, wherein said molecular beacon comprises at least a portion of a reverse complement of SEQ ID NO: 16.
In a further embodiment, the present invention relates to the *in vitro* use of the above-described kit for screening individuals for the heterozygous presence of one or more SNPs with the individual's Huntingtin genes, wherein said one or more SNPs are selected from SNP2, SNP4, SNP7 and SNP8.
In a further embodiment, the present invention relates to the above-described kit for use in allele-specific treatment of individuals heterozygous at one or more SNPs with the individual's Huntingtin genes, wherein said one or more SNPs are selected from SNP2, SNP4, SNP7 and SNP8.
Further disclosed herein are methods, nucleic acid sequences and molecules, expression cassettes and vectors for using RNA interference ("RNAi") to preferentially suppress the expression of the disease-causing HD gene allele while allowing expression of the normal HD gene allele. Specifically, RNAi is mediated by double stranded RNA ("d5RNA"), short hairpin RNA ("shRNA") or other nucleic acid molecules with similar characteristics. These nucleic acid molecules are processed or cut into smaller pieces by cellular enzymes including Dicer and Drosha. The smaller fragments of the nucleic acid molecules can then be taken up by a protein complex called the RNA-induced silencing complex ("RISC complex") that mediates degradation of specific mRNAs. The RISC complex will degrade mRNA that complementarily base pairs with the nucleic acid molecules it has taken up. In this manner, the mRNA is specifically destroyed, thus preventing the encoded-for protein from being made.

The understanding of the mechanism of RNA1 now allows geneticists to create nucleic acid molecules with sequences that are homologous to known gene sequences in order to suppress the expression or formation of certain proteins within a cell. In this invention, individuals (including patients and/or human or non-human research subjects) are screened for the presence of specific single nucleotide polymorphisms (SNPs) in their HD genes until a SNP is found in one of the patient's HD alleles that is not present in the other of the HD patient's alleles. The genetic information obtained from the screening then is used to choose nucleic acid sequences and molecules that are homologous to the mutated htt mRNA sequences. These nucleic acid sequences and molecules are introduced into cells to preferentially suppress the expression of mutated htt protein while allowing expression of the normal htt protein. Preferentially suppressing the expression of the mutated htt protein while allowing expression of the normal htt protein provides beneficial techniques to study the physiological mechanisms underlying HD and may also prevent or treat the symptoms of HD without fully blocking the expression of htt.

Further disclosed herein are methods based on nucleic acid molecules comprising nucleotide sequences that preferentially suppress the expression of amino acid sequences encoding for mutated huntingtin ("htt") over suppressing the expression of amino acid sequences encoding for normal htt by targeting an area of a Huntington's disease gene that is heterozygous for the presence of one or more single nucleotide polymorphisms wherein the individual has been screened for the heterozygous presence of one or more single nucleotide polymorphisms within the individual's Huntington's genes. In another embodiment of the methods of the present invention, the nucleic acid molecules comprise a nucleotide sequence that comprises SEQ ID NO: 1. Further disclosed herein are methods, the nucleic acid molecules comprise a nucleotide sequence that comprises SEQ ID NO: 2.
Further disclosed herein are methods, wherein the nucleic acid molecules comprise a nucleotide sequence that comprises SEQ ID NO: 3. Further disclosed herein are methods, wherein the nucleic acid molecules comprise a nucleotide sequence that comprises SEQ ID NO: 4. Further disclosed herein are methods, wherein the nucleic acid molecules comprise a nucleotide sequence that comprises SEQ ID NO: 5. Further disclosed herein are methods, wherein the nucleic acid molecules comprise a nucleotide sequence that comprises SEQ ID NO: 6. Further disclosed herein are methods, wherein the nucleic acid molecules comprise a nucleotide sequence that comprises SEQ ID NO: 7. Further disclosed herein are methods, wherein the nucleic acid molecules comprise a nucleotide sequence that comprises SEQ ID NO: 8. Further disclosed herein are methods, wherein the nucleic acid molecules comprise a nucleotide sequence that comprises SEQ ID NO: 9. Further disclosed herein are methods, wherein the nucleic acid molecules comprise a nucleotide sequence that comprises SEQ ID NO: 10.
Further disclosed herein are methods, wherein the nucleic acid molecules comprise a nucleotide sequence that comprises SEQ ID NO: 11 Further disclosed herein are methods, wherein the nucleic acid molecules comprise a nucleotide sequence that comprises SEQ ID NO: 12. Further disclosed herein are methods, wherein the nucleic acid molecules comprise a nucleotide sequence that comprises SEQ ID NO: 13. Further disclosed herein are methods, wherein the nucleic acid molecules comprise a nucleotide sequence that comprises SEQ ID NO: 14.
Further disclosed herein are methods, wherein the nucleic acid molecules comprise a nucleotide sequence that comprises SEQ ID NO: 15. Further disclosed herein are methods, wherein the nucleic acid molecules comprise a nucleotide sequence that comprises SEQ ID NO: 16. Further disclosed herein are methods , wherein the nucleic acid molecules comprise a nucleotide sequence that comprises SEQ ID NO: 17. Further disclosed herein are methods, wherein the nucleic acid molecules comprise a nucleotide sequence that comprises SEQ ID NO: 18.
Further disclosed herein are methods, wherein the nucleic acid molecules comprise a nucleotide sequence that comprises SEQ ID NO: 19. Further disclosed herein are methods, wherein the nucleic acid molecules comprise a nucleotide sequence that comprises SEQ ID NO: 20. Further disclosed herein are methods, wherein the nucleic acid molecules comprise a nucleotide sequence that comprises SEQ ID NO: 21. Further disclosed herein are methods, wherein the nucleic acid molecules comprise a nucleotide sequence that comprises SEQ ID NO: 22. Further disclosed herein are methods, wherein the nucleic acid molecules comprise a nucleotide sequence that comprises SEQ ID NO: 23. Further disclosed herein are methods, wherein the nucleic acid molecules comprise a nucleotide sequence that comprises SEQ ID NO: 24. Further disclosed herein are methods,wherein the nucleic acid molecules comprise a nucleotide sequence that comprises SEQ ID NO: 25. Further disclosed herein are methods, wherein the nucleic acid molecules comprise a nucleotide sequence that comprises SEQ ID NO: 26.
Further disclosed herein are methods, wherein the nucleic acid molecules comprise a nucleotide sequence that comprises SEQ ID NO: 27. Further disclosed herein are methods, wherein the nucleic acid molecules comprise a nucleotide sequence that comprises SEQ ID NO: 28. Further disclosed herein are methods, wherein the nucleic acid molecules comprise a nucleotide sequence that comprises SEQ ID NO: 29. Further disclosed herein are methods, wherein the nucleic acid molecules comprise a nucleotide sequence that comprises SEQ ID NO: 30. Further disclosed herein are methods, wherein the nucleic acid molecules comprise a nucleotide sequence that comprises SEQ ID NO: 31. Further disclosed herein are methods, wherein the nucleic acid molecules comprise a nucleotide sequence that comprises SEQ ID NO: 32. Further disclosed herein are methods, wherein the nucleic acid molecules comprise a nucleotide sequence that comprises SEQ ID NO: 33. Further disclosed herein are methods, wherein the nucleic acid molecules comprise a nucleotide sequence that comprises SEQ ID NO: 34. Further disclosed herein are methods, wherein the nucleic acid molecules comprise a nucleotide sequence that comprises SEQ ID NO: 35. Further disclosed herein are methods, wherein the nucleic acid molecules comprise a nucleotide sequence that comprises SEQ ID NO: 36. Further disclosed herein are methods, wherein the nucleic acid molecules comprise a nucleotide sequence that comprises SEQ ID NO: 37. Further disclosed herein are methods, wherein the nucleic acid molecules comprise a nucleotide sequence that comprises SEQ ID NO: 38. Further disclosed herein are methods, wherein the nucleic acid molecules comprise a nucleotide sequence that comprises SEQ ID NO: 39. Further disclosed herein are methods,wherein the nucleic acid molecules comprise a nucleotide sequence that comprises SEQ ID NO: 40. Further disclosed herein are methods, the nucleic acid molecules comprise a nucleotide sequence that comprises SEQ ID NO: 41. Further disclosed herein are methods, wherein the nucleic acid molecules comprise a nucleotide sequence that comprises SEQ ID NO: 42.
Further disclosed herein are methods, wherein the nucleic acid molecules comprise a nucleotide sequence that comprises SEQ ID NO: 43. Further disclosed herein are methods, wherein the nucleic acid molecules comprise a nucleotide sequence that comprises SEQ ID NO: 44. Further disclosed herein are methods, the nucleic acid molecules comprise a nucleotide sequence that comprises SEQ ID NO: 45. Further disclosed herein are methods, wherein the nucleic acid molecules comprise a nucleotide sequence that comprises SEQ ID NO: 46.
Further disclosed herein are methods, wherein the nucleic acid molecules comprise a nucleotide sequence that comprises SEQ ID NO: 47. Further disclosed herein are methods, wherein the nucleic acid molecules comprise a nucleotide sequence that comprises SEQ ID NO: 48. Further disclosed herein are methods, wherein the nucleic acid molecules comprise a nucleotide sequence that comprises SEQ ID NO: 49. Further disclosed herein are methods, wherein the nucleic acid molecules comprise a nucleotide sequence that comprises SEQ ID NO: 50. Further disclosed herein are methods, wherein the nucleic acid molecules comprise a nucleotide sequence that comprises SEQ ID NO: 51. Further disclosed herein are methods, wherein the nucleic acid molecules comprise a nucleotide sequence that comprises SEQ ID NO: 52. Further disclosed herein are methods, wherein the nucleic acid molecules comprise a nucleotide sequence that comprises SEQ ID NO: 53. Further disclosed herein are methods, wherein the nucleic acid molecules comprise a nucleotide sequence that comprises SEQ ID NO: 54.
Further disclosed herein are methods, wherein the nucleic acid molecules comprise a nucleotide sequence that comprises SEQ ID NO: 55. Further disclosed herein are methods, wherein the nucleic acid molecules comprise a nucleotide sequence that comprises SEQ ID NO: 56. Further disclosed herein are methods, wherein the nucleic acid molecules comprise a nucleotide sequence that comprises SEQ ID NO: 57. Further disclosed herein are methods, wherein the nucleic acid molecules comprise a nucleotide sequence that comprises SEQ ID NO: 58. Further disclosed herein are methods, wherein the nucleic acid molecules comprise a nucleotide sequence that comprises SEQ ID NO: 59. Further disclosed herein are methods, wherein the nucleic acid molecules comprise a nucleotide sequence that comprises SEQ ID NO: 60. Further disclosed herein are methods, wherein the nucleic acid molecules comprise a nucleotide sequence that comprises SEQ ID NO: 61. Further disclosed herein are methods, wherein the nucleic acid molecules comprise a nucleotide sequence that comprises SEQ ID NO: 62.
Further disclosed herein are methods, wherein the nucleic acid molecules comprise a nucleotide sequence that comprises SEQ ID NO: 63. Further disclosed herein are methods, wherein the nucleic acid molecules comprise a nucleotide sequence that comprises SEQ ID NO: 64. Further disclosed herein are methods, wherein the nucleic acid molecules comprise a nucleotide sequence that comprises SEQ ID NO: 65. Further disclosed herein are methods, wherein the nucleic acid molecules comprise a nucleotide sequence that comprises SEQ ID NO: 66. Further disclosed herein are methods, wherein the nucleic acid molecules comprise a nucleotide sequence that comprises SEQ ID NO: 67. Further disclosed herein are methods, wherein the nucleic acid molecules comprise a nucleotide sequence that comprises SEQ ID NO: 68. Further disclosed herein are methods, wherein the nucleic acid molecules comprise a nucleotide sequence that comprises SEQ ID NO: 69. Further disclosed herein are methods, wherein the nucleic acid molecules comprise a nucleotide sequence that comprises SEQ ID NO: 70.
Further disclosed herein are methods, wherein the nucleic acid molecules comprise a nucleotide sequence that comprises SEQ ID NO: 71 Further disclosed herein are methods, wherein the nucleic acid molecules comprise a nucleotide sequence that comprises SEQ ID NO: 72. Further disclosed herein are methods, wherein the nucleic acid molecules comprise a nucleotide sequence that comprises SEQ ID NO: 73. Further disclosed herein are methods, wherein the nucleic acid molecules comprise a nucleotide sequence that comprises SEQ ID NO: 74.
Further disclosed herein are methods, wherein the nucleic acid molecules comprise a nucleotide sequence that comprises SEQ ID NO: 75. Further disclosed herein are methods,wherein the nucleic acid molecules comprise a nucleotide sequence that comprises SEQ ID NO: 76. Further disclosed herein are methods, wherein the nucleic acid molecules comprise a nucleotide sequence that comprises SEQ ID NO: 77. Further disclosed herein are methods, wherein the nucleic acid molecules comprise a nucleotide sequence that comprises SEQ ID NO: 78.
Further disclosed herein are methods, wherein the nucleic acid molecules comprise a nucleotide sequence that comprises SEQ ID NO: 79. Further disclosed herein are methods, wherein the nucleic acid molecules comprise a nucleotide sequence that comprises SEQ ID NO: 80. Further disclosed herein are methods, wherein the nucleic acid molecules comprise a nucleotide sequence that comprises SEQ 10 NO: 81. Further disclosed herein are methods, wherein the nucleic acid molecules comprise a nucleotide sequence that comprises SEQ ID NO: 82.
Further disclosed herein are methods, wherein the nucleic acid molecules comprise a nucleotide sequence that comprises SEQ ID NO: 83. Further disclosed herein are methods of the present invention, the nucleic acid molecules comprise a nucleotide sequence that comprises SEQ ID NO: 84. Disclosed herein are methods, wherein the nucleic acid molecules comprise a nucleotide sequence that comprises SEQ ID NO: 85. Disclosed herein are methods, wherein the nucleic acid molecules comprise a nucleotide sequence that comprises SEQ ID NO: 86. Disclosed herein are methods, wherein the nucleic acid molecules comprise a nucleotide sequence that comprises SEQ ID NO: 87. Disclosed herein are methods, wherein the nucleic acid molecules comprise a nucleotide sequence that comprises SEPA ID NO: 88. Disclosed herein are methods, wherein the nucleic acid molecules comprise a nucleotide sequence that comprises SEQ ID NO: 89. Disclosed herein are methods, wherein the nucleic acid molecules comprise a nucleotide sequence that comprises SEQ 10 NO: 90.
Disclosed herein are methods, wherein the nucleic acid molecules comprise a nucleotide sequence that comprises SEQ ID NO: 91. Disclosed herein are methods, wherein the nucleic acid molecules comprise a nucleotide sequence that comprises SEQ ID NO: 92. Disclosed herein are methods, wherein the nucleic acid molecules comprise a nucleotide sequence that comprises SEQ ID NO: 93. Disclosed herein are methods, wherein the nucleic acid molecules comprise a nucleotide sequence that comprises SEQ ID NO: 94.
Disclosed herein are methods, wherein the nucleic acid molecules comprise a nucleotide sequence that comprises SEQ ID NO: 95. Disclosed herein are methods, wherein the nucleic acid molecules comprise a nucleotide sequence that comprises SEQ ID NO: 96. Disclosed herein are methods, wherein the nucleic acid molecules comprise a nucleotide sequence that comprises SEQ ID NO: 97. Disclosed herein are methods, wherein the nucleic acid molecules comprise a nucleotide sequence that comprises SEQ ID NO: 98.
Disclosed herein are methods, wherein the nucleic acid molecules comprise a nucleotide sequence that comprises SEQ ID NO: 99. Disclosed herein are methods, wherein the nucleic acid molecules comprise a nucleotide sequence that comprises SEQ ID NO: 100. Disclosed herein are methods, wherein the nucleic acid molecules comprise a nucleotide sequence that comprises SEQ ID NO: 101. Disclosed herein are methods, wherein the nucleic acid molecules comprise a nucleotide sequence that comprises SEQ ID NO: 102. Disclosed herein are methods, wherein the nucleic acid molecules comprise a nucleotide sequence that comprises SEQ ID NO: 103. Disclosed herein are methods, wherein the nucleic acid molecules comprise a nucleotide sequence that comprises SEQ ID NO: 104. Disclosed herein are methods, wherein the nucleic acid molecules comprise a nucleotide sequence that comprises SEQ ID NO: 105. Disclosed herein are methods, wherein the nucleic acid molecules comprise a nucleotide sequence that comprises SEQ ID NO: 106. Disclosed herein are methods, wherein the nucleic acid molecules comprise a nucleotide sequence that comprises SEQ ID NO: 107. Disclosed herein are methods, wherein the nucleic acid molecules comprise a nucleotide sequence that comprises SEQ ID NO: 108. Disclosed herein are methods, wherein the nucleic acid molecules comprise a nucleotide sequence that comprises SEQ ID NO: 109. Disclosed herein are methods, wherein the nucleic acid molecules comprise a nucleotide sequence that comprises SEQ ID NO: 110. Disclosed herein are methods, wherein the nucleic acid molecules comprise a nucleotide sequence that comprises SEQ ID NO: 114. Disclosed herein are methods, wherein the nucleic acid molecules comprise a nucleotide sequence that comprises SEQ ID NO: 115. Disclosed herein are methods, wherein the nucleic acid molecules comprise a nucleotide sequence that comprises SEQ ID NO: 116. Disclosed herein are methods, wherein the nucleic acid molecules comprise a nucleotide sequence that comprises SEQ ID NO: 117. Disclosed herein are methods, wherein the nucleic acid molecules comprise a nucleotide sequence that comprises SEQ ID NO: 118. Disclosed herein are methods, wherein the nucleic acid molecules comprise a nucleotide sequence that comprises SEQ ID NO: 119. Disclosed herein are methods, wherein the nucleic acid molecules comprise a nucleotide sequence that comprises SEQ ID NO: 120. Disclosed herein are methods, wherein the nucleic acid molecules comprise a nucleotide sequence that comprises SEQ ID NO: 121. Disclosed herein are methods, wherein the nucleic acid molecules comprise a nucleotide sequence that comprises SEQ ID NO: 122. Disclosed herein are methods, wherein the nucleic acid molecules comprise a nucleotide sequence that comprises SEQ ID NO: 123. Disclosed herein are methods, wherein the nucleic acid molecules comprise a nucleotide sequence that comprises SEQ ID NO: 124. Disclosed herein are methods, wherein the nucleic acid molecules comprise a nucleotide sequence that comprises SEQ ID NO: 125. Disclosed herein are methods, wherein the nucleic acid molecules comprise a nucleotide sequence that comprises SEQ ID NO: 126. Disclosed herein are methods, wherein the nucleic acid molecules comprise a nucleotide sequence that comprises SEQ ID NO: 127. Disclosed herein are methods, wherein the nucleic acid molecules comprise a nucleotide sequence that comprises SEQ ID NO: 128. Disclosed herein are methods, wherein the nucleic acid molecules comprise a nucleotide sequence that comprises SEQ ID NO: 129. Disclosed herein are methods, wherein the nucleic acid molecules comprise a nucleotide sequence that comprises SEQ ID NO: 130. Disclosed herein are methods, wherein the nucleic acid molecules comprise a nucleotide sequence that comprises SEQ ID NO: 131. Disclosed herein are methods, wherein the nucleic acid molecules comprise a nucleotide sequence that comprises SEQ ID NO: 132. Disclosed herein are methods, wherein the nucleic acid molecules comprise a nucleotide sequence that comprises SEQ ID NO: 133. Disclosed herein are methods, wherein the nucleic acid molecules comprise a nucleotide sequence that comprises SEQ ID NO: 134. Disclosed herein are methods, wherein the nucleic acid molecules comprise a nucleotide sequence that comprises SEQ ID NO: 135. Disclosed herein are methods, wherein the nucleic acid molecules comprise nucleotide sequences selected from SEQ ID NO: 1; SEQ ID NO: 2; SEQ ID NO: 3; SEQ ID NO: 4; SEQ ID NO: 5; SEQ ID NO: 6; SEQ ID NO: 7; SEQ ID NO: 8; SEQ ID NO: 9; SEQ ID NO: 10; SEQ ID NO: 11; SEQ ID NO: 12; SEQ ID NO: 13; SEQ ID NO: 14; SEQ ID NO: 15; SEQ ID NO: 16; SEQ ID NO: 17; SEQ ID NO: 18; SEQ ID NO: 19; SEQ ID NO: 20; SEQ ID NO: 21; SEQ ID NO: 22; SEQ ID NO: 23; SEQ ID NO: 24; SEQ ID NO: 25; SEQ ID NO: 26; SEQ ID NO: 27; SEQ ID NO: 28; SEQ ID NO: 29; SEQ ID NO: 30; SEQ ID NO: 31; SEQ ID NO: 32; SEQ ID NO: 33; SEQ ID NO: 34; SEQ ID NO: 35; SEQ ID NO: 36; SEQ ID NO: 37; SEQ ID NO: 38; SEQ ID NO: 39; SEQ ID NO: 40; SEQ ID NO: 41; SEQ ID NO: 42; SEQ ID NO: 43; SEQ ID NO: 44; SEQ ID NO: 45; SEQ ID NO: 46; SEQ ID NO: 47; SEQ ID NO: 48; SEQ ID NO: 49; SEQ ID NO: 50; SEQ ID NO: 51; SEQ ID NO: 52; SEQ ID NO: 53; SEQ ID NO: 54; SEQ ID NO: 55; SEQ ID NO: 56; SEQ ID NO: 57; SEQ ID NO: 58; SEQ ID NO: 59; SEQ ID NO: 60; SEQ ID NO: 61; SEQ ID NO: 62; SEQ ID NO: 63; SEQ ID NO: 64; SEQ ID NO: 65; SEQ ID NO: 66; SEQ ID NO: 67; SEQ ID NO: 68; SEQ ID NO: 69; SEQ ID NO: 70; SEQ ID NO: 71; SEQ ID NO: 72; SEQ ID NO: 73; SEQ ID NO: 74; SEQ ID NO: 75; SEQ ID NO: 76; SEQ ID NO: 77; SEQ ID NO: 78; SEQ ID NO: 79; SEQ ID NO: 80; SEQ ID NO: 81; SEQ ID NO: 82; SEQ ID NO: 83; SEQ ID NO: 84; SEQ ID NO: 85; SEQ ID NO: 86; SEQ ID NO: 87; SEQ ID NO: 88; SEQ ID NO: 89; SEQ ID NO: 90; SEQ ID NO: 91; SEQ ID NO: 92; SEQ ID NO: 93; SEQ ID NO: 94; SEQ ID NO: 95; SEQ ID NO: 96; SEQ ID NO: 97; SEQ ID NO: 98; SEQ ID NO: 99; SEQ ID NO: 100; SEQ ID NO: 101; SEQ ID NO: 102; SEQ ID NO: 103; SEQ ID NO: 104; SEQ ID NO: 105; SEQ ID NO: 106; SEQ ID NO: 107; SEQ ID NO: 108; SEQ ID NO: 109; SEQ ID NO: 110; SEQ ID NO: 114; SEQ ID NO: 115; SEQ ID NO: 116; SEQ ID NO: 117; SEQ ID NO: 118; SEQ ID NO: 119; SEQ ID NO: 120; SEQ ID NO: 121; SEQ ID NO: 122; SEQ ID NO: 123; SEQ ID NO: 124; SEQ ID NO: 125; SEQ ID NO: 126; SEQ ID NO: 127; SEQ ID NO: 128; SEQ ID NO: 129; SEQ ID NO: 130; SEQ ID NO: 131; SEQ ID NO: 132; SEQ ID NO: 133; SEQ ID NO: 134; SEQ ID NO: 135; and combinations thereof. Disclosed herein are methods , wherein the nucleic acid molecules comprise a nucleotide sequence that comprises SEQ ID NO: I in shNA format. Disclosed herein are methods , wherein the nucleic acid molecules comprise a nucleotide sequence that comprises SEQ ID NO: 2 in shNA format. Disclosed herein are methods , wherein the nucleic acid molecules comprise a nucleotide sequence that comprises SEQ ID NO: 3 in shNA format. Disclosed herein are methods , wherein the nucleic acid molecules comprise a nucleotide sequence that comprises SEQ ID NO: 4 in shNA format. Disclosed herein are methods , wherein the nucleic acid molecules comprise a nucleotide sequence that comprises SEQ ID NO: 5 in shNA format. Disclosed herein are methods , wherein the nucleic acid molecules comprise a nucleotide sequence that comprises SEQ ID NO: 6 in shNA format. Disclosed herein are methods , wherein the nucleic acid molecules comprise a nucleotide sequence that comprises SEQ ID NO: 7 in shNA format. Disclosed herein are methods , wherein the nucleic acid molecules comprise a nucleotide sequence that comprises SEQ ID NO: 8 in shNA format. Disclosed herein are methods , wherein the nucleic acid molecules comprise a nucleotide sequence that comprises SEQ ID NO: 9 in shNA format. Disclosed herein are methods , wherein the nucleic acid molecules comprise a nucleotide sequence that comprises SEQ ID NO: 10 in shNA format. Disclosed herein are methods , wherein the nucleic acid molecules comprise a nucleotide sequence that comprises SEQ ID NO: 11 in shNA format. Disclosed herein are methods , wherein the nucleic acid molecules comprise a nucleotide sequence that comprises SEQ ID NO: 12 in shNA format. Disclosed herein are methods , wherein the nucleic acid molecules comprise a nucleotide sequence that comprises SEQ ID NO: 13 in shNA format. Disclosed herein are methods , wherein the nucleic acid molecules comprise a nucleotide sequence that comprises SEQ ID NO: 14 in shNA format. Disclosed herein are methods , wherein the nucleic acid molecules comprise a nucleotide sequence that comprises SEQ ID NO: 15 in shNA format. Disclosed herein are methods , wherein the nucleic acid molecules comprise a nucleotide sequence that comprises SEQ ID NO: 16 in shNA format. Disclosed herein are methods , wherein the nucleic acid molecules comprise a nucleotide sequence that comprises SEQ ID NO: 17 in shNA format. Disclosed herein are methods , wherein the nucleic acid molecules comprise a nucleotide sequence that comprises SEQ ID NO: 18 in shNA format. Disclosed herein are methods , wherein the nucleic acid molecules comprise a nucleotide sequence that comprises SEQ ID NO: 19 in shNA format. Disclosed herein are methods , wherein the nucleic acid molecules comprise a nucleotide sequence that comprises SEQ ID NO: 20 in shNA format. Disclosed herein are methods , wherein the nucleic acid molecules comprise a nucleotide sequence that comprises SEQ ID NO: 21 in shNA format. Disclosed herein are methods , wherein the nucleic acid molecules comprise a nucleotide sequence that comprises SEQ ID NO: 22 in shNA format. Disclosed herein are methods , wherein the nucleic acid molecules comprise a nucleotide sequence that comprises SEQ ID NO: 23 in shNA format. Disclosed herein are methods , wherein the nucleic acid molecules comprise a nucleotide sequence that comprises SEQ ID NO: 24 in shNA format. Disclosed herein are methods , wherein the nucleic acid molecules comprise a nucleotide sequence that comprises SEQ ID NO: 25 in shNA format. Disclosed herein are methods , wherein the nucleic acid molecules comprise a nucleotide sequence that comprises SEQ ID NO: 26 in shNA format. Disclosed herein are methods , wherein the nucleic acid molecules comprise a nucleotide sequence that comprises SEQ ID NO: 27 in shNA format. Disclosed herein are methods , wherein the nucleic acid molecules comprise a nucleotide sequence that comprises SEQ ID NO: 28 in shNA format. Disclosed herein are methods , wherein the nucleic acid molecules comprise a nucleotide sequence that comprises SEQ ID NO: 29 in shNA format. Disclosed herein are methods , wherein the nucleic acid molecules comprise a nucleotide sequence that comprises SEQ ID NO: 30 in shNA format. Disclosed herein are methods , wherein the nucleic acid molecules comprise a nucleotide sequence that comprises SEQ ID NO: 31 in shNA format. Disclosed herein are methods , wherein the nucleic acid molecules comprise a nucleotide sequence that comprises SEQ ID NO: 32 in shNA format. Disclosed herein are methods , wherein the nucleic acid molecules comprise a nucleotide sequence that comprises SEQ ID NO: 33 in shNA format. Disclosed herein are methods , wherein the nucleic acid molecules comprise a nucleotide sequence that comprises SEQ ID NO: 34 in shNA format. Disclosed herein are methods , wherein the nucleic acid molecules comprise a nucleotide sequence that comprises SEQ ID NO: 35 in shNA format. Disclosed herein are methods , wherein the nucleic acid molecules comprise a nucleotide sequence that comprises SEQ ID NO: 36 in shNA format. Disclosed herein are methods , wherein the nucleic acid molecules comprise a nucleotide sequence that comprises SEQ ID NO: 37 in shNA format. Disclosed herein are methods , wherein the nucleic acid molecules comprise a nucleotide sequence that comprises SEQ ID NO: 38 in shNA format. Disclosed herein are methods , wherein the nucleic acid molecules comprise a nucleotide sequence that comprises SEQ ID NO: 39 in shNA format. Disclosed herein are methods , wherein the nucleic acid molecules comprise a nucleotide sequence that comprises SEQ ID NO: 40 in shNA format. Disclosed herein are methods , wherein the nucleic acid molecules comprise a nucleotide sequence that comprises SEQ ID NO: 41 in shNA format. Disclosed herein are methods , wherein the nucleic acid molecules comprise a nucleotide sequence that comprises SEQ ID NO: 42 in shNA format. Disclosed herein are methods , wherein the nucleic acid molecules comprise a nucleotide sequence that comprises SEQ ID NO: 43 in shNA format. Disclosed herein are methods , wherein the nucleic acid molecules comprise a nucleotide sequence that comprises SEQ ID NO: 44 in shNA format. Disclosed herein are methods , wherein the nucleic acid molecules comprise a nucleotide sequence that comprises SEQ ID NO: 45 in shNA format. Disclosed herein are methods , wherein the nucleic acid molecules comprise a nucleotide sequence that comprises SEQ ID NO: 46 in shNA format. Disclosed herein are methods , wherein the nucleic acid molecules comprise a nucleotide sequence that comprises SEQ ID NO: 47 in shNA format. Disclosed herein are methods , wherein the nucleic acid molecules comprise a nucleotide sequence that comprises SEQ ID NO: 48 in shNA format. Disclosed herein are methods , wherein the nucleic acid molecules comprise a nucleotide sequence that comprises SEQ ID NO: 49 in shNA format. Disclosed herein are methods , wherein the nucleic acid molecules comprise a nucleotide sequence that comprises SEQ ID NO: 50 in shNA format. Disclosed herein are methods , wherein the nucleic acid molecules comprise a nucleotide sequence that comprises SEQ ID NO: 51 in shNA format. Disclosed herein are methods , wherein the nucleic acid molecules comprise a nucleotide sequence that comprises SEQ ID NO: 52 in shNA format. Disclosed herein are methods , wherein the nucleic acid molecules comprise a nucleotide sequence that comprises SEQ ID NO 53 in shNA format. Disclosed herein are methods , wherein the nucleic acid molecules comprise a nucleotide sequence that comprises SEQ ID NO: 54 in shNA format. Disclosed herein are methods , wherein the nucleic acid molecules comprise a nucleotide sequence that comprises SEQ ID NO: 55 in shNA format. Disclosed herein are methods , wherein the nucleic acid molecules comprise a nucleotide sequence that comprises SEQ ID NO: 56 in shNA format. Disclosed herein are methods , wherein the nucleic acid molecules comprise a nucleotide sequence that comprises SEQ ID NO: 57 in shNA format. Disclosed herein are methods , wherein the nucleic acid molecules comprise a nucleotide sequence that comprises SEQ ID NO: 58 in shNA format. Disclosed herein are methods , wherein the nucleic acid molecules comprise a nucleotide sequence that comprises SEQ ID NO: 59 in shNA format. Disclosed herein are methods , wherein the nucleic acid molecules comprise a nucleotide sequence that comprises SEQ ID NO: 60 in shNA format. Disclosed herein are methods , wherein the nucleic acid molecules comprise a nucleotide sequence that comprises SEQ ID NO: 61 in shNA format. Disclosed herein are methods , wherein the nucleic acid molecules comprise a nucleotide sequence that comprises SEQ ID NO: 62 in shNA format. Disclosed herein are methods , wherein the nucleic acid molecules comprise a nucleotide sequence that comprises SEQ ID NO: 63 in shNA format. Disclosed herein are methods , wherein the nucleic acid molecules comprise a nucleotide sequence that comprises SEQ ID NO: 64 in shNA format. Disclosed herein are methods , wherein the nucleic acid molecules comprise a nucleotide sequence that comprises SEQ ID NO: 65 in shNA format. Disclosed herein are methods , wherein the nucleic acid molecules comprise a nucleotide sequence that comprises SEQ ID NO: 66 in shNA format. Disclosed herein are methods , wherein the nucleic acid molecules comprise a nucleotide sequence that comprises SEQ ID NO: 67 in shNA format. Disclosed herein are methods , wherein the nucleic acid molecules comprise a nucleotide sequence that comprises SEQ ID NO: 68 in shNA format. Disclosed herein are methods , wherein the nucleic acid molecules comprise a nucleotide sequence that comprises SEQ ID NO: 69 in shNA format. Disclosed herein are methods , wherein the nucleic acid molecules comprise a nucleotide sequence that comprises SEQ ID NO: 70 in shNA format. Disclosed herein are methods , wherein the nucleic acid molecules comprise a nucleotide sequence that comprises SEQ ID NO: 71 in shNA format. Disclosed herein are methods , wherein the nucleic acid molecules comprise a nucleotide sequence that comprises SEQ ID NO: 72 in shNA format. Disclosed herein are methods , wherein the nucleic acid molecules comprise a nucleotide sequence that comprises SEQ ID NO: 73 in shNA format. Disclosed herein are methods , wherein the nucleic acid molecules comprise a nucleotide sequence that comprises SEQ ID NO: 74 in shNA format. Disclosed herein are methods , wherein the nucleic acid molecules comprise a nucleotide sequence that comprises SEQ ID NO: 75 in shNA format. Disclosed herein are methods , wherein the nucleic acid molecules comprise a nucleotide sequence that comprises SEQ ID NO: 76 in shNA format. Disclosed herein are methods , wherein the nucleic acid molecules comprise a nucleotide sequence that comprises SEQ ID NO: 77 in shNA format. Disclosed herein are methods , wherein the nucleic acid molecules comprise a nucleotide sequence that comprises SEQ ID NO: 78 in shNA format. Disclosed herein are methods , wherein the nucleic acid molecules comprise a nucleotide sequence that comprises SEQ ID NO: 79 in shNA format. Disclosed herein are methods , wherein the nucleic acid molecules comprise a nucleotide sequence that comprises SEQ ID NO: 80 in shNA format. Disclosed herein are methods , wherein the nucleic acid molecules comprise a nucleotide sequence that comprises SEQ ID NO: 81 in shNA format. Disclosed herein are methods , wherein the nucleic acid molecules comprise a nucleotide sequence that comprises SEQ ID NO: 82 in shNA format. Disclosed herein are methods , wherein the nucleic acid molecules comprise a nucleotide sequence that comprises SEQ ID NO: 83 in shNA format. Disclosed herein are methods , wherein the nucleic acid molecules comprise a nucleotide sequence that comprises SEQ ID NO: 84 in shNA format. Disclosed herein are methods , wherein the nucleic acid molecules comprise a nucleotide sequence that comprises SEQ ID NO: 85 in shNA format. Disclosed herein are methods , wherein the nucleic acid molecules comprise a nucleotide sequence that comprises SEQ ID NO: 86 in shNA format. Disclosed herein are methods , wherein the nucleic acid molecules comprise a nucleotide sequence that comprises SEQ ID NO: 87 in shNA format. Disclosed herein are methods , wherein the nucleic acid molecules comprise a nucleotide sequence that comprises SEQ ID NO: 88 in shNA format. Disclosed herein are methods , wherein the nucleic acid molecules comprise a nucleotide sequence that comprises SEQ ID NO: 89 in shNA format. Disclosed herein are methods , wherein the nucleic acid molecules comprise a nucleotide sequence that comprises SEQ ID NO: 90 in shNA format. Disclosed herein are methods , wherein the nucleic acid molecules comprise a nucleotide sequence that comprises SEQ ID NO: 91 in shNA format. Disclosed herein are methods , wherein the nucleic acid molecules comprise a nucleotide sequence that comprises SEQ ID NO: 92 in shNA format. Disclosed herein are methods , wherein the nucleic acid molecules comprise a nucleotide sequence that comprises SEQ ID NO: 93 in shNA format. Disclosed herein are methods , wherein the nucleic acid molecules comprise a nucleotide sequence that comprises SEO ID NO: 94 in shNA format. Disclosed herein are methods , wherein the nucleic acid molecules comprise a nucleotide sequence that comprises SEQ ID NO: 95 in shNA format. Disclosed herein are methods , wherein the nucleic acid molecules comprise a nucleotide sequence that comprises SEQ ID NO: 96 in shNA format. Disclosed herein are methods , wherein the nucleic acid molecules comprise a nucleotide sequence that comprises SEQ ID NO: 97 in shNA format. Disclosed herein are methods , wherein the nucleic acid molecules comprise a nucleotide sequence that comprises SEQ ID NO: 98 in shNA format. Disclosed herein are methods , wherein the nucleic acid molecules comprise a nucleotide sequence that comprises SEQ ID NO: 99 in shNA format. Disclosed herein are methods , wherein the nucleic acid molecules comprise a nucleotide sequence that comprises SEQ ID NO: 100 in shNA format. Disclosed herein are methods , wherein the nucleic acid molecules comprise a nucleotide sequence that comprises SEQ ID NO: 101 in shNA format. Disclosed herein are methods , wherein the nucleic acid molecules comprise a nucleotide sequence that comprises SEQ ID NO: 102 in shNA format. Disclosed herein are methods , wherein the nucleic acid molecules comprise a nucleotide sequence that comprises SEQ ID NO: 103 in shNA format. Disclosed herein are methods , wherein the nucleic acid molecules comprise a nucleotide sequence that comprises SEQ ID NO: 104 in shNA format. Disclosed herein are methods , wherein the nucleic acid molecules comprise a nucleotide sequence that comprises SEQ ID NO: 105 in shNA format. Disclosed herein are methods , wherein the nucleic acid molecules comprise a nucleotide sequence that comprises SEQ ID NO: 106 in shNA format. Disclosed herein are methods , wherein the nucleic acid molecules comprise a nucleotide sequence that comprises SEQ ID NO: 107 in shNA format. Disclosed herein are methods , wherein the nucleic acid molecules comprise a nucleotide sequence mat comprises SEQ ID NO: 108 in shNA format. Disclosed herein are methods , wherein the nucleic acid molecules comprise a nucleotide sequence that comprises SEQ ID NO: 109 in shNA format. Disclosed herein are methods , wherein the nucleic acid molecules comprise a nucleotide sequence that comprises SEQ ID NO: 110 in shNA format. Disclosed herein are methods , wherein the nucleic acid molecules comprise a nucleotide sequence that comprises SEQ ID NO: 114 in shNA format. Disclosed herein are methods , wherein the nucleic acid molecules comprise a nucleotide sequence that comprises SEQ ID NO: 115 in shNA format. Disclosed herein are methods , wherein the nucleic acid molecules comprise a nucleotide sequence that comprises SEQ ID NO: 116 in shNA format. Disclosed herein are methods , wherein the nucleic acid molecules comprise a nucleotide sequence that comprises SEQ ID NO: 117 in shNA format. Disclosed herein are methods , wherein the nucleic acid molecules comprise a nucleotide sequence that comprises SEQ ID NO: 118 in shNA format. Disclosed herein are methods , wherein the nucleic acid molecules comprise a nucleotide sequence that comprises SEQ ID NO: 119 in shNA format. Disclosed herein are methods , wherein the nucleic acid molecules comprise a nucleotide sequence that comprises SEQ ID NO: 120 in shNA format. Disclosed herein are methods , wherein the nucleic acid molecules comprise a nucleotide sequence that comprises SEQ ID NO: 121 in shNA format. Disclosed herein are methods , wherein the nucleic acid molecules comprise a nucleotide sequence that comprises SEQ ID NO: 122 in shNA format. Disclosed herein are methods , wherein the nucleic acid molecules comprise a nucleotide sequence that comprises SEQ ID NO: 123 in shNA format. Disclosed herein are methods , wherein the nucleic acid molecules comprise a nucleotide sequence that comprises SEQ ID NO: 124 in shNA format. Disclosed herein are methods , wherein the nucleic acid molecules comprise a nucleotide sequence that comprises SEQ ID NO: 125 in shNA format. Disclosed herein are methods , wherein the nucleic acid molecules comprise a nucleotide sequence that comprises SEQ ID NO: 126 in shNA format. Disclosed herein are methods , wherein the nucleic acid molecules comprise a nucleotide sequence that comprises SEQ ID NO: 127 in shNA format. Disclosed herein are methods , wherein the nucleic acid molecules comprise a nucleotide sequence that comprises SEQ ID NO: 128 in shNA format. Disclosed herein are methods , wherein the nucleic acid molecules comprise a nucleotide sequence that comprises SEQ ID NO: 129 in shNA format. Disclosed herein are methods , wherein the nucleic acid molecules comprise a nucleotide sequence that comprises SEQ ID NO: 130 in shNA format. Disclosed herein are methods , wherein the nucleic acid molecules comprise a nucleotide sequence that comprises SEQ ID NO: 131 in shNA format. Disclosed herein are methods , wherein the nucleic acid molecules comprise a nucleotide sequence that comprises SEQ ID NO: 132 in shNA format. Disclosed herein are methods , wherein the nucleic acid molecules comprise a nucleotide sequence that comprises SEQ ID NO: 133 in shNA format. Disclosed herein are methods , wherein the nucleic acid molecules comprise a nucleotide sequence that comprises SEQ ID NO: 134 in shNA format. Disclosed herein are methods , wherein the nucleic acid molecules comprise a nucleotide sequence that comprises SEQ ID NO: 135 in shNA format. In another embodiment of the methods of the present invention, the nucleic acid molecules comprise nucleotide sequences selected from the group consisting of SEQ ID NO: 3; SEQ ID NO: 4, SEQ ID NO: 7; SEQ ID NO: 8; SEQ ID NO: 13; SEQ ID NO: 14, SEQ ID NO: 15; SEQ ID NO: 16, and combinations thereof in shNA format. Further disclosed herein is the nucleic acid molecule comprising nucleotide sequences selected from the group consisting of SEQ ID NO: 1; SEQ ID NO: 2; SEQ ID NO: 5; SEQ ID NO: 6; SEQ ID NO: 9; SEQ ID NO: 10; SEQ ID NO: 11; SEQ ID NO: 12; SEQ ID NO: 17; SEQ ID NO: 18; SEQ ID NO: 19; SEQ ID NO: 20; SEQ ID NO: 21; SEQ ID NO: 22; SEQ ID NO: 23; SEQ ID NO: 24; SEQ ID NO: 25; SEQ ID NO: 26; SEQ ID NO: 27; SEQ ID NO: 28; SEQ ID NO: 29; SEQ ID NO: 30; SEQ ID NO: 31; SEQ ID NO: 32; SEQ ID NO: 33; SEQ ID NO: 34; SEQ ID NO: 35; SEQ ID NO: 36; SEQ ID NO: 37; SEQ ID NO: 38; SEQ ID NO: 39; SEQ ID NO: 40; SEQ ID NO: 41; SEQ ID NO: 42; SEQ ID NO: 43; SEQ ID NO: 44; SEQ ID NO: 45; SEQ ID NO: 46; SEQ ID NO: 47; SEQ ID NO: 48; SEQ ID NO: 49; SEQ ID NO: 50; SEQ ID NO: 51; SEQ ID NO: 52; SEQ ID NO: 53; SEQ ID NO: 54; SEQ ID NO: 55; SEQ ID NO: 56; SEQ ID NO: 57; SEQ ID NO: 58; SEQ ID NO: 59; SEQ ID NO: 60; SEQ ID NO: 61; SEQ ID NO: 62; SEQ ID NO: 63; SEQ ID NO: 64; SEQ ID NO: 65; SEQ ID NO: 66; SEQ ID NO: 67; SEQ ID NO: 68; SEQ ID NO: 69; SEQ ID NO: 70; SEQ ID NO: 71; SEQ ID NO: 72; SEQ ID NO: 73; SEQ ID NO: 74; SEQ ID NO: 75; SEQ ID NO: 76; SEQ ID NO: 77; SEQ ID NO: 78; SEQ ID NO: 79; SEQ ID NO: 80; SEQ ID NO: 81; SEQ ID NO: 82; SEQ ID NO: 83; SEQ ID NO: 84; SEQ ID NO: 85; SEQ ID NO: 86; SEQ ID NO: 87; SEQ ID NO: 88; SEQ ID NO: 89; SEQ ID NO: 90; SEQ ID NO: 91; SEQ ID NO: 92; SEQ ID NO: 93; SEQ ID NO: 94; SEQ ID NO: 95; SEQ ID NO: 96; SEQ ID NO: 97; SEQ ID NO: 98; SEQ ID NO: 99; SEQ ID NO: 100; SEQ ID NO: 101; SEQ ID NO: 102; SEQ ID NO: 103; SEQ ID NO: 104; SEQ ID NO: 105; SEQ ID NO: 106; SEQ ID NO: 107; SEQ ID NO: 108; SEQ ID NO: 109; SEQ ID NO: 110; SEQ ID NO: 114; SEQ ID NO: 115; SEQ ID NO: 116; SEQ ID NO: 117; SEQ ID NO: 118; SEQ ID NO: 119; SEQ ID NO: 120; SEQ ID NO: 121; SEQ ID NO: 122; SEQ ID NO: 123; SEQ ID NO: 124; SEQ ID NO: 125; SEQ ID NO: 126; SEQ ID NO: 127; SEQ ID NO: 128; SEQ ID NO: 129; SEQ ID NO: 130; SEQ ID NO: 131; SEQ ID NO: 132; SEQ ID NO: 133; SEQ ID NO: 134; SEQ ID NO: 135; and combinations thereof in shNA format.

Disclosed herein are methods, wherein the nucleic acid molecules are administered to the intrathecal space of the spinal cord Further disclosed herein are methods, wherein the nucleic acid molecules are administered intracranially. Further disclosed herein are methods, wherein the nucleic acid molecules are administered to the striatum.

Administering nucleic acid sequences includes through an administration system selected from the group consisting of a depot, an infusion pump, an osmotic pump, an interbody pump, a catheter, and combinations thereof. One additional embodiment based on present invention includes administering nucleic acid sequences of the present invention through an implanted pump that controls the delivery of the nucleic acid sequences to an area selected from the group consisting of the intrathecal space of the spinal cord; the striatum; intracranially; and combinations thereof wherein the nucleic acid sequences are delivered in a pharmaceutically effective amount to improve at least one symptom of Huntington's disease.

Also disclosed herein are nucleic acid molecules. The nucleic acid molecule comprises a nucleotide sequence that preferentially suppresses the expression of amino acid sequences encoding for mutated huntingtin ("htt") over suppressing the expression of amino acid sequences encoding for normal htt. The nucleic acid molecules may comprise a nucleotide sequence comprising SEQ ID NO: 1. The nucleic acid molecules may comprise a nucleotide sequence comprising SEQ ID NO: 2. In another embodiment of the nucleic acid molecules of the present invention, the nucleic acid molecule comprises a nucleotide sequence comprising SEQ ID NO: 3. In another embodiment of the nucleic acid molecules of the present invention, the nucleic acid molecule comprises a nucleotide sequence comprising SEQ ID NO: 4. The nucleic acid molecules may comprise a nucleotide sequence comprising SEQ ID NO: 5. The nucleic acid molecules may comprise a nucleotide sequence comprising SEQ ID NO: 6. In another embodiment of the nucleic acid molecules of the present invention, the nucleic acid molecule comprises a nucleotide sequence comprising SEQ ID NO: 7. In another embodiment of the nucleic acid molecules of the present invention, the nucleic acid molecule comprises a nucleotide sequence comprising SEQ ID NO: 8. The nucleic acid molecules may comprise a nucleotide sequence comprising SEQ ID NO: 9. The nucleic acid molecules may comprise a nucleotide sequence comprising SEQ ID NO: 10. The nucleic acid molecules may comprise a nucleotide sequence comprising SEQ ID NO: 11. The nucleic acid molecules may comprise a nucleotide sequence comprising SEQ ID NO: 12. In another embodiment of the nucleic acid molecules of the present invention, the nucleic acid molecule comprises a nucleotide sequence comprising SEQ ID NO: 13. In another embodiment of the nucleic acid molecules of the present invention, the nucleic acid molecule comprises a nucleotide sequence comprising SEQ ID NO: 14. In another embodiment of the nucleic acid molecules of the present invention, the nucleic acid molecule comprises a nucleotide sequence comprising SEQ ID NO: 15. In another embodiment of the nucleic acid molecules of the present invention, the nucleic acid molecule comprises a nucleotide sequence comprising SEQ ID NO: 16. The nucleic acid molecules may comprise a nucleotide sequence comprising SEQ ID NO: 17. The nucleic acid molecules may comprise a nucleotide sequence comprising SEQ ID NO: 18. The nucleic acid molecules may comprise a nucleotide sequence comprising SEQ ID NO: 19. The nucleic acid molecules may comprises a nucleotide sequence comprising SEQ ID NO: 20. The nucleic acid molecules may comprise a nucleotide sequence comprising SEQ ID NO: 21. The nucleic acid molecules may comprise a nucleotide sequence comprising SEQ ID NO: 22. The nucleic acid molecules may comprise a nucleotide sequence comprising SEQ ID NO: 23. The nucleic acid molecules may comprise a nucleotide sequence comprising SEQ ID NO: 24. The nucleic acid molecules may comprise a nucleotide sequence comprising SEQ ID NO: 25. The nucleic acid molecules may comprise a nucleotide sequence comprising SEQ ID NO: 26. The nucleic acid molecules may comprise a nucleotide sequence comprising SEQ ID NO: 27. The nucleic acid molecules may comprise a nucleotide sequence comprising SEQ ID NO: 28. The nucleic acid molecules may comprise a nucleotide sequence comprising SEQ ID NO: 29. The nucleic acid molecules may comprise a nucleotide sequence comprising SEQ ID NO: 30. The nucleic acid may comprise a nucleotide sequence comprising SEQ 1D NO: 31. The nucleic acid may comprise a nucleotide sequence comprising SEQ ID NO: 32. The nucleic acid may comprise a nucleotide sequence comprising SEQ ID NO: 33. The nucleic acid molecules may comprise a nucleotide sequence comprising SEQ ID NO: 34. The nucleic acid molecules may comprise a nucleotide sequence comprising SEQ ID NO: 35. The nucleic acid molecules may comprise a nucleotide sequence comprising SEQ ID NO: 36. The nucleic acid molecules may comprise a nucleotide sequence comprising SEQ ID NO: 37. The nucleic acid molecules may comprise a nucleotide sequence comprising SEQ ID NO: 38. The nucleic acid molecules may comprise a nucleotide sequence comprising SEQ ID NO: 39. The nucleic acid molecules may comprise a nucleotide sequence comprising SEQ ID NO: 40. The nucleic acid molecules may comprise a nucleotide sequence comprising SEQ ID NO: 41. The nucleic acid molecules may comprise a nucleotide sequence comprising SEQ ID NO: 42. The nucleic acid molecules may comprise a nucleotide sequence comprising SEQ ID NO: 43. The nucleic acid molecules may comprise a nucleotide sequence comprising SEQ ID NO: 44. The nucleic acid molecules may comprise a nucleotide sequence comprising SEQ ID NO: 45. The nucleic acid molecules may comprise a nucleotide sequence comprising SEQ ID NO: 46. The nucleic acid molecules may comprise a nucleotide sequence comprising SEQ ID NO: 47. The nucleic acid molecules may comprise a nucleotide sequence comprising SEQ ID NO: 48. The nucleic acid molecules may comprise a nucleotide sequence comprising SEQ ID NO: 49. The nucleic acid molecules may comprise a nucleotide sequence comprising SEQ ID NO: 50. The nucleic acid molecules may comprise a nucleotide sequence comprising SEQ ID NO: 51. The nucleic acid molecules may comprise a nucleotide sequence comprising SEQ ID NO: 52. The nucleic acid molecules may comprise a nucleotide sequence comprising SEQ ID NO: 53. The nucleic acid molecules may comprise a nucleotide sequence comprising SEQ ID NO: 54. The nucleic acid molecules may comprise a nucleotide sequence comprising SEQ ID NO: 55. The nucleic acid molecules may comprise a nucleotide sequence comprising SEQ ID NO: 56. The nucleic acid molecules may comprise a nucleotide sequence comprising SEQ ID NO: 57. The nucleic acid molecules may comprise a nucleotide sequence comprising SEQ ID NO: 58. The nucleic acid molecules may comprise a nucleotide sequence comprising SEQ ID NO: 59. The nucleic acid molecules may comprise a nucleotide sequence comprising SEQ ID NO: 60. The nucleic acid molecules may comprise a nucleotide sequence comprising SEQ ID NO: 61. The nucleic acid molecules may comprise a nucleotide sequence comprising SEQ ID NO: 62. The nucleic acid molecules may comprise a nucleotide sequence comprising SEQ ID NO: 63. The nucleic acid molecules may comprise a nucleotide sequence comprising SEQ ID NO: 64. The nucleic acid molecules may comprise a nucleotide sequence comprising SEQ ID NO: 65. The nucleic acid molecules may comprise a nucleotide sequence comprising SEQ ID NO: 66. The nucleic acid molecules may comprise a nucleotide sequence comprising SEQ ID NO: 67. The nucleic acid molecules may comprise a nucleotide sequence comprising SEQ ID NO: 68. The nucleic acid molecules may comprise a nucleotide sequence comprising SEQ ID NO: 69. The nucleic acid molecules may comprise a nucleotide sequence comprising SEQ ID NO: 70. The nucleic acid molecules may comprise a nucleotide sequence comprising SEQ ID NO: 71. The nucleic acid molecules may comprise a nucleotide sequence comprising SEQ ID NO: 72. The nucleic acid molecules may comprise a nucleotide sequence comprising SEQ ID NO: 73. The nucleic acid molecules may comprise a nucleotide sequence comprising SEQ ID NO: 74. The nucleic acid molecules may comprise a nucleotide sequence comprising SEQ ID NO: 75. The nucleic acid molecules may comprise a nucleotide sequence comprising SEQ ID NO: 76. The nucleic acid molecules may comprise a nucleotide sequence comprising SEQ ID NO: 77. The nucleic acid molecules may comprise a nucleotide sequence comprising SEQ ID NO: 78. The nucleic acid molecules may comprise a nucleotide sequence comprising SEQ ID NO: 79. The nucleic acid molecules may comprise a nucleotide sequence comprising SEQ ID NO: 80. The nucleic acid molecules may comprise a nucleotide sequence comprising SEQ ID NO: 81. The nucleic acid molecules may comprise a nucleotide sequence comprising SEQ ID NO: 82. The nucleic acid molecules may comprise a nucleotide sequence comprising SEQ ID NO: 83. The nucleic acid molecules may comprise a nucleotide sequence comprising SEQ ID NO: 84. The nucleic acid molecules may comprise a nucleotide sequence comprising SEQ ID NO: 85. The nucleic acid molecules may comprise a nucleotide sequence comprising SEQ ID NO: 86. The nucleic acid molecules may comprise a nucleotide sequence comprising SEQ ID NO: 87. The nucleic acid molecules may comprise a nucleotide sequence comprising SEQ ID NO: 88. The nucleic acid molecules may comprise a nucleotide sequence comprising SEQ ID NO: 89. The nucleic acid molecules may comprise a nucleotide sequence comprising SEQ ID NO: 90. The nucleic acid molecules may comprise a nucleotide sequence comprising SEQ ID NO: 91. The nucleic acid molecules may comprise a nucleotide sequence comprising SEQ ID NO: 92. The nucleic acid molecules may comprise a nucleotide sequence comprising SEQ ID NO: 93. The nucleic acid molecules may comprise a nucleotide sequence comprising SEQ ID NO: 94. The nucleic acid molecules may comprise a nucleotide sequence comprising SEQ ID NO: 95. The nucleic acid molecules may comprise a nucleotide sequence comprising SEQ ID NO: 96. The nucleic acid molecules may comprise a nucleotide sequence comprising SEQ ID NO: 97. The nucleic acid molecules may comprise a nucleotide sequence comprising SEQ ID NO: 98. The nucleic acid molecules may comprise a nucleotide sequence comprising SEQ ID NO: 99. The nucleic acid molecules may comprise a nucleotide sequence comprising SEQ ID NO: 100. The nucleic acid molecules may comprise a nucleotide sequence comprising SEQ ID NO: 101. The nucleic acid molecules may comprise a nucleotide sequence comprising SEQ ID NO: 102. The nucleic acid molecules may comprise a nucleotide sequence comprising SEQ ID NO: 103. The nucleic acid molecules may comprise a nucleotide sequence comprising SEQ ID NO: 104. The nucleic acid molecules may comprise a nucleotide sequence comprising SEQ ID NO: 105. The nucleic acid molecules may comprise a nucleotide sequence comprising SEQ ID NO: 106. The nucleic acid molecules may comprise a nucleotide sequence comprising SEQ ID NO:107. The nucleic acid molecules may comprise a nucleotide sequence comprising SEQ ID NO: 108. The nucleic acid molecules may comprise a nucleotide sequence comprising SEQ ID NO: 109. The nucleic acid molecules may comprise a nucleotide sequence comprising SEQ ID NO: 110. The nucleic acid molecules may comprise a nucleotide sequence comprising **SEQ** ID NO: 114. The nucleic acid molecules may comprise a nucleotide sequence comprising **SEQ** ID NO: 115. The nucleic acid molecules may comprise a nucleotide sequence comprising **SEQ** ID NO: 116. The nucleic acid molecules may comprise a nucleotide sequence comprising SEQ ID NO: 117. The nucleic acid molecules may comprise a nucleotide sequence comprising SEQ ID NO: 118. The nucleic acid molecules may comprise a nucleotide sequence comprising SEQ ID NO: 119. The nucleic acid molecules may comprise a nucleotide sequence comprising SEQ ID NO: 120. The nucleic acid molecules may comprise a nucleotide sequence comprising SEQ ID NO: 121. The nucleic acid molecules may comprise a nucleotide sequence comprising SEQ ID NO: 122. The nucleic acid molecules may comprise a nucleotide sequence comprising SEQ ID NO: 123. The nucleic acid molecules may comprise a nucleotide sequence comprising SEQ ID NO: 124. The nucleic acid molecules may comprise a nucleotide sequence comprising SEQ ID NO: 125. The nucleic acid molecules may comprise a nucleotide sequence comprising SEQ ID NO: 126. The nucleic acid molecules may comprise a nucleotide sequence comprising SEQ ID NO: 127. The nucleic acid molecules may comprise a nucleotide sequence comprising SEQ ID NO: 128. The nucleic acid molecules may comprise a nucleotide sequence comprising SEQ ID NO: 129. The nucleic acid molecules may comprise a nucleotide sequence comprising SEQ ID NO: 130. The nucleic acid molecules may comprise a nucleotide sequence comprising SEQ ID NO: 131. The nucleic acid molecules may comprise a nucleotide sequence comprising SEQ ID NO: 132. The nucleic acid molecules may comprise a nucleotide sequence comprising SEQ ID NO: 133. The nucleic acid molecules may comprise a nucleotide sequence comprising SEQ ID NO: 134. The nucleic acid molecules may comprise a nucleotide sequence comprising SEQ ID NO: 135. The another embodiment of the nucleic acid molecules of the present invention, the nucleic acid molecule comprises nucleotide sequences selected from the group consisting of SEQ ID NO: 3; SEQ ID NO: 4, SEQ ID NO: 7; SEQ ID NO: 8; SEQ ID NO: 13; SEQ ID NO: 14, SEQ ID NO: 15; SEQ ID NO: 16, and combinations thereof. Further disclosed herein is the nucleic acid molecule comprising nucleotide sequences selected from the group consisting of SEQ ID NO: 1; SEQ ID NO: 2;; SEQ ID NO: 5; SEQ ID NO: 6; SEQ ID NO: 9; SEQ ID NO: 10; SEQ ID NO: 11; SEQ ID NO: 12;;; SEQ ID NO: 17; SEQ ID NO: 18; SEQ ID NO: 19; SEQ ID NO: 20; SEQ ID NO: 21; SEQ ID NO: 22; SEQ ID NO: 23; SEQ ID NO: 24; SEQ ID NO: 25; SEQ ID NO: 26; SEQ ID NO: 27; SEQ ID NO: 28; SEQ ID NO: 29; SEQ ID NO: 30; SEQ ID NO: 31; SEQ ID NO: 32; SEQ ID NO: 33; SEQ ID NO: 34; SEQ ID NO: 35; SEQ ID NO: 36; SEQ ID NO: 37; SEQ ID NO: 38; SEQ ID NO: 39; SEQ ID NO: 40; SEQ ID NO: 41; SEQ ID NO: 42; SEQ ID NO: 43; SEQ ID NO: 44; SEQ ID NO: 45; SEQ ID NO: 46; SEQ ID NO: 47; SEQ ID NO: 48; SEQ ID NO: 49; SEQ ID NO: 50; SEQ ID NO: 51; SEQ ID NO: 52; SEQ ID NO: 53; SEQ ID NO: 54; SEQ ID NO: 55; SEQ ID NO: 56; SEQ ID NO: 57; SEQ ID NO: 58; SEQ ID NO: 59; SEQ ID NO: 60; SEQ ID NO: 61; SEQ ID NO: 62; SEQ ID NO: 63; SEQ ID NO: 64; SEQ ID NO: 65; SEQ ID NO: 66; SEQ ID NO: 67; SEQ ID NO: 68; SEQ ID NO: 69; SEQ ID NO: 70; SEQ ID NO: 71; SEQ ID NO: 72; SEQ ID NO: 73; SEQ ID NO: 74; SEQ ID NO: 75; SEQ ID NO: 76; SEQ ID NO: 77; SEQ ID NO: 78; SEQ ID NO: 79; SEQ ID NO: 80; SEQ ID NO: 81; SEQ ID NO: 82; SEQ ID NO: 83; SEQ ID NO: 84; SEQ ID NO: 85; SEQ ID NO: 86; SEQ ID NO: 87; SEQ ID NO: 88; SEQ ID NO: 89; SEQ ID NO: 90; SEQ ID NO: 91; SEQ ID NO: 92; SEQ ID NO: 93; SEQ ID NO: 94; SEQ ID NO: 95; SEQ ID NO: 96; SEQ ID NO: 97; SEQ ID NO: 98; SEQ ID NO: 99; SEQ ID NO: 100; SEQ ID NO: 101; SEQ ID NO: 102; SEQ ID NO: 103; SEQ ID NO: 104; SEQ ID NO: 105; SEQ ID NO: 106; SEQ ID NO: 107; SEQ ID NO: 108; SEQ ID NO: 109; SEQ ID NO: 110; SEQ ID NO: 114; SEQ ID NO: 115; SEQ ID NO:116; SEQ ID NO: 117; SEQ ID NO: 118; SEQ ID NO: 119; SEQ ID NO: 120; SEQ ID NO: 121; SEQ ID NO: 122; SEQ ID NO: 123; SEQ ID NO: 124; SEQ ID NO: 125; SEQ ID NO: 126; SEQ ID NO: 127; SEQ ID NO: 128; SEQ ID NO: 129; SEQ ID NO: 130; SEQ ID NO: 131; SEQ ID NO: 132; SEQ ID NO: 133; SEQ ID NO: 134; SEQ ID NO: 135; and combinations thereof. The nucleic acid molecules may comprise a nucleotide sequence comprising SEQ ID NO: I in shNA format. The nucleic acid molecules may comprise a nucleotide sequence comprising SEQ **ID** NO: 2 in shNA format. In another embodiment of the nucleic acid molecules of the present invention, the nucleic acid molecule comprises a nucleotide sequence comprising SEQ ID NO: 3 in shNA format. In another embodiment of the nucleic acid molecules of the present invention, the nucleic acid molecule comprises a nucleotide sequence comprising SEQ ID NO: 4 in shNA format. The nucleic acid molecules may comprise a nucleotide sequence comprising SEQ ID NO: 5 in shNA format. The nucleic acid molecules may comprise a nucleotide sequence comprising SEQ ID NO: 6 in shNA format. In another embodiment of the nucleic acid molecules of the present invention, the nucleic acid molecule comprises a nucleotide sequence comprising SEQ ID NO: 7 in shNA format. In another embodiment of the nucleic acid molecules of the present invention, the nucleic acid molecule comprises a nucleotide sequence comprising SEQ ID NO: 8 in shNA format. The nucleic acid molecules may comprise a nucleotide sequence comprising SEQ ID NO: 9 in shNA format. The nucleic acid molecules may comprise a nucleotide sequence comprising SEQ ID NO: 10 in shNA format. The nucleic acid molecules may comprise a nucleotide sequence comprising SEQ ID NO: 11 in shNA format. The nucleic acid molecules may comprise a nucleotide sequence comprising SEQ ID NO: 12 in shNA format. In another embodiment of the nucleic acid molecules of the present invention, the nucleic acid molecule comprises a nucleotide sequence comprising SEQ ID NO: 13 in shNA format. In another embodiment of the nucleic acid molecules of the present invention, the nucleic acid molecule comprises a nucleotide sequence comprising SEQ ID NO: 14 in shNA format. In another embodiment of the nucleic acid molecules of the present invention, the nucleic acid molecule comprises a nucleotide, sequence comprising SEQ ID NO: 15 in shNA format. In another embodiment of the nucleic acid molecules of the present invention, the nucleic acid molecule comprises a nucleotide sequence comprising SEQ ID NO: 16 in shNA format. The nucleic acid molecules may comprise a nucleotide sequence comprising SEQ ID NO: 17 in shNA format. The nucleic acid molecules may comprise a nucleotide sequence comprising SEQ ID NO: 18 in shNA format. The nucleic acid molecules may comprise a nucleotide sequence comprising SEQ ID NO: 19 in shNA format. The nucleic acid molecules may comprise a nucleotide sequence comprising SEQ ID NO: 20 in shNA format. The nucleic acid molecules may comprise a nucleotide sequence comprising **SEQ** ID NO: 21 in shNA format. The nucleic acid molecules may comprise a nucleotide sequence comprising **SEQ** ID NO: 22 in shNA format. The nucleic acid molecules may comprise a nucleotide sequence comprising SEQ ID NO: 23 in shNA format. The nucleic acid molecules may comprise a nucleotide sequence comprising SEQ ID NO: 24 in shNA format. The nucleic acid molecules may comprise a nucleotide sequence comprising SEQ ID NO: 25 in shNA format. The nucleic acid molecules may comprise a nucleotide sequence comprising SEQ ID NO: 26 in shNA format. The nucleic acid molecules may comprise a nucleotide sequence comprising SEQ ID NO: 27 in shNA format. The nucleic acid molecules may comprise a nucleotide sequence comprising SEQ ID NO: 28 in shNA format. The nucleic acid molecules may comprise a nucleotide sequence comprising **SEQ** ID NO: 29 in shNA format. The nucleic acid molecules may comprise a nucleotide sequence comprising SEQ ID NO: 30 in shNA format. The nucleic acid molecules may comprise a nucleotide sequence comprising SEQ ID NO: 31 in shNA format. The nucleic acid molecules may comprise a nucleotide sequence comprising SEQ ID NO: 32 in shNA format. The nucleic acid molecules may comprise a nucleotide sequence comprising SEQ ID NO: 33 in shNA format. The nucleic acid molecules may comprise a nucleotide sequence comprising SEQ ID NO: 34 in shNA format. The nucleic acid molecules may comprise a nucleotide sequence comprising SEQ ID NO: 35 in shNA format. The nucleic acid molecules may comprise a nucleotide sequence comprising **SEQ** ID NO: 36 in shNA format. The nucleic acid molecules may comprise a nucleotide sequence comprising SEQ ID NO: 37 in shNA format. The nucleic acid molecules may comprise a nucleotide sequence comprising SEQ ID NO: 38 in shNA format. The nucleic acid molecules may comprise a nucleotide sequence comprising SEQ ID NO: 39 in shNA format. The nucleic acid molecules may comprise a nucleotide sequence comprising SEQ 1 D NO: 40 in shNA format. The nucleic acid molecules may comprise a nucleotide sequence comprising SEQ ID NO: 41 in shNA format. The nucleic acid molecules may comprise a nucleotide sequence comprising SEQ ID NO: 42 in shNA format. The nucleic acid molecules may comprise a nucleotide sequence comprising **SEQ** ID NO: 43 in shNA format. The nucleic acid molecules may comprise a nucleotide sequence comprising SEQ ID NO: 44 in shNA format. The nucleic acid molecules may comprise a nucleotide sequence comprising SEQ ID NO: 45 in shNA format. The nucleic acid molecules may comprise a nucleotide sequence comprising **SEQ** ID NO: 46 in shNA format. The nucleic acid molecules may comprise a nucleotide sequence comprising SEQ 1 D NO: 47 in shNA format, The nucleic acid molecules may comprise a nucleotide sequence comprising SEQ ID NO: 48 in shNA format. The nucleic acid molecules may comprise a nucleotide sequence comprising **SEQ** ID NO: 49 in shNA format. The nucleic acid molecules may comprise a nucleotide sequence comprising SEQ ID NO: 50 in shNA format. The nucleic acid molecules may comprise a nucleotide sequence comprising SEQ ID NO: 51 in shNA format. The nucleic acid molecules may comprise a nucleotide sequence comprising SEQ ID NO: 52 in shNA format. The nucleic acid molecules may comprise a nucleotide sequence comprising SEQ ID NO: 53 in shNA format. The nucleic acid molecules may comprise a nucleotide sequence comprising SEQ IL) NO: 54 in shNA format. The nucleic acid molecules may comprise a nucleotide sequence comprising **SEQ** ID NO: 55 in shNA format. The nucleic acid molecules may comprise a nucleotide sequence comprising SEQ ID NO: 56 in shNA format. The nucleic acid molecules may comprise a nucleotide sequence comprising SEQ ID NO: 57 in shNA format. The nucleic acid molecules may comprise a nucleotide sequence comprising **SEQ** ID NO: 58 in shNA format. The nucleic acid molecules may comprise a nucleotide sequence comprising **SEQ** ID NO: 59 in shNA format. The nucleic acid molecules may comprise a nucleotide sequence comprising SEQ ID NO: 60 in shNA format. The nucleic acid molecules may comprise a nucleotide sequence comprising SEQ ID NO: 61 in shNA format. The nucleic acid molecules may comprise a nucleotide sequence comprising SEQ ID NO: 62 in shNA format. The nucleic acid molecules may comprise a nucleotide sequence comprising SEQ ID NO: 63 in shNA format. The nucleic acid molecules may comprise a nucleotide sequence comprising SEQ ID NO: 64 in shNA format. The nucleic acid molecules may comprise a nucleotide sequence comprising SEQ ID NO: 65 In shNA format. The nucleic acid molecules may comprise a nucleotide sequence comprising SEQ ID NO: 66 in shNA format. The nucleic acid molecules may comprise a nucleotide sequence comprising SEQ ID NO: 67 in shNA format. The nucleic acid molecules may comprise a nucleotide sequence comprising SEQ ID NO: 68 in shNA format. The nucleic acid molecules may comprise a nucleotide sequence comprising SEQ ID NO: 69 in shNA format. The nucleic acid molecules may comprise a nucleotide sequence comprising SEQ ID NO: 70 in shNA format. The nucleic acid molecules may comprise a nucleotide sequence comprising SEQ ID NO: 71 in shNA format. The nucleic acid molecules may comprise a nucleotide sequence comprising SEQ ID NO: 72 in shNA format. The nucleic acid molecules may comprise a nucleotide sequence comprising SEQ ID NO: 73 in shNA format. The nucleic acid molecules may comprise a nucleotide sequence comprising SEQ ID NO: 74 in shNA format. The nucleic acid molecules may comprise a nucleotide sequence comprising SEQ ID NO: 75 in shNA format. The nucleic acid molecules may comprise a nucleotide sequence comprising SEQ ID NO: 76 in shNA format. The nucleic acid molecules may comprise a nucleotide sequence comprising SEQ ID NO: 77 in shNA format. The nucleic acid molecules may comprise a nucleotide sequence comprising SEQ ID NO: 78 in shNA format. The nucleic acid molecules may comprise a nucleotide sequence comprising SEQ ID NO: 79 in shNA format. The nucleic acid molecules may comprise a nucleotide sequence comprising SEQ ID NO: 80 in shNA format. The nucleic acid molecules may comprise a nucleotide sequence comprising SEQ ID NO: 81 in shNA format. The nucleic acid molecules may comprise a nucleotide sequence comprising SEQ ID NO: 82 in shNA format. The nucleic acid molecules may comprise a nucleotide sequence comprising SEQ ID NO: 83 in shNA format. The nucleic acid molecules may comprise a nucleotide sequence comprising SEQ ID NO: 84 in shNA format. The nucleic acid molecules may comprise a nucleotide sequence comprising SEQ ID NO: 85 in shNA format. The nucleic acid molecules may comprise a nucleotide sequence comprising SEQ ID NO: 86 in shNA format. The nucleic acid molecules may comprise a nucleotide sequence comprising SEQ ID NO: 87 in shNA format. The nucleic acid molecules may comprise a nucleotide sequence comprising SEQ ID NO: 88 in shNA format. The nucleic acid molecules may comprise a nucleotide sequence comprising SEQ ID NO: 89 in shNA format. The nucleic acid molecules may comprise a nucleotide sequence comprising SEQ ID NO: 90 in shNA format. The nucleic acid molecules may comprise a nucleotide sequence comprising SEQ ID NO: 91 in shNA format. The nucleic acid molecules may comprise a nucleotide sequence comprising SEQ ID NO: 92 in shNA format. The nucleic acid molecules may comprise a nucleotide sequence comprising SEQ ID NO: 93 in shNA format. The nucleic acid molecules may comprise a nucleotide sequence comprising SEQ ID NO: 94 in shNA format. The nucleic acid molecules may comprise a nucleotide sequence comprising SEQ ID NO: 95 in shNA format. The nucleic acid molecules may comprise a nucleotide sequence comprising SEQ ID NO: 96 in shNA format. The nucleic acid molecules may comprise a nucleotide sequence comprising SEQ ID NO: 97 in shNA format. The nucleic acid molecules may comprise a nucleotide sequence comprising SEQ ID NO: 98 in shNA format. The nucleic acid molecules may comprise a nucleotide sequence comprising SEQ ID NO: 99 in shNA format. The nucleic acid molecules may comprise a nucleotide sequence comprising SEQ ID NO: 100 in shNA format. The nucleic acid molecules may comprise a nucleotide sequence comprising SEQ ID NO: 101 in shNA format. The nucleic acid molecules may comprise a nucleotide sequence comprising SEQ ID NO: 102 in shNA format. The nucleic acid molecules may comprise a nucleotide sequence comprising SEQ ID NO: 103 in shNA format. The nucleic acid molecules may comprise a nucleotide sequence comprising SEQ ID NO: 104 in shNA format. The nucleic acid molecules may comprise a nucleotide sequence comprising SEQ ID NO: 105 in shNA format. The nucleic acid molecules may comprise a nucleotide sequence comprising SEQ ID NO: 106 in shNA format. The nucleic acid molecules may comprise a nucleotide sequence comprising SEQ ID NO: 107 in shNA format. The nucleic acid molecules may comprise a nucleotide sequence comprising SEQ ID NO: 108 in shNA format. The nucleic acid molecules may comprise a nucleotide sequence comprising SEQ ID NO: 109 in shNA format. The nucleic acid molecules may comprise a nucleotide sequence comprising SEQ ID NO: 110 in shNA format. The nucleic acid molecules may comprise a nucleotide sequence comprising SEQ ID NO: 114 in shNA format. The nucleic acid molecules may comprise a nucleotide sequence comprising SEQ ID NO: 115 in shNA format. The nucleic acid molecules may comprise a nucleotide sequence comprising SEQ ID NO: 116 in shNA format. The nucleic acid molecules may comprise a nucleotide sequence comprising SEQ ID NO: 117 in shNA format. The nucleic acid molecules may comprise a nucleotide sequence comprising SEQ ID NO: 118 in shNA format. The nucleic acid molecules may comprise a nucleotide sequence comprising SEQ ID NO: 119 in shNA format. The nucleic acid molecules may comprise a nucleotide sequence comprising SEQ ID NO: 120 in shNA format. The nucleic acid molecules may comprise a nucleotide sequence comprising SEQ ID NO: 121 in shNA format. The nucleic acid molecules may comprise a nucleotide sequence comprising SEQ ID NO: 122 in shNA format. The nucleic acid molecules may comprise a nucleotide sequence comprising SEQ ID NO: 123 in shNA format. The nucleic acid molecules may comprise a nucleotide sequence comprising SEQ ID NO: 124 in shNA format. The nucleic acid molecules may comprise a nucleotide sequence comprising SEQ ID NO: 125 in shNA format. The nucleic acid molecules may comprise a nucleotide sequence comprising SEQ ID NO: 126 in shNA format. The nucleic acid molecules may comprise a nucleotide sequence comprising SEQ ID NO: 127 in shNA format. The nucleic acid molecules may comprise a nucleotide sequence comprising SEQ ID NO: 128 in shNA format. The nucleic acid molecules may comprise a nucleotide sequence comprising SEQ ID NO: 129 in shNA format. The nucleic acid molecules may comprise a nucleotide sequence comprising SEQ ID NO: 130 in shNA format. The nucleic acid molecules may comprise a nucleotide sequence comprising SEQ ID NO: 131 in shNA format. The nucleic acid molecules may comprise a nucleotide sequence comprising SEQ ID NO: 132 in shNA format. The nucleic acid molecules may comprise a nucleotide sequence comprising SEQ ID NO: 133 in shNA format. The nucleic acid molecules may comprise a nucleotide sequence comprising SEQ ID NO: 134 in shNA format. The nucleic acid molecules may comprise a nucleotide sequence comprising SEQ ID NO: 135 in shNA format. In another embodiment of the nucleic acid molecules of the present invention, the nucleic acid molecule comprises nucleotide sequences selected from the group consisting of SEQ ID NO: 3; SEQ ID NO: 4; SEQ ID NO: 7; SEQ ID NO: 8; ; SEQ ID NO: 13; SEQ ID NO: 14; SEQ ID NO: 15; SEQ ID NO: 16, and combinations thereof in shNA format. Further disclosed herein is the nucleic acid molecule comprising nucleotide sequences selected from the group consisting of SEQ ID NO: 1; SEQ ID NO: 2; SEQ ID NO: 5; SEQ ID NO: 6; SEQ ID NO: 9; SEQ ID NO: 10; SEQ ID NO: 11; SEQ ID NO: 12 SEQ ID NO: 17; SEQ ID NO: 18; SEQ ID NO: 19; SEQ ID NO: 20; SEQ ID NO: 21; SEQ ID NO: 22; SEQ ID NO: 23; SEQ ID NO: 24; SEQ ID NO: 25; SEQ ID NO: 26; SEQ ID NO: 27; SEQ ID NO: 28; SEQ ID NO: 29; SEQ ID NO: 30;' SEQ ID NO: 31; SEQ ID NO: 32; SEQ ID NO: 33; SEQ ID NO: 34; SEQ ID NO: 35; SEQ ID NO: 36; SEQ ID NO: 37; SEQ ID NO: 38; SEQ ID NO: 39; SEQ ID NO: 40; SEQ ID NO: 41; SEQ ID NO: 42; SEQ ID NO: 43; SEQ ID NO: 44; SEQ ID NO: 45; SEQ ID NO: 46; SEQ ID NO: 47; SEQ ID NO: 48; SEQ ID NO: 49; SEQ ID NO: 50; SEQ ID NO: 51; SEQ ID NO: 52; SEQ ID NO: 53; SEQ ID NO: 54; SEQ ID NO: 55; SEQ ID NO: 56; SEQ ID NO: 57; SEQ ID NO: 58; SEQ ID NO: 59; SEQ ID NO: 60; SEQ ID NO: 61; SEQ ID NO: 62; SEQ ID NO: 63; SEQ ID NO: 64; SEQ ID NO: 65; SEQ ID NO: 66; SEQ ID NO: 67; SEQ ID NO: 68; SEQ ID NO: 69; SEQ ID NO: 70; SEQ ID NO: 71; SEQ ID NO: 72; SEQ ID NO: 73; SEQ ID NO: 74; SEQ ID NO: 75; SEQ ID NO: 76; SEQ ID NO: 77; SEQ ID NO: 78; SEQ ID NO: 79; SEQ ID NO: 80; SEQ ID NO: 81; SEQ ID NO: 82; SEQ ID NO: 83; SEQ ID NO: 84; SEQ ID NO: 85; SEQ ID NO: 86; SEQ ID NO: 87; SEQ ID NO: 88; SEQ ID NO: 89; SEQ ID NO: 90; SEQ ID NO: 91; SEQ ID NO: 92; SEQ ID NO: 93; SEQ ID NO: 94; SEQ ID NO: 95; SEQ ID NO: 96; SEQ ID NO: 97; SEQ ID NO: 98; SEQ ID NO: 99; SEQ ID NO: 100; SEQ ID NO: 101; SEQ ID NO: 102; SEQ ID NO: 103; SEQ ID NO: 104; SEQ ID NO: 105; SEQ ID NO: 106; SEQ ID NO: 107; SEQ ID NO: 108; SEQ ID NO: 109; SEQ ID NO: 110; SEQ ID NO: 114; SEQ ID NO: 115; SEQ ID NO: 116; SEQ ID NO: 117; SEQ ID NO: 118; SEQ ID NO: 119; SEQ ID NO: 120; SEQ ID NO: 121; SEQ ID NO: 122; SEQ ID NO: 123; SEQ ID NO: 124; SEQ ID NO: 125; SEQ ID NO: 126; SEQ ID NO: 127; SEQ ID NO: 128; SEQ ID NO: 129; SEQ ID NO: 130; SEQ ID NO: 131; SEQ ID NO: 132; SEQ ID NO: 133; SEQ ID NO: 134; SEQ ID NO: 135; and combinations thereof in shNA format.

One embodiment of the present invention includes an expression cassette comprising a nucleotide sequence. In one embodiment of the expression cassette the nucleotide sequence is a human sequence. In the expression cassette the nucleotide sequence may comprise SEQ ID NO: 1. In the expression cassette the nucleotide sequence may comprise SEQ ID NO: 2. In another embodiment of the expression cassette of the present invention, the nucleotide sequence comprises SEQ ID NO: 3. In another embodiment of the expression cassette of the present invention, the nucleotide sequence comprises SEQ ID NO: 4. In the expression cassette the nucleotide sequence may comprise SEQ ID NO: 5. In the expression cassette the nucleotide sequence may comprise SEQ ID NO: 6. In another embodiment of the expression cassette of the present invention, the nucleotide sequence comprises SEQ ID NO: 7. In another embodiment of the expression cassette of the present invention, the nucleotide sequence comprises SEQ ID NO: 8. In the expression cassette the nucleotide sequence may comprise SEQ ID NO: 9. In the expression cassette the nucleotide sequence may comprise SEQ ID NO: 10. In the expression cassette the nucleotide sequence may comprise SEQ ID NO: 11. In the expression cassette the nucleotide sequence may comprise SEQ ID NO: 12. In another embodiment of the expression cassette of the present invention, the nucleotide sequence comprises SEQ ID NO: 13. In another embodiment of the expression cassette of the present invention, the nucleotide sequence comprises SEQ ID NO: 14. In another embodiment of the expression cassette of the present invention, the nucleotide sequence comprises SEQ ID NO: 15. In another embodiment of the expression cassette of the present invention, the nucleotide sequence comprises SEQ ID NO: 16. In the expression cassette the nucleotide sequence may comprise SEQ ID NO: 17. In the expression cassette the nucleotide sequence may comprise SEQ ID NO: 18. In the expression cassette the nucleotide sequence may comprise SEQ ID NO: 19. In the expression cassette the nucleotide sequence may comprise SEQ ID NO: 20. In the expression cassette the nucleotide sequence may comprise SEQ ID NO: 21. In the expression cassette the nucleotide sequence may comprise SEQ ID NO: 22. In the expression cassette the nucleotide sequence may comprise SEQ ID NO: 23. In the expression cassette the nucleotide sequence may comprise SEQ ID NO: 24. In the expression cassette the nucleotide sequence may comprise SEQ ID NO: 25. In the expression cassette the nucleotide sequence may comprise SEQ ID NO: 26. In the expression cassette the nucleotide sequence may comprise SEQ ID NO: 27. In the expression cassette the nucleotide sequence may comprises SEQ ID NO: 28. In the expression cassette the nucleotide sequence may comprise SEQ ID NO: 29. In the expression cassette the nucleotide sequence may comprise SEQ ID NO: 30. In the expression cassette of the present invention, the nucleotide sequence comprises SEQ ID NO: 31. In the expression cassette the nucleotide sequence may comprise SEQ ID NO: 32. In the expression cassette the nucleotide sequence may comprise SEQ ID NO: 33. In the expression cassette the nucleotide sequence may comprise SEQ ID NO: 34. In the expression cassette the nucleotide sequence may comprise SEQ ID NO: 35. In the expression cassette the nucleotide sequence comprises SEQ ID NO: 36. In the expression cassette the nucleotide sequence may comprise SEQ ID NO: 37. In the expression cassette the nucleotide sequence may comprise SEQ ID NO: 38. In the expression cassette the nucleotide sequence may comprise SEQ ID NO: 39. In the expression cassette the nucleotide sequence may comprise SEQ ID NO: 40. In the expression cassette the nucleotide sequence may comprise SEQ ID NO: 41. In the expression cassette the nucleotide sequence may comprise SEQ ID NO: 42. In the expression cassette the nucleotide sequence may comprise SEQ ID NO: 43. In the expression cassette the nucleotide sequence may comprise SEQ ID NO: 44. In the expression cassette the nucleotide sequence may comprise SEQ ID NO: 45. In the expression cassette of the present invention, the nucleotide sequence comprises SEQ ID NO: 46. In the expression cassette the nucleotide sequence may comprise SEQ ID NO: 47. In the expression cassette the nucleotide sequence may comprise SEQ ID NO: 48. In the expression cassette the nucleotide sequence may comprise SEQ ID NO: 49. In the expression cassette the nucleotide sequence may comprise SEQ ID NO: 50. In the expression cassette the nucleotide sequence may comprise SEQ ID NO: 51. In the expression cassette the nucleotide sequence may comprise SEQ ID NO: 52. In the expression cassette the nucleotide sequence may comprise SEQ ID NO: 53. In the expression cassette the nucleotide sequence may comprise SEQ ID NO: 54. In the expression cassette the nucleotide sequence may comprise SEQ ID NO: 55. In the expression cassette the nucleotide sequence may comprise SEQ ID NO: 56. In the expression cassette the nucleotide sequence may comprise SEQ ID NO: 57. In the expression cassette the nucleotide sequence may comprise SEQ ID NO: 58. In the expression cassette the nucleotide sequence may comprise SEQ ID NO: 59. In the expression cassette the nucleotide sequence may comprise SEQ ID NO: 60. In the expression cassette the nucleotide sequence may comprise SEQ ID NO: 61. In the expression cassette the nucleotide sequence may comprise SEQ ID NO: 62. In the expression cassette the nucleotide sequence may comprise SEQ ID NO: 63. In the expression cassette the nucleotide sequence may comprise SEQ ID NO: 64. In the expression cassette the nucleotide sequence may comprise SEQ ID NO: 65. In the expression cassette the nucleotide sequence may comprise SEQ ID NO: 66. In the expression cassette the nucleotide sequence may comprise SEQ ID NO: 67. In the expression cassette the nucleotide sequence may comprise SEQ ID NO: 68. In the expression cassette the nucleotide sequence may comprise SEQ ID NO: 69. In the expression cassette the nucleotide sequence may comprise SEQ ID NO: 70. In the expression cassette the nucleotide sequence comprises SEQ ID NO: 71. In the expression cassette the nucleotide sequence may comprise SEQ ID NO: 72. In the expression cassette the nucleotide sequence may comprise SEQ ID NO: 73. In the expression cassette the nucleotide sequence may comprise SEQ ID NO: 74. In the expression cassette the nucleotide sequence may comprise SEQ ID NO: 75. In the expression cassette the nucleotide sequence may comprise SEQ ID NO: 76. In the expression cassette the nucleotide sequence may comprise SEQ ID NO: 77. In the expression cassette the nucleotide sequence may comprise SEQ ID NO: 78. In the expression cassette the nucleotide sequence may comprise SEQ ID NO: 79. In the expression cassette the nucleotide sequence may comprise SEQ ID NO: 80. In the expression cassette the nucleotide sequence may comprise SEQ ID NO: 81. In the expression cassette the nucleotide sequence may comprise SEQ ID NO: 82. In the expression cassette the nucleotide sequence may comprise SEQ ID NO: 83. In the expression cassette the nucleotide sequence may comprise SEQ ID NO: 84. In the expression cassette the nucleotide sequence may comprise SEQ ID NO: 85. In the expression cassette the nucleotide sequence may comprise SEQ ID NO: 86. In the expression cassette the nucleotide sequence may comprise SEQ ID NO: 87. In the expression cassette the nucleotide sequence may comprise SEQ ID NO: 88. In the expression cassette the nucleotide sequence may comprise SEQ ID NO: 89. In the expression cassette the nucleotide sequence may comprise SEQ ID NO: 90. In the expression cassette the nucleotide sequence may comprise SEQ ID NO: 91. In the expression cassette the nucleotide sequence may comprise SEQ ID NO: 92. In the expression cassette the nucleotide sequence may comprise SEQ ID NO: 93. In the expression cassette the nucleotide sequence may comprise SEQ ID NO: 94. In the expression cassette the nucleotide sequence may comprise SEQ ID NO: 95. In the expression cassette the nucleotide sequence may comprise SEQ ID NO: 96. In the expression cassette the nucleotide sequence may comprise SEQ ID NO: 97. In the expression cassette the nucleotide sequence may comprise SEQ ID NO: 98. In the expression cassette the nucleotide sequence may comprise SEQ ID NO: 99. In the expression cassette the nucleotide sequence comprises SEQ ID NO: 100. In the expression cassette the nucleotide sequence may comprise SEQ ID NO: 101. In the expression cassette the nucleotide sequence may comprise SEQ ID NO: 102. In the expression cassette the nucleotide sequence may comprise SEQ ID NO: 103. In the expression cassette the nucleotide sequence comprises SEQ ID NO: 104. In the expression cassette the nucleotide sequence may comprise SEQ ID NO: 105. In the expression cassette the nucleotide sequence may comprise SEQ ID NO: 106. In the expression cassette the nucleotide sequence may comprise SEQ ID NO: 107. In the expression cassette the nucleotide sequence may comprise SEQ ID NO: 108. In the expression cassette the nucleotide sequence may comprise SEQ ID NO: 109. In the expression cassette the nucleotide sequence may comprise SEQ ID NO: 110. In the expression cassette the nucleotide sequence may comprise SEQ ID NO: 114. In the expression cassette the nucleotide sequence may comprise SEQ ID NO: 115. In the expression cassette the nucleotide sequence may comprise SEQ ID NO: 116. In the expression cassette the nucleotide sequence may comprise SEQ ID NO: 117. In the expression cassette the nucleotide sequence may comprise SEQ ID NO: 118. In the expression cassette the nucleotide sequence may comprise SEQ ID NO: 119. In the expression cassette the nucleotide sequence may comprise SEQ ID NO: 120. In the expression cassette the nucleotide sequence may comprise SEQ ID NO: 121. In the expression cassette the nucleotide sequence may comprise SEQ ID NO: 122. In the expression cassette the nucleotide sequence may comprise SEQ ID NO: 123. In the expression cassette the nucleotide sequence comprises SEQ ID NO: 124. In the expression cassette the nucleotide sequence may comprise SEQ ID NO: 125. In the expression cassette the nucleotide sequence may comprise SEQ ID NO: 126. In the expression cassette the nucleotide sequence may comprise SEQ ID NO: 127. In the expression cassette the nucleotide sequence may comprise SEQ ID NO: 128. In the expression cassette the nucleotide sequence may comprise SEQ ID NO: 129. In the expression cassette the nucleotide sequence may comprise SEQ ID NO: 130. In the expression cassette the nucleotide sequence comprises SEQ ID NO: 131. In the expression cassette the nucleotide sequence comprises SEQ ID NO: 132. In the expression cassette the nucleotide sequence comprises SEQ ID NO: 133. In the expression cassette the nucleotide sequence comprises SEQ ID NO: 134. In the expression cassette the nucleotide sequence comprises SEQ ID NO: 135. In another embodiment of the expression cassette of the present invention, the nucleotide sequence is selected from ; SEQ ID NO: 3; SEQ ID NO: 4; SEQ ID NO: 7; SEQ ID NO: 8;; SEQ ID NO: 14; SEQ ID NO: 15; SEQ ID NO: 16; SEMI ID NO: 17; and any combination thereof. Further disclosed are expression cassettes, which the nucleotide sequence is selected from. SEQ ID NO: 1; SEQ ID NO: 2SEQ ID NO: 5; SEQ ID NO: 6; SEQ ID NO: 9; SEQ ID NO: 10; SEQ ID NO: 11; SEQ ID NO: 12; SEQ ID NO: 13; SEQ ID NO: 18; SEQ ID NO: 19; SEQ ID NO: 20; SEQ ID NO: 21; SEQ ID NO: 22; SEQ ID NO: 23; SEQ ID NO: 24; SEQ ID NO: 25; SEQ ID NO: 26; SEQ ID NO: 27; SEQ ID NO: 28; SEQ ID NO: 29; SEQ ID NO: 30; SEQ ID NO: 31; SEQ ID NO: 32; SEQ ID NO: 33; SEQ ID NO: 34; SEQ ID NO: 35; SEQ ID NO: 36; SEQ ID NO: 37; SEQ ID NO: 38; SEQ ID NO: 39; SEQ ID NO: 40; SEQ ID NO: 41; SEQ ID NO: 42; SEQ ID NO: 43; SEQ ID NO: 44; SEQ ID NO: 45; SEQ ID NO: 46; SEQ ID NO: 47; SEQ ID NO: 48; SEQ ID NO: 49; SEQ ID NO: 50; SEQ ID NO: 51; SEQ ID NO: 52; SEQ ID NO: 53; SEQ ID NO: 54; SEQ ID NO: 55; SEQ ID NO: 56; SEQ ID NO: 57; SEQ ID NO: 58; SEQ ID NO: 59; SEQ ID NO: 60; SEQ ID NO: 61; SEQ ID NO: 62; SEQ ID NO: 63; SEQ ID NO: 64; SEQ ID NO: 65; SEQ ID NO: 66; SEQ ID NO: 67; SEQ ID NO: 68; SEQ ID NO: 69; SEQ ID NO: 70; SEQ ID NO: 71; SEQ ID NO: 72; SEQ ID NO: 73; SEQ ID NO: 74; SEQ ID NO: 75; SEQ ID NO: 76; SEQ ID NO: 77; SEQ ID NO: 78; SEQ ID NO: 79; SEQ ID NO: 80; SEQ ID NO: 81; SEQ ID NO: 82; SEQ ID NO: 83; SEQ ID NO: 84; SEQ ID NO: 85; SEQ ID NO: 86; SEQ ID NO: 87; SEQ ID NO: 88; SEQ ID NO: 89; SEQ ID NO: 90; SEQ ID NO: 91; SEQ ID NO: 92; SEQ ID NO: 93; SEQ ID NO: 94; SEQ ID NO: 95; SEQ ID NO: 96; SEQ ID NO: 97; SEQ ID NO: 98; SEQ ID NO: 99; SEQ ID NO: 100; SEQ ID NO: 101; SEQ ID NO: 102; SEQ ID NO: 103; SEQ ID NO: 104; SEQ ID NO: 105; SEQ ID NO: 106; SEQ ID NO: 107; SEQ ID NO: 108; SEQ ID NO: 109; SEQ ID NO: 110; SEQ ID NO: 114; SEQ ID NO: 115; SEQ ID NO: 116; SEQ ID NO: 117; SEQ ID NO: 118; SEQ ID NO: 119; SEQ ID NO: 120; SEQ ID NO: 121; SEQ ID NO: 122; SEQ ID NO: 123; SEQ ID NO: 124; SEQ ID NO: 125; SEQ ID NO: 126; SEQ ID NO: 127; SEQ ID NO: 128; SEQ ID NO: 129; SEQ ID NO: 130; SEQ ID NO: 131; SEQ ID NO: 132; SEQ ID NO: 133; SEQ ID NO: 134; SEQ ID NO: 135; and any combination thereof. In another embodiment of the expression cassette of the present invention, the expression cassette further comprises a regulatable or a constitutive promoter operably linked to the nucleic acid sequence.

Further disclosed herein is a cell comprising an expression cassette of the present invention. Further disclosed herein is a vector comprising an expression cassette as described above. The vector is preferably a viral vector. The vector is an adenoviral virus vector or a retroviral virus vector (e.g. lentivirus, Rous sarcoma virus, Harvey sarcoma virus, Moloney murine leukemia virus, feline immunodeficiency virus). Preferably, the vector is a parvoviral virus vector (e.g. adeno-associated virus (AAV) or the vector is a picornaviral virus vector (e.g. poliovirus) or the vector is a herpes virus vector (e.g. herpes simplex virusThe vector comprises a regulatable or a constitutive promoter operably linked to a nucleic acid sequence described herein above.

One embodiment of the present invention includes a kit for screening individuals for the heterozygous presence of one or more single nucleotide polymorphisms within the individual's Huntington's genes. In one embodiment of the kits of the present invention, the kit comprises a molecular beacon that detects a single nucleotide polymorphism found in SEQ ID NO: 3 wherein said molecular beacon comprises at least a portion of the reverse complement of SEQ ID NO: 3. In another embodiment of the kits of the present invention, the kit comprises a molecular beacon that detects a single nucleotide polymorphism found in SEQ ID NO: 4 wherein said molecular beacon comprises at least a portion of the reverse complement of SEQ ID NO: 4. In another embodiment of the kits of the present invention, the kit comprises a molecular beacon that detects a single nucleotide polymorphism found in SEQ ID NO: 7 wherein said molecular beacon comprises at least a portion of the reverse complement of SEQ ID NO: 7. In another embodiment of the kits of the present invention, the kit comprises a molecular beacon that detects a single nucleotide polymorphism found in SEQ ID NO: 8 wherein said molecular beacon comprises at least a portion of the reverse complement of SEQ ID NO: 8. In another embodiment of the kits of the present invention, the kit comprises a molecular beacon that detects a single nucleotide polymorphism found in SEQ ID NO: 13 wherein said molecular beacon comprises at least a portion of the reverse complement of SEQ ID NO: 13. In another embodiment of the kits of the present invention, the kit comprises a molecular beacon that detects a single nucleotide polymorphism found in SEQ ID NO: 14 wherein said molecular beacon comprises at least a portion of the reverse complement of SEQ ID NO: 14. In another embodiment of the kits of the present invention, the kit comprises a molecular beacon that detects a single nucleotide polymorphism found in SEQ ID NO: 15 wherein said molecular beacon comprises at least a portion of the reverse complement of SEQ ID NO: 15. In another embodiment of the kits of the present invention, the kit comprises a molecular beacon that detects a single nucleotide polymorphism found in SEQ ID NO: 16 wherein said molecular beacon comprises at least a portion of the reverse complement of SEQ ID NO: 16. Further disclosed herein are the following kits: .The kit comprising a molecular beacon that detects a single nucleotide polymorphism found in SEQ ID NO: 1 wherein said molecular beacon comprises at least a portion of the reverse complement of SEQ ID NO: 1. The kit comprising a molecular beacon that detects a single nucleotide polymorphism found in SEQ ID NO: 2 wherein said molecular beacon comprises at least a portion of the reverse complement of SEQ ID NO: 2. The Kit comprising a molecular beacon that detects a single nucleotide polymorphism found in SEQ ID NO: 5 wherein said molecular beacon comprises at least a portion of the reverse complement of SEQ ID NO: 5. The kit comprising a molecular beacon that detects a single nucleotide polymorphism found in SEQ ID NO: 6 wherein said molecular beacon comprises at least a portion of the reverse complement of SEQ ID NO: 6. The kit comprising a molecular beacon that detects a single nucleotide polymorphism found in SEQ ID NO: 9 wherein said molecular beacon comprises at least a portion of the reverse complement of SEQ ID NO: 9. The kit comprises a molecular beacon that detects a single nucleotide polymorphism found in SEQ ID NO: 10 wherein said molecular beacon comprises at least a portion of the reverse complement of SEQ ID NO: 10. The kit comprises a molecular beacon that detects a single nucleotide polymorphism found in SEQ ID NO: 11 wherein said molecular beacon comprises at least a portion of the reverse complement of SEQ ID NO: 11. The kit comprises a molecular beacon that detects a single nucleotide polymorphism found in SEQ ID NO: 12 wherein said molecular beacon comprises at least a portion of the reverse complement of SEQ ID NO: 12. The kit comprising a molecular beacon that detects a single nucleotide polymorphism found in SEQ ID NO: 17 wherein said molecular beacon comprises at least a portion of the reverse complement of SEQ ID NO: 17. The kit comprising a molecular beacon that detects a single nucleotide polymorphism found in SEQ ID NO: 18 wherein said molecular beacon comprises at least a portion of the reverse complement of SEQ ID NO: 18. The kit comprising a molecular beacon that detects a single nucleotide polymorphism found in SEQ ID NO: 19 wherein said molecular beacon comprises at least a portion of the reverse complement of SEQ ID NO: 19. The kit comprising a molecular beacon that detects a single nucleotide polymorphism found in SEQ ID NO: 20 wherein said molecular beacon comprises at least a portion of the reverse complement of SEQ ID NO: 20. The kit comprising a molecular beacon that detects a single nucleotide polymorphism found in SEQ ID NO: 21 wherein said molecular beacon comprises at least a portion of the reverse complement of SEQ ID NO: 21. The kit comprising a molecular beacon that detects a single nucleotide polymorphism found in SEQ ID NO: 22 wherein said molecular beacon comprises at least a portion of the reverse complement of SEQ ID NO: 22. In another embodiment according to the kits of the present invention, the kit comprises at least one molecular beacon that detects a single nucleotide polymorphism found in a sequence selected from the group consisting of SEQ ID NO: 1; SEQ ID NO: 2; SEQ ID NO: 3; SEQ ID NO: 4; SEQ ID NO: 5; SEQ ID NO: 6; SEQ ID NO: 7; SEQ ID NO: 8; SEQ ID NO: 9; SEQ ID NO: 10; SEQ ID NO: 11; SEQ ID NO: 12; SEQ ID NO: 13; SEQ ID NO: 14; SEQ ID NO: 15; SEQ ID NO: 16; SEQ ID NO: 17; SEQ ID NO: 18; SEQ ID NO: 19; SEQ ID NO: 20; SEQ ID NO: 21; and SEQ ID NO: 22 wherein the molecular beacon comprises at least a portion of the reverse complement of the sequence containing the detected single nucleotide polymorphism. [0020] In one embodiment of the kit of the present invention, the kit comprises nucleic acid molecules that preferentially suppress the expression of amino acid sequences encoding for mutated huntingtin ("htt") over suppressing the expression of amino acid sequences encoding for normal htt by targeting an area of a Huntington's disease gene that is heterozygous for the presence of one or more single nucleotide polymorphism. The kit may include nucleic acid molecules comprising nucleotide sequences comprising SEQ ID NO: 1. The kit may include nucleic acid molecules comprising nucleotide sequences comprising SEQ ID NO: 2. In another embodiment of the kit of the present invention, the kit includes nucleic acid molecules comprising nucleotide sequences comprising SEQ ID NO: 3. In another embodiment of the kit of the present invention, the kit includes nucleic acid molecules comprising nucleotide sequences comprising SEQ ID NO: 4.
The kit may include nucleic acid molecules comprising nucleotide sequences comprising SEQ ID NO: 5. The kit may include nucleic acid molecules comprising nucleotide sequences comprising SEQ ID NO: 6. In another embodiment of the kit of the present invention, the kit includes nucleic acid molecules comprising nucleotide sequences comprising SEQ ID NO: 7. In another embodiment of the kit of the present invention, the kit includes nucleic acid molecules comprising nucleotide sequences comprising SEQ ID NO: 8. The kit may include nucleic acid molecules comprising nucleotide sequences comprising SEQ ID NO: 9.
The kit may include nucleic acid molecules comprising nucleotide sequences comprising SEQ ID NO: 10The kit may include nucleic acid molecules comprising nucleotide sequences comprising SEQ ID NO: 11. The kit may include nucleic acid molecules comprising nucleotide sequences comprising SEQ ID NO: 12. In another embodiment of the kit of the present invention, the kit includes nucleic acid molecules comprising nucleotide sequences comprising SEQ ID NO: 13. In another embodiment of the kit of the present invention, the kit includes nucleic acid molecules comprising nucleotide sequences comprising SEQ ID NO: 14. In another embodiment of the kit of the present invention, the kit includes nucleic acid molecules comprising nucleotide sequences comprising SEQ ID NO: 15. In another embodiment of the kit of the present invention, the kit includes nucleic acid molecules comprising nucleotide sequences comprising SEQ ID NO: 16The kit may include nucleic acid molecules comprising nucleotide sequences comprising SEQ ID NO: 17. The kit may include nucleic acid molecules comprising nucleotide sequences comprising SEQ ID NO: 18. The kit may include nucleic acid molecules comprising nucleotide sequences comprising SEQ ID NO: 19. The kit may include nucleic acid molecules comprising nucleotide sequences comprising SEQ ID NO: 20. The kit may include nucleic acid molecules comprising nucleotide sequences comprising SEQ ID NO: 21. The kit may include nucleic acid molecules comprising nucleotide sequences comprising SEQ ID NO: 22. The kit may include nucleic acid molecules comprising nucleotide sequences comprising SEQ ID NO: 23. The kit may include nucleic acid molecules comprising nucleotide sequences comprising SEQ ID NO: 24. The kit may include nucleic acid molecules comprising nucleotide sequences comprising SEQ ID NO: 25. The kit may include nucleic acid molecules comprising nucleotide sequences comprising SEQ D NO: 26. The kit may include nucleic acid molecules comprising nucleotide sequences comprising SEQ ID NO: 27. The kit may include nucleic acid molecules comprising nucleotide sequences comprising SEQ ID NO: 28. The kit may include nucleic acid molecules comprising nucleotide sequences comprising SEQ ID NO: 29. The kit may include nucleic acid molecules comprising nucleotide sequences comprising SEQ ID NO: 30. The kit may include nucleic acid molecules comprising nucleotide sequences comprising SEQ ID NO: 31. The kit may include nucleic acid molecules comprising nucleotide sequences comprising SEQ ID NO: 32. The kit may include nucleic acid molecules comprising nucleotide sequences comprising SEQ ID NO: 33. The kit may include nucleic acid molecules comprising nucleotide sequences comprising SEQ ID NO: 34. The kit may include nucleic acid molecules comprising nucleotide sequences comprising SEQ ID NO: 35. The kit may include nucleic acid molecules comprising nucleotide sequences comprising SEQ ID NO: 36. The kit may include nucleic acid molecules comprising nucleotide sequences comprising SEQ ID NO: 37. The kit may include nucleic acid molecules comprising nucleotide sequences comprising SEQ ID NO: 38. The kit may include nucleic acid molecules comprising nucleotide sequences comprising SEQ ID NO: 39. The kit may include nucleic acid molecules comprising nucleotide sequences comprising SEQ ID NO: 40. The kit may include nucleic acid molecules comprising nucleotide sequences comprising SEQ D NO: 41. The kit may include nucleic acid molecules comprising nucleotide sequences comprising SEQ ID NO: 42. The kit may include nucleic acid molecules comprising nucleotide sequences comprising SEQ ID NO: 43. The kit may include nucleic acid molecules comprising nucleotide sequences comprising SEQ ID NO: 44. The kit may include nucleic acid molecules comprising nucleotide sequences comprising SEQ ID NO: 45. The kit may include nucleic acid molecules comprising nucleotide sequences comprising SEQ ID NO: 46. The kit may include nucleic acid molecules comprising nucleotide sequences comprising SEQ ID NO: 47. The kit may include nucleic acid molecules comprising nucleotide sequences comprising SEQ ID NO: 48. The kit may include nucleic acid molecules comprising nucleotide sequences comprising SEQ ID NO: 49. The kit may include nucleic acid molecules comprising nucleotide sequences comprising SEQ ID NO: 50. The kit may include nucleic acid molecules comprising nucleotide sequences comprising SEQ ID NO: 51. The kit may include nucleic acid molecules comprising nucleotide sequences comprising SEQ ID NO: 52. The kit may include nucleic acid molecules comprising nucleotide sequences comprising SEQ ID NO: 53. The kit may include nucleic acid molecules comprising nucleotide sequences comprising SEQ ID NO: 54. The kit may include nucleic acid molecules comprising nucleotide sequences comprising SEQ ID NO: 55. The kit may include nucleic acid molecules comprising nucleotide sequences comprising SEQ ID NO: 56. The kit may include nucleic acid molecules comprising nucleotide sequences comprising SEQ ID NO: 57. The kit may include nucleic acid molecules comprising nucleotide sequences comprising SEQ ID NO: 58. The kit may include nucleic acid molecules comprising nucleotide sequences comprising SEQ ID NO: 59. The kit may include nucleic acid molecules comprising nucleotide sequences comprising SEQ ID NO: 60. The kit may include nucleic acid molecules comprising nucleotide sequences comprising SEQ ID NO: 61. The kit may include nucleic acid molecules comprising nucleotide sequences comprising SEQ. ID NO: 62. The kit may include nucleic acid molecules comprising nucleotide sequences comprising SEQ ID NO: 63. The kit may include nucleic acid molecules comprising nucleotide sequences comprising SEQ ID NO: 64. The kit may include nucleic acid molecules comprising nucleotide sequences comprising SEQ ID NO: 65. The kit may include nucleic acid molecules comprising nucleotide sequences comprising SEQ ID NO: 66. The kit may include nucleic acid molecules comprising nucleotide sequences comprising SEQ ID NO: 67. The kit may include nucleic acid molecules comprising nucleotide sequences comprising SEQ ID NO: 68. The kit may include nucleic acid molecules comprising nucleotide sequences comprising SEQ ID NO: 69. The kit may include nucleic acid molecules comprising nucleotide sequences comprising SEQ D NO: 70. The kit may include nucleic acid molecules comprising nucleotide sequences comprising SEQ ID NO: 71. The kit may include nucleic acid molecules comprising nucleotide sequences comprising SEQ ID NO: 72. The kit may include nucleic acid molecules comprising nucleotide sequences comprising SEQ ID NO: 73. The kit may include nucleic acid molecules comprising nucleotide sequences comprising SEQ ID NO: 74. The kit may include nucleic acid molecules comprising nucleotide sequences comprising SEQ ID NO: 75. The kit may include nucleic acid molecules comprising nucleotide sequences comprising SEQ ID NO: 76. The kit may include nucleic acid molecules comprising nucleotide sequences comprising SEQ ID NO: 77. The kit may include nucleic acid molecules comprising nucleotide sequences comprising SEQ ID NO: 78. The kit may include nucleic acid molecules comprising nucleotide sequences comprising SEQ ID NO: 79. The kit may include nucleic acid molecules comprising nucleotide sequences comprising SEQ ID NO: 80. The kit may include nucleic acid molecules comprising nucleotide sequences comprising SEQ ID NO: 81. The kit may include nucleic acid molecules comprising nucleotide sequences comprising SEQ ID NO: 82. The kit may include nucleic acid molecules comprising nucleotide sequences comprising SEQ ID NO: 83. The kit may include nucleic acid molecules comprising nucleotide sequences comprising SEQ ID NO: 84. The kit may include nucleic acid molecules comprising nucleotide sequences comprising SEQ ID NO: 85. The kit may include nucleic acid molecules comprising nucleotide sequences comprising SEQ ID NO: 86. The kit may include nucleic acid molecules comprising nucleotide sequences comprising SEQ ID NO: 87. The kit may include nucleic acid molecules comprising nucleotide sequences comprising SEQ ID NO: 88. The kit may include nucleic acid molecules comprising nucleotide sequences comprising SEQ ID NO: 89. The kit may include nucleic acid molecules comprising nucleotide sequences comprising SEQ ID NO: 90. The kit may include nucleic acid molecules comprising nucleotide sequences comprising SEQ ID NO: 91. The kit may include nucleic acid molecules comprising nucleotide sequences comprising SEQ ID NO: 92. The kit may include nucleic acid molecules comprising nucleotide sequences comprising SEQ ID NO: 93. The kit may include nucleic acid molecules comprising nucleotide sequences comprising SEQ ID NO: 94. The kit may include nucleic acid molecules comprising nucleotide sequences comprising SEQ ID NO: 95. The kit may include nucleic acid molecules comprising nucleotide sequences comprising SEQ ID NO: 96. The kit may include nucleic acid molecules comprising nucleotide sequences comprising SEQ ID NO: 97. The kit may include nucleic acid molecules comprising nucleotide sequences comprising SEQ ID NO: 98. The kit may include nucleic acid molecules comprising nucleotide sequences comprising SEQ ID NO: 99. The kit may include nucleic acid molecules comprising nucleotide sequences comprising SEQ ID NO: 100. The kit may include nucleic acid molecules comprising nucleotide sequences comprising SEQ ID NO: 101. The kit may include nucleic acid molecules comprising nucleotide sequences comprising SEQ ID NO: 102. The kit may include nucleic acid molecules comprising nucleotide sequences comprising SEQ ID NO: 103. The kit may include nucleic acid molecules comprising nucleotide sequences comprising SEQ 1 D NO: 104. The kit may include nucleic acid molecules comprising nucleotide sequences comprising SEQ ID NO: 105. The kit may include nucleic acid molecules comprising nucleotide sequences comprising SEQ ID NO: 106. The kit may include nucleic acid molecules comprising nucleotide sequences comprising SEQ ID NO: 107. The kit may include nucleic acid molecules comprising nucleotide sequences comprising SEQ ID NO: 108. The kit may include nucleic acid molecules comprising nucleotide sequences comprising SEQ ID NO: 109. The kit may include nucleic acid molecules comprising nucleotide sequences comprising SEQ ID NO: 110. The kit may include nucleic acid molecules comprising nucleotide sequences comprising SEQ ID NO: 114. The kit may include nucleic acid molecules comprising nucleotide sequences comprising SEQ ID NO: 115. The kit may include nucleic acid molecules comprising nucleotide sequences comprising SEQ ID NO: 116. The kit may include nucleic acid molecules comprising nucleotide sequences comprising SEQ ID NO: 117. The kit may include nucleic acid molecules comprising nucleotide sequences comprising SEQ ID NO: 118. The kit may include nucleic acid molecules comprising nucleotide sequences comprising SEQ ID NO: 119. The kit may include nucleic acid molecules comprising nucleotide sequences comprising SEQ ID NO: 120. The kit may include nucleic acid molecules comprising nucleotide sequences comprising SEQ ID NO: 121. The kit may include nucleic acid molecules comprising nucleotide sequences comprising SEQ ID NO: 122. The kit may include nucleic acid molecules comprising nucleotide sequences comprising SEQ 1 D NO: 123. The kit may include nucleic acid molecules comprising nucleotide sequences comprising SEQ ID NO: 124. The kit may include nucleic acid molecules comprising nucleotide sequences comprising SEQ ID NO: 125. The kit may include nucleic acid molecules comprising nucleotide sequences comprising SEQ ID NO: 126. The kit may include nucleic acid molecules comprising nucleotide sequences comprising SEQ ID NO: 127. The kit may include nucleic acid molecules comprising nucleotide sequences comprising SEQ ID NO: 128. The kit may include nucleic acid molecules comprising nucleotide sequences comprising SEQ ID NO: 129. The kit may include nucleic acid molecules comprising nucleotide sequences comprising SEQ ID NO: 130. The kit may include nucleic acid molecules comprising nucleotide sequences comprising SEQ ID NO: 131. The kit may include nucleic acid molecules comprising nucleotide sequences comprising SEQ ID NO: 132. The kit may include nucleic acid molecules comprising nucleotide sequences comprising SEQ ID NO: 133. The kit may include nucleic acid molecules comprising nucleotide sequences comprising SEQ ID NO: 134. The kit may include nucleic acid molecules comprising nucleotide sequences comprising SEQ ID NO: 135. In another embodiment of the kit of the present invention, the kit includes nucleic acid molecules comprising nucleotide sequences selected from the group consisting of; SEQ ID NO: 3; SEQ ID NO: 4; SEQ ID NO: 7; SEQ ID NO: 8; SEQ ID NO: 13; SEQ ID NO: 14; SEQ ID NO: 15; SEQ ID NO: 16 and combinations thereof. Further disclosed are kits including nucleic acid molecules comprising nucleotide sequences selected from the group consisting of SEQ ID NO: 1; SEQ ID NO: 2SEQ ID NO: 5; SEQ ID NO: 6; SEQ ID NO: 9; SEQ ID NO: 10; SEQ ID NO: 11; SEQ ID NO: 12;; SEQ ID NO: 17; SEQ ID NO: 18; SEQ ID NO: 19; SEQ ID NO: 20; SEQ ID NO: 21; SEQ ID NO: 22; SEQ ID NO: 23; SEQ ID NO: 24; SEQ ID NO: 25; SEQ ID NO: 26; SEQ ID NO: 27; SEQ ID NO: 28; SEQ ID NO: 29; SEQ ID NO: 30; SEQ ID NO: 31; SEQ ID NO: 32; SEQ ID NO: 33; SEQ ID NO: 34; SEQ ID NO: 35; SEQ ID NO: 36; SEQ ID NO: 37; SEQ ID NO: 38; SEQ ID NO: 39; SEQ ID NO: 40; SEQ ID NO: 41; SEQ ID NO: 42; SEQ ID NO: 43; SEQ ID NO: 44; SEQ ID NO: 45; SEQ ID NO: 46; SEQ ID NO: 47; SEQ ID NO: 48; SEQ ID NO: 49; SEQ ID NO: 50; SEQ ID NO: 51; SEQ ID NO: 52; SEQ 1D NO: 53; SEQ ID NO: 54; SEQ ID NO: 55; SEQ ID NO: 56; SEQ ID NO: 57; SEQ ID NO: 58; SEQ ID NO: 59; SEQ ID NO: 60; SEQ ID NO: 61; SEQ ID NO: 62; SEQ ID NO: 63; SEQ ID NO: 64; SEQ ID NO: 65; SEQ ID NO: 66; SEQ ID NO: 67; SEQ ID NO: 68; SEQ ID NO: 69; SEQ ID NO: 70; SEQ ID NO: 71; SEQ ID NO: 72; SEQ ID NO: 73; SEQ ID NO: 74; SEQ ID NO: 75; SEQ ID NO: 76; SEQ ID NO: 77; SEQ ID NO: 78; SEQ ID NO: 79; SEQ ID NO: 80; SEQ ID NO: 81; SEQ ID NO: 82; SEQ ID NO: 83; SEQ ID NO: 84; SEQ ID NO: 85; SEQ ID NO: 86; SEQ ID NO: 87; SEQ ID NO: 88; SEQ ID NO: 89; SEQ ID NO: 90; SEQ ID NO: 91; SEQ ID NO: 92; SEQ ID NO: 93; SEQ ID NO: 94; SEQ ID NO: 95; SEQ ID NO: 96; SEQ ID NO: 97; SEQ ID NO: 98; SEQ ID NO: 99; SEQ ID NO: 100; SEQ ID NO: 101; SEQ ID NO: 102; SEQ ID NO: 103; SEQ ID NO: 104; SEQ ID NO: 105; SEQ ID NO: 106; SEQ ID NO: 107; SEQ ID NO: 108; SEQ ID NO: 109; SEQ ID NO: 110; SEQ ID NO: 114; SEQ ID NO: 115; SEQ ID NO: 116; SEQ ID NO: 117; SEQ ID NO: 118; SEQ ID NO: 119; SEQ ID NO: 120; SEQ ID NO: 121; SEQ ID NO: 122; SEQ ID NO: 123; SEQ ID NO: 124; SEQ ID NO: 125; SEQ ID NO: 126; SEQ ID NO: 127; SEQ ID NO: 128; SEQ ID NO: 129; SEQ ID NO: 130; SEQ ID NO: 131; SEQ ID NO: 132; SEQ ID NO: 133; SEQ ID NO: 134; SEQ ID NO: 135; and combinations thereof.
The kit may include nucleic acid molecules comprising nucleotide sequences comprising SEQ ID NO: 1 in shNA format.
The kit may include nucleic acid molecules comprising nucleotide sequences comprising SEQ ID NO: 2 in shNA format. In another embodiment of the kit of the present invention, the kit includes nucleic acid molecules comprising nucleotide sequences comprising SEQ ID NO: 3 in shNA format. In another embodiment of the kit of the present invention, the kit includes nucleic acid molecules comprising nucleotide sequences comprising SEQ ID NO: 4 in shNA format. The kit may include nucleic acid molecules comprising nucleotide sequences comprising SEQ ID NO: 5 in shNA format. The kit may include nucleic acid molecules comprising nucleotide sequences comprising SEQ ID NO: 6 in shNA format. In another embodiment of the kit of the present invention, the kit includes nucleic acid molecules comprising nucleotide sequences comprising SEQ ID NO: 7 in shNA format. In another embodiment of the kit of the present invention, the kit includes nucleic acid molecules comprising nucleotide sequences comprising SEQ ID NO: 8 in shNA format. The kit may include nucleic acid molecules comprising nucleotide sequences comprising SEQ ID NO: 9 in shNA format. The kit may include nucleic acid molecules comprising nucleotide sequences comprising SEQ ID NO: 10 in shNA format. The kit may include nucleic acid molecules comprising nucleotide sequences comprising SEQ ID NO: 11 in shNA format. The kit may include nucleic acid molecules comprising nucleotide sequences comprising SEQ ID NO: 12 in shNA format. In another embodiment of the kit of the present invention, the kit includes nucleic acid molecules comprising nucleotide sequences comprising SEQ ID NO: 13 in shNA format. In another embodiment of the kit of the present invention, the kit includes nucleic acid molecules comprising nucleotide sequences comprising SEQ ID NO: 14 in shNA format. In another embodiment of the kit of the present invention, the kit includes nucleic acid molecules comprising nucleotide sequences comprising SEQ ID NO: 15 in shNA format. In another embodiment of the kit of the present invention, the kit includes nucleic acid molecules comprising nucleotide sequences comprising SEQ ID NO: 16 in shNA format. The kit may include- nucleic acid molecules comprising nucleotide sequences comprising SEQ ID NO: 17 in shNA format The kit may include nucleic acid molecules comprising nucleotide sequences comprising SEQ ID NO: 18 in shNA formatThe kit may include nucleic acid molecules comprising nucleotide sequences comprising SEQ ID NO: 19 in shNA formatThe kit may include nucleic acid molecules comprising nucleotide sequences comprising SEQ ID NO: 20 in shNA formatThe kit may include nucleic acid molecules comprising nucleotide sequences comprising SEQ ID NO: 21 in shNA format. The kit may include nucleic acid molecules comprising nucleotide sequences comprising SEQ ID NO: 22 in shNA format. The kit may include nucleic acid molecules comprising nucleotide sequences comprising SEQ ID NO: 23 in shNA formatThe kit may include nucleic acid molecules comprising nucleotide sequences comprising SEQ ID NO: 24 in shNA format. The kit may include nucleic acid molecules comprising nucleotide sequences comprising SEQ ID NO: 25 in shNA format. The kit may include nucleic acid molecules comprising nucleotide sequences comprising SEQ ID NO: 26 in shNA format. The kit may include nucleic acid molecules comprising nucleotide sequences comprising SEQ ID NO: 27 in shNA format. The kit may include nucleic acid molecules comprising nucleotide sequences comprising SEQ ID NO: 28 in shNA formatThe kit may include nucleic acid molecules comprising nucleotide sequences comprising SEQ ID NO: 29 in shNA formatThe kit may include nucleic acid molecules comprising nucleotide sequences comprising SEQ ID NO: 30 in shNA format. The kit may include nucleic acid molecules comprising nucleotide sequences comprising SEQ ID NO: 31 in shNA format. The kit may include nucleic acid molecules comprising nucleotide sequences comprising SEQ ID NO: 32 in shNA format. The kit may include nucleic acid molecules comprising nucleotide sequences comprising SEQ ID NO: 33 in shNA format. The kit may include nucleic acid molecules comprising nucleotide sequences comprising SEQ ID NO: 34 in shNA format. The kit may include nucleic acid molecules comprising nucleotide sequences comprising SEQ ID NO: 35 in shNA format. The kit may include nucleic acid molecules comprising nucleotide sequences comprising SEQ ID NO: 36 in shNA format. The kit may include nucleic acid molecules comprising nucleotide sequences comprising SEQ ID NO: 37 in shNA formatThe kit may include nucleic acid molecules comprising nucleotide sequences comprising SEQ ID NO: 38 in shNA format. The kit may include nucleic acid molecules comprising nucleotide sequences comprising SEQ ID NO: 39 in shNA format. The kit may include nucleic acid molecules comprising nucleotide sequences comprising SEQ ID NO: 40 in shNA format. The kit may include nucleic acid molecules comprising nucleotide sequences comprising SEQ ID NO: 41 in shNA format. The kit may include nucleic acid molecules comprising nucleotide sequences comprising SEQ ID NO: 42 in shNA format. The kit may include nucleic acid molecules comprising nucleotide sequences comprising SEQ ID NO: 43 in shNA format. The kit may include nucleic acid molecules comprising nucleotide sequences comprising SEQ ID NO: 44 in shNA format. The kit may include nucleic acid molecules comprising nucleotide sequences comprising SEQ ID NO: 45 in shNA format. The kit may include nucleic acid molecules comprising nucleotide sequences comprising SEQ ID NO: 46 in shNA format. The kit may include nucleic acid molecules comprising nucleotide sequences comprising SEQ ID NO: 47 in shNA format. The kit may include nucleic acid molecules comprising nucleotide sequences comprising SEQ ID NO: 48 in shNA format. The kit may include nucleic acid molecules comprising nucleotide sequences comprising SEQ ID NO: 49 in shNA format. The kit may include nucleic acid molecules comprising nucleotide sequences comprising SEQ ID NO: 50 in shNA format. The kit may include nucleic acid molecules comprising nucleotide sequences comprising SEQ ID NO: 51 in shNA format. The kit may include nucleic acid molecules comprising nucleotide sequences comprising SEQ ID NO: 52 in shNA format. The kit may include nucleic acid molecules comprising nucleotide sequences comprising SEQ ID NO: 53 in shNA format. The kit may include nucleic acid molecules comprising nucleotide sequences comprising SEQ ID NO: 54 in shNA format. The kit may include nucleic acid molecules comprising nucleotide sequences comprising SEQ ID NO: 55 in shNA format. The kit may include nucleic acid molecules comprising nucleotide sequences comprising SEQ ID NO: 56 in shNA format. The kit may include nucleic acid molecules comprising nucleotide sequences comprising SEQ ID NO: 57 in shNA format. The kit may include nucleic acid molecules comprising nucleotide sequences comprising SEQ ID NO: 58 in shNA format. The kit may include nucleic acid molecules comprising nucleotide sequences comprising SEQ ID NO: 59 in shNA format. The kit may include nucleic acid molecules comprising nucleotide sequences comprising SEQ ID NO: 60 in shNA format. The kit may include nucleic acid molecules comprising nucleotide sequences comprising SEQ ID NO: 61 in shNA format. The kit may include nucleic acid molecules comprising nucleotide sequences comprising SEQ ID NO: 62 in shNA format. The kit may include nucleic acid molecules comprising nucleotide sequences comprising SEQ ID NO: 63 in shNA format. The kit may include nucleic acid molecules comprising nucleotide sequences comprising SEQ ID NO: 64 in shNA format. The kit may include nucleic acid molecules comprising nucleotide sequences comprising SEQ ID NO: 65 in shNA format. The kit may include nucleic acid molecules comprising nucleotide sequences comprising SEQ ID NO: 66 in shNA format. The kit may include nucleic acid molecules comprising nucleotide sequences comprising SEQ ID NO: 67 in shNA format. The kit may include nucleic acid molecules comprising nucleotide sequences comprising SEQ ID NO: 68 in shNA format. The kit may include nucleic acid molecules comprising nucleotide sequences comprising SEQ ID NO: 69 in shNA format. The kit may include nucleic acid molecules comprising nucleotide sequences comprising SEQ ID NO: 70 in shNA format. The kit may include nucleic acid molecules comprising nucleotide sequences comprising SEQ ID NO: 71 in shNA format. The kit may include nucleic acid molecules comprising nucleotide sequences comprising SEQ ID NO: 72 in shNA format. The kit may include nucleic acid molecules comprising nucleotide sequences comprising SEQ ID NO: 73 in shNA format. The kit may include nucleic acid molecules comprising nucleotide sequences comprising SEQ ID NO: 74 in shNA format. The kit may include nucleic acid molecules comprising nucleotide sequences comprising SEQ ID NO: 75 in shNA format. The kit may include nucleic acid molecules comprising nucleotide sequences comprising SEQ ID NO: 76 in shNA format. The kit may include nucleic acid molecules comprising nucleotide sequences comprising SEQ ID NO: 77 in shNA format. The kit may include nucleic acid molecules comprising nucleotide sequences comprising SEQ ID NO: 78 in shNA format. The kit may include nucleic acid molecules comprising nucleotide sequences comprising SEQ ID NO: 79 in shNA format. The kit may include nucleic acid molecules comprising nucleotide sequences comprising SEQ ID NO: 80 in shNA format. The kit may include nucleic acid molecules comprising nucleotide sequences comprising SEQ ID NO: 81 in shNA format. The kit may include nucleic acid molecules comprising nucleotide sequences comprising SEQ ID NO: 82 in shNA format. The kit may include nucleic acid molecules comprising nucleotide sequences comprising SEQ ID NO: 83 in shNA format. The kit may include nucleic acid molecules comprising nucleotide sequences comprising SEQ ID NO: 84 in shNA format. The kit may include nucleic acid molecules comprising nucleotide sequences comprising SEQ ID NO: 85 in shNA format. The kit may include nucleic acid molecules comprising nucleotide sequences comprising SEQ ID NO: 86 in shNA format. The kit may include nucleic acid molecules comprising nucleotide sequences comprising SEQ ID NO: 87 in shNA format. The kit may include nucleic acid molecules comprising nucleotide sequences comprising SEQ ID NO: 88 in shNA format. The kit may include nucleic acid molecules comprising nucleotide sequences comprising SEQ ID NO: 89 in shNA format. The kit may include nucleic acid molecules comprising nucleotide sequences comprising SEQ ID NO: 90 in shNA format. The kit may include nucleic acid molecules comprising nucleotide sequences comprising SEQ ID NO: 91 in shNA format. The kit may include nucleic acid molecules comprising nucleotide sequences comprising SEQ ID NO: 92 in shNA format. The kit may include nucleic acid molecules comprising nucleotide sequences comprising SEQ ID NO: 93 in shNA format. The kit may include nucleic acid molecules comprising nucleotide sequences comprising SEQ ID NO: 94 in shNA format. The kit may include nucleic acid molecules comprising nucleotide sequences comprising SEQ ID NO: 95 In shNA format. The kit may include nucleic acid molecules comprising nucleotide sequences comprising SEQ ID NO: 96 in shNA format. The kit may include nucleic acid molecules comprising nucleotide sequences comprising SEQ ID NO: 97 in shNA format. The kit may include nucleic acid molecules comprising nucleotide sequences comprising SEQ ID NO: 98 in shNA format. The kit may include nucleic acid molecules comprising nucleotide sequences comprising SEQ ID NO: 99 in shNA format. The kit may include nucleic acid molecules comprising nucleotide sequences comprising SEQ ID NO: 100 in shNA format. The kit may include nucleic acid molecules comprising nucleotide sequences comprising SEQ ID NO: 101 in shNA format. The kit may include nucleic acid molecules comprising nucleotide sequences comprising SEQ ID NO: 102 in shNA format. The kit may include nucleic acid molecules comprising nucleotide sequences comprising SEQ ID NO: 103 in shNA format. The kit may include nucleic acid molecules comprising nucleotide sequences comprising SEQ ID NO: 104 in shNA format. The kit may include nucleic acid molecules comprising nucleotide sequences comprising SEQ ID NO: 105 in shNA format. The kit may include nucleic acid molecules comprising nucleotide sequences comprising SEQ ID NO: 106 in shNA format. The kit may include nucleic acid molecules comprising nucleotide sequences comprising SEQ ID NO: 107 in shNA format. The kit may include nucleic acid molecules comprising nucleotide sequences comprising SEQ ID NO: 108 in shNA format. The kit may include nucleic acid molecules comprising nucleotide sequences comprising SEQ ID NO: 109 in shNA format. The kit may include nucleic acid molecules comprising nucleotide sequences comprising SEQ 1 D NO: 110 in shNA format. The kit may include nucleic acid molecules comprising nucleotide sequences comprising SEQ ID NO: 114 in shNA format. The kit may include nucleic acid molecules comprising nucleotide sequences comprising SEQ ID NO: 115 in shNA format. The kit may include nucleic acid molecules comprising nucleotide sequences comprising SEQ ID NO: 116 in shNA format. The kit may include nucleic acid molecules comprising nucleotide sequences comprising SEQ ID NO: 117 in shNA format. The kit may include nucleic acid molecules comprising nucleotide sequences comprising SEQ ID NO: 118 in shNA format. The kit may include nucleic acid molecules comprising nucleotide sequences comprising SEQ ID NO: 119 in shNA format. The kit may include nucleic acid molecules comprising nucleotide sequences comprising SEQ ID NO: 120 in shNA format. The kit may include nucleic acid molecules comprising nucleotide sequences comprising SEQ ID NO: 121 in shNA format. The kit may include nucleic acid molecules comprising nucleotide sequences comprising SEQ ID NO: 122 in shNA format. The kit may include nucleic acid molecules comprising nucleotide sequences comprising SEQ ID NO: 123 in shNA format. The kit may include nucleic acid molecules comprising nucleotide sequences comprising SEQ ID NO: 124 in shNA format. The kit may include nucleic acid molecules comprising nucleotide sequences comprising SEQ ID NO: 125 in shNA format. The kit may include nucleic acid molecules comprising nucleotide sequences comprising SEQ ID NO: 126 in shNA format. The kit may include nucleic acid molecules comprising nucleotide sequences comprising SEQ ID NO: 127 in shNA format. The kit may include nucleic acid molecules comprising nucleotide sequences comprising SEQ ID NO: 128 in shNA format. The kit may include nucleic acid molecules comprising nucleotide sequences comprising SEQ ID NO: 129 in shNA format. The kit may include nucleic acid molecules comprising nucleotide sequences comprising SEQ ID NO: 130 in shNA format. The kit may include nucleic acid molecules comprising nucleotide sequences comprising SEQ ID NO: 131 in shNA format. The kit may include nucleic acid molecules comprising nucleotide sequences comprising SEQ ID NO: 132 in shNA format. The kit may include nucleic acid molecules comprising nucleotide sequences comprising SEQ ID NO: 133 in shNA format. The kit may include nucleic acid molecules comprising nucleotide sequences comprising SEQ ID NO: 134 in shNA format, The kit may include nucleic acid molecules comprising nucleotide sequences comprising SEQ ID NO: 135 in shNA format. In another embodiment of the kit of the present invention, the kit includes nucleic acid molecules comprising nucleotide sequences selected from the group consisting of SEQ ID NO: 3; SEQ ID NO: 4; SEQ ID NO: 7; SEQ ID NO: 8; SEQ ID NO: 13; SEQ ID NO: 14; SEQ ID NO: 15; SEQ ID NO: 16, and combinations thereof in shNA format. Further disclosed are kits including nucleic acid molecules comprising nucleotide sequences selected from the group consisting of: SEQ ID NO: 1; SEQ ID NO: 2; SEQ ID NO: 5; SEQ ID NO: 6; SEQ ID NO: 9; SEQ ID NO: 10; SEQ ID NO: 11; SEQ ID NO: 12; SEQ ID NO: 17; SEQ ID NO: 18; SEQ ID NO: 19; SEQ ID NO: 20; SEQ ID NO: 21; SEQ ID NO: 22; SEQ ID NO: 23; SEQ ID NO: 24; SEQ ID NO: 25; SEQ ID NO: 26; SEQ ID NO: 27; SEQ ID NO: 28; SEQ ID NO: 29; SEQ ID NO: 30; SEQ ID NO: 31; SEQ 1 D NO: 32; SEQ ID NO: 33; SEQ ID NO: 34; SEQ ID NO: 35; SEQ ID NO: 36; SEQ ID NO: 37; SEQ ID NO: 38; SEQ ID NO: 39; SEQ ID NO: 40; SEQ ID NO: 41; SEQ ID NO: 42; SEQ ID NO: 43; SEQ ID NO: 44; SEQ ID NO: 45; SEQ ID NO: 46; SEQ ID NO: 47; SEQ ID NO: 48; SEQ ID NO: 49; SEQ ID NO: 50; SEQ ID NO: 51; SEQ ID NO: 52; SEQ ID NO: 53; SEQ ID NO: 54; SEQ ID NO: 55; SEQ ID NO: 56; SEQ ID NO: 57; SEQ ID NO: 58; SEQ ID NO: 59; SEQ ID NO: 60; SEQ 1D NO: 61; SEQ ID NO: 62; SEQ ID NO: 63; SEQ ID NO: 64; SEQ ID NO: 65; SEQ ID NO: 66; SEQ ID NO: 67; SEQ ID NO: 68; SEQ ID NO: 69; SEQ ID NO: 70; SEQ ID NO: 71; SEQ ID NO: 72; SEQ ID NO: 73; SEQ ID NO: 74; SEQ ID NO: 75; SEQ ID NO: 76; SEQ ID NO: 77; SEQ ID NO: 78; SEQ ID NO: 79; SEQ ID NO: 80; SEQ ID NO: 81; SEQ ID NO: 82; SEQ ID NO: 83; SEQ ID NO: 84; SEQ ID NO: 85; SEQ ID NO: 86; SEQ ID NO: 87; SEQ 1D NO: 88; SEQ ID NO: 89; SEQ ID NO: 90; SEQ ID NO: 91; SEQ ID NO: 92; SEQ ID NO: 93; SEQ ID NO: 94; SEQ ID NO: 95; SEQ ID NO: 96; SEQ ID NO: 97; SEQ ID NO: 98; SEQ ID NO: 99; SEQ ID NO: 100; SEQ ID NO: 101; SEQ ID NO: 102; SEQ ID NO: 103; SEQ ID NO: 104; SEQ ID NO: 105; SEQ ID NO: 106; SEQ ID NO: 107; SEQ ID NO: 108; SEQ ID NO: 109; SEQ ID NO: 110; SEQ ID NO: 114; SEQ 1D NO: 115; SEQ ID NO: 116; SEQ ID NO: 117; SEQ ID NO: 118; SEQ ID NO: 119; SEQ ID NO: 120; SEQ ID NO: 121; SEQ ID NO: 122; SEQ ID NO: 123; SEQ ID NO: 124; SEQ ID NO: 125; SEQ ID NO: 126; SEQ ID NO: 127; SEQ ID NO: 128; SEQ ID NO: 129; SEQ ID NO: 130; SEQ ID NO: 131; SEQ ID NO: 132; SEQ ID NO: 133; SEQ ID NO: 134; SEQ ID NO: 135; and combinations thereof in shNA format.

The kit may comprise a vector comprising an expression cassetteThe vector is a viral vector, preferably an adenoviral virus vectorThe vector is a retroviral virus vector (e. g. lentivirus, Rous sarcoma virus, Harvey sarcoma virus, Moloney murine leukemia virus, feline immunodeficiency virus or a parvoviral virus vector (e.g. adeno-associated virus (AAV)). In another embodiment of the kit of the present invention, the vector is a picornaviral virus vector (e.g. poliovirus). In another embodiment of the kit of the present invention, the vector is a herpes virus vector (e.g. herpes simplex virus).

### BRIEF DESCRIPTION OF THE FIGURES

FIG. 1 shows the basic structure and operation principles of molecular beacons.

FIG. 2 shows a potential target plasmid for nucleic acid sequence characterization studies.

FIG. 3 depicts a structure and construction of anti- htt (FIG. 3A) and control (FIG. 3.) shNA sequences.

FIG. 4 shows a schematic description of a pSilencer 1. O-U6 plasmid that can be used in the preparation of anti-hit and control shNA sequences.

FIG. 5 shows the format of AAV viral constructs.

FIG. 6 shows allele-specific suppression of a mutated huntingtin gene.

### DETAILED DESCRIPTION OF THE INVENTION

The terms "nucleic acid" or "nucleic acid molecules" refer to deoxyribonucleotides or ribonucleotides and polymers thereof in either single- or double-stranded form, composed of monomers (nucleotides) containing a sugar, phosphate and a base that is either a purine or pyrimidine. Unless specifically limited, the term encompasses nucleic acids containing known analogs of natural nucleotides that have similar binding properties as the reference nucleic acid and are metabolized in a manner similar to naturally occurring nucleotides.
Unless otherwise indicated, a particular nucleic acid sequence also encompasses conservatively modified variants thereof (e.g., degenerate codon substitutions} and complementary sequences, as well as the sequence explicitly indicated. Specifically, degenerate codon substitutions can be achieved by generating sequences in which the third position of one or more selected (or all) codons is substituted with mixed-base and/or deoxyinosine residues. The nucleic acid molecules of the present invention can include any type of nucleic acid molecule capable of mediating RNA interference, such as, without limitation, short interfering nucleic acid (siNA), short hairpin nucleic acid (shNA), short interfering RNA (siRNA), short hairpin RNA (shRNA), micro-RNA (m1 RNA), and double- stranded RNA (dsRNA). The nucleic acid molecules of the present invention also include DNA sequences that encode for nucleic acid molecules capable of mediating RNA interference, such as, without limitation, siNA, shNA, sIRNA, shRNA, m1 RNA, and dsRNA.
Further, the nucleic acid and nucleic acid molecules can contain unmodified or modified nucleotides. Modified nucleotides refer to nucleotides which contain a modification in the chemical structure of a nucleotide base, sugar and/or phosphate. Such modifications can be made to improve the stability and/or efficacy of nucleic acid molecules and are described in patents and publications such as United States Patent Number ("USPN") 6,617,438, USPN 5,334,711; USPN 5,716,824; USPN 5,627,053; United States Patent Application Number 60/082, 404; International Patent Cooperation Treaty Publication Number ("PCTPN") WO 98/1 3526; PCTPN WO 92/07065; PCTPN WO 03/070897; PCTPN WO 97/26270; PCTPN WO 93/15187; Beigelman et al., 1995, J Biol Chem, 270, 25702-8; Usman and Cedergren, 1992, Trends Biochem Sci 17, 334-9; Usman et al., 1994, Nucleic Acids Symp Ser 31, 163; Burgin et al., 1996, Biochemistry, 35, 14090-7; Perreault et al. Nature, 1990, 344, 565-567; Pieken et al., 1991, Science, 253, 314- 7; Karpeisky et al., 1998, Tetrahedron Lett, 39, 1131-4; Earnshaw and Gait, 1998, Biopolymers (Nucleic Acid Sciences), 48, 39-55; Verma and Eckstein, 1998, Annu Rev Biochem, 67, 99-1 34; Burlina et al., 1997, Bioorg Med Chem, 5, 1999-2010; Limbach et al., 1994, Nucleic Acids Res 22, 21 83-96; and Burgin et al., 1996, Biochemistry, 35, 14090-7. Such patents and publications describe general methods and strategies to modify nucleic acid molecules and as such define the state of the art. Thus, those having ordinary skill in the art of molecular biology would readily understand the techniques and methods described in the above-identified references and no further teaching is required.

The phrase "expression cassette" as used herein means a nucleic acid sequence capable of directing expression of a particular nucleotide sequence in an appropriate host cell with additional sequences that facilitate appropriate transcription of the nucleic acid sequence of interest. In addition to the nucleotide sequence of interest, the expression cassette can include a promoter operably linked to the nucleotide sequence of interest that also can be operably linked to termination signals. The expression cassette also can include expression enhancers. The expression cassette including the nucleotide sequence of interest can be chimeric. The expression cassette also can be one that is naturally occurring but has been obtained in a recombinant form useful for heterologous expression. The expression of the nucleotide sequence in the expression cassette can be under the control of a constitutive promoter or of a regulatable promoter that initiates transcription only when the host cell is exposed to some particular stimulus. In the case of a multicellular organism, the promoter also can be specific to a particular tissue or organ or stage of development.

The term "promoter" refers to a nucleotide sequence, usually upstream (5) of the nucleotide sequence of interest, which directs and/or controls expression of the nucleotide sequence of interest by providing for recognition by RNA polymerase and other factors required for proper transcription. As used herein, the term "promoter" includes a minimal promoter that is a short DNA sequence and other sequences that serve to specify the site of transcription initiation, to which regulatory elements are added for control of expression. The term "promoter" also refers to a nucleotide sequence that includes a minimal promoter plus regulatory elements that is capable of controlling the expression of a coding sequence or functional RNA. This type of promoter sequence consists of proximal and more distal upstream elements, the latter elements often referred to as enhancers. The term "enhancer" refers to a DNA sequence that can stimulate promoter activity and can be an innate element of the promoter or a heterologous element inserted to enhance the level or tissue specificity of a promoter: Enhancers are capable of operating in both orientations (normal or flipped), and are capable of functioning even when moved either upstream or downstream of the promoter. Both enhancers and other upstream promoter elements bind sequence- specific DNA-binding proteins that mediate their effects. Promoters can be derived in their entirety from a native gene, or be composed of different elements derived from different promoters found in nature, or even be comprised of synthetic DNA segments. A promoter also can contain DNA sequences that are involved in the binding of protein factors that control the effectiveness of transcription initiation in response to physiological or developmental conditions. Specific promoters used in accordance with the present invention can include, for example and without limitation p01 II promoters (including, without limitation cytomegalovirus ("CMV") promoters, chicken 3-actin ("CBA") promoters, Rous sarcoma virus ("RSV") promoters and neuron-specific enolase ("NSE") promoters) and pol III promoters.

The term "vector" is defined to include any virus, as well as any plasmid, cosmid, phage or binary vector in double or single stranded linear or circular form that may or may not be self transmissible or mobilizable, and that can transform eukaryotic host cells either by integration into the cellular genome or by existing extrachromosomally (e. g., autonomous replicating plasmid with an origin of replication).

The gene (IT-15) involved in Huntington's disease ("HD") is located on chromosome 4 at the end of the short arm. This gene encodes for the protein huntingtin ("htt"). The mutation in the HD gene responsible for HD is an unstable expanded CAG trinucleotide repeat within the coding region of the gene. This mutation results in an htt protein with an expanded polyglutamine sequence. While the normal and abnormal functions of normal and mutated htt are not known, the presence of the mutated form of htt has been correlated with the occurrence of ND symptoms. Further, the abnormal htt protein appears to misfold and aggregate in brain cells, such as neurons, due to the presence of the expanded polyglutamine sequence.

Recently, a number of single nucleotide polymorphisms (SNPs) have been identified in the HD gene through the National Center for Biotechnology Information ("NCBI") nucleotide website. These SNPs provide an avenue to use RNA1 to preferentially suppress expression of the HO- causing gene allele. Individual identified SNPs were selected with use of the basic local alignment search tool ("BLAST" analysis; NCBI). The selected SNPs included:

| SEQ ID NO: | Nucleic Acid Sequence Name | Target Gene* | Nucleotide Base Sequence | Position of SNP in cDNA of NM 002111.3 |
|---|---|---|---|---|
| 1 | SNP01a T allele | HD | 5'-AGGCCTTCATAGCGAACCT- 3' | 1079 |
| 2 | SNP01b C allele | HD | 5' AGGCCTTCACAGCGAACCT-3' | 1079 |
| 3 | SNP02a G allele | HD | 5' -AAATGTGCTGTTAGGCTTA- 3' | 1197 |
| 4 | SNP02b C allele | HD | 5' -AAATGTGCTCTTAGGCTTA- 3' | 1197 |
| 5 | SNP03a A allele | HD | 5' -CAATAAAGGAAGCCTTGCC- 3' | 3812 |
| 6 | SNP03b C allele | HD | 5'-CAATAAAGGCAGCCTTGCC- 3' | 3812 |
| 7 | SNP04a C allele | HD | 5'-AGGGTTTCTCCGCTCAGCC-3' | 4113 |
| 8 | SNP04b T allele | HD | 5' -AGGGTTTCTTCGCTCAGCC- 3' | 4113 |
| 9 | SNPO5a A allele | HD | 5' -GAGCAGGAGAACGACACCT- 3' | 4465 |
| 10 | SNP05b' C allele | HD | 5' -GAGCAGGAGCACGACACCT- 3' | 4465 |
| 11 | SNP06a C allele | HD | 5'-TAAGAGGAACAAATAAAGC- 3' | 4976 |
| 12 | SNP06b T allele | HD | 5' -TAAGAGGAATAAATAAAGC- 3' | 4976 |
| 13 | SNP07a C allele | HD | 5' -GGGACAGTACTTCAACGCT- 3' | 5471 |
| 14 | SNP07b A allele | HD | 5' -GGGACAGTAATTCAACGCT- 3' | 5471 |
| 15 | SNP08a T allele | HD | 5' -TCCCTCATCTACTGTGTGC- 3' | 7237 |
| 16 | SNP08b C allele | HD | 5' TCCCTCATCCACTGTGTGC- 3' | 7237 |
| 17 | SNP09a C allele | HD | 5' -CCGCATCAACACACTAGGC- 3' | 7674 |
| | | | | |
| 18 | SNP09b T allele | HD | 5' -CCGCATCAATACACTAGGC- 3' | 7674 |
| 19 | SNP010a C allele | HD | 5` -CTGGTGACGCAGCCCCTCG- 3' | 7744 |
| 20 | SNP010b T allele | HD | 5' -CTGGTGACGTAGCCCCTCG- 3' | 7744 |
| 21 | SNP011a T allele | HD | 5' -AAGCCCATATCACCGGCTG- 3' | 9690 |
| 22 | SNP011b C allele | HD | 5'-AAGCCCATACCACCGGCTG- 3' | 9690 |

| | | | | |
|---|---|---|---|---|
| *SNPs bolded. | | | | |

As will be explained more fully below, testing performed on the SNPs listed in the preceding table demonstrated that of 21 HD patients tested, 9% were heterozygous for SNP02; 33% were heterozygous for SNP04; 14% were heterozygous for SNP07; and 48% were heterozygous for SNP08. None of the 21 patients tested were heterozygous for SNP01 (T on both alleles); SNP03 (C on both alleles); SNP05 (A on both alleles); SNP06 (C on both alleles); SNP09 (all C on both alleles); SNP10 (C on both alleles) ; and SNP11 (T on both alleles). Of all 21 cell lines from HD patients tested 10 were heterozygous for one or more of the above mentioned SNPs. This data demonstrates that while some patients can be homozygous for all of these 11 SNPs within the HD gene, many are heterozygous for at least one known SNP of the HD genes.

Note that, as will be understood by one of ordinary skill in the art, the nucleic acid molecules of the present invention will include, without limitation, all nucleic acid sequences targeting the SNPs in the preceding table, the reverse complement of these sequences and RNA based sequences including uracils in the place of the listed thymines. In addition, the RNA interfering nucleic acids can also be created by shifting the sequence up- or downstream from the sequences in the preceding table. Furthermore allele specificity of siNA or shNA can also be achieved by creating extra mismatches in the sequence. Non- limiting examples of nucleic acid sequences targeting SNPs in the preceding table (following the respective targeted sequence) include:

| SEQ ID NO. | Nucleotide Base Sequence |
|---|---|
| 1 | 5' AGGCCTTCATAGCGAACCT 3' |
| 23 | 5' GGCCTTCATAGCGAACCTG 3' |
| 24 | 5' GCCTTCATAGCGAACCTGA 3' |
| 25 | 5' AAGGCCTTCATAGCGAACC 3' |
| 26 | 5' AAAGGCCTTCATAGCGAAC 3' |
| 2 | 5' AGGCCTTCACAGCGAACCT 3' |
| 27 | 5' GGCCTTCACAGCGAACCTG 3' |
| 28 | 5' GCCTTCACAGCGAACCTGA 3' |
| 29 | 5' AAGGCCTTCACAGCGAACC 3' |
| 30 | 5' AAAGGCCTTCACAGCGAAC 3' |
| 3 | 5' AAATGTGCT**G**TTAGGCTTA 3' |
| 31 | 5' TAAATGTGCT**G**TTAGGCTT 3' |
| 32 | 5' CTAAATGTGCT**G**TTAGGCT 3' |
| 33 | 5' AATGTGCT**G**TT**AGG**CTTAC 3' |
| 34 | 5' ATGTGCT**G**TTAGGCTTACT 3' |
| 114 | 5' AAATGTGCT**G**TGAGGCTTA 3' |
| 115 | 5' GCTACTAAATGTGCTGTTA 3' |
| 4 | 5' AAATGTGCTCTTAGGCTTA 3' |
| 35 | 5' TAAATGTGCTCTTAGGCTT 3' |
| 36 | 5' CTAATGTGCTCTTAGGCT 3' |
| 37 | 5' AATGTGCTCTTAGGCTTAC 3' |
| 38 | 5' ATGTGCTCTTAGGCTTACT 3' |
| 116 | 5' AAATGTGCTCTGAGGCTTA 3' |
| 117 | 5' GCTACT**AAA**TGTGCTCTTA 3' |
| 5 | 5' TATTTTAGG**A**AGCCTTGCC 3' |
| 39 | 5' GCAATAAAGG**AA**GCCTTGC 3' |
| 40 | 5' CGCAATAAAGG**AA**GCCTTG 3' |
| 41 | 5' AATAAAGG**AA**GCCTTGCCT 3' |
| 42 | 5' ATAAAGG**AA**GCCTTGCCTT 3' |
| 6 | 5' TATTTTAGG**C**AGCCTTGCC 3' |
| 43 | 5' GCAATAAAGGCAGCCTTGC 3' |
| 44 | 5' CGCAATAAAGGCAGCCTTG 3' |
| 45 | 5' AATAAAGGCAGCCTTGCCT 3' |
| 46 | 5' ATAAGGCAGCCTTGCCTT 3' |
| 7 | 5' AGGGTTTCTCCGCTCAGCC 3' |
| 47 | 5' GAGGGTTTCTCCGCTCAGC 3' |
| 48 | 5' GGAGGGTTTCTCCGCTCAG 3' |
| 49 | 5' GGGTTTCTCCGCTCAGCCT 3' |
| 50 | 5' GGTTTCTCCGCTCAGCCTT 3' |
| 118 | 5' GTTTGGAGGGTTTCTCCGC 3' |
| 119 | 5' AGGGTTTCTCCTCTCAGCC 3' |
| 120 | 5' AGGGTTTCTCCACTCAGCC 3' |
| 8 | 5' AGGGTTTCTTCGCTCAGCC 3' |
| 51 | 5' GAGGGTTTCTTCGCTCAGC 3' |
| 52 | 5' GGAGGGTTTCTTCGCTCAG 3' |
| 53 | 5' GGGTTTCTTCGCTCAGCCT 3' |
| 54 | 5' GGTTTCTTCGCTCAGCCTT 3' |
| 121 | 5' GTTTGGAGGGTTTCTTCGC 3' |
| 122 | 5' AGGGTCTTCTCTCAGCC 3' |
| 123 | 5' AGGGTTTCTTCACTCAGCC 3' |
| 9 | 5' GAGCAGGAG**AA**CGACACCT 3' |
| 55 | 5' GGAGCAGGAG**AA**CGACACC 3' |
| 56 | 5' CGGAGCAGGAG**AA**CGACAC 3' |
| 57 | 5' AGCAGGAG**AA**CGACACCTC 3' |
| 58 | 5' GCAGGAG**AA**CGACACCTCG 3' |
| 10 | 5' GAGCAGGAGCACGACACCT 3' |
| 59 | 5' GGAGCAGGAGCACGACACC 3' |
| 60 | 5' CGGAGCAGGAGCACGACAC 3' |
| 61 | 5' AGCAGGAGCACGACACCTC 3' |
| 62 | 5' GCAGGAGCACGACACCTCG 3' |
| 11 | 5' TAAGAGGAACAAATAAAGC 3' |
| 63 | 5' TTAAGAGGAACAAATAAAG 3' |
| 64 | 5' ATTAAGAGGAACAAATAAA 3' |
| 65 | 5' AAGAGGAACAAATAAAGCT 3' |
| 66 | 5' AGAGGAACAAATAAAGCTG 3' |
| 12 | 5' TAAGAGGAATAAATAAAGC 3' |
| 67 | 5'TTAAGAGGAATAAATAAAG3' |
| 68 | 5' ATTAAGAGGAATAAATAAA 3' |
| 69 | 5' AAGAGGAATAAATAAAGCT 3' |
| 70 | 5' AGAGGAATAAATAAAGCTG 3' |
| 13 | 5' GGGACAGTACTTCAACGCT 3' |
| 71 | 5' GGACAGTACTTCAACGCTA 3' |
| 72 | 5' GACAGTACTTCAACGCTAG 3' |
| 73 | 5' GGGGACAGTACTTCAACGC 3' |
| 74 | 5' TGGGGACAGTACTTCAACG 3' |
| 124 | 5' GGACAGTACATCAACGCTA 3' |
| 125 | 5' -GAGATGGGGACAGTACTTC 3' |
| 126 | 5' GGGGACAGTACTTAAACGC 3' |
| 14 | 5' GGGACAGT**AA**TTCAACGCT 3' |
| 75 | 5' GGACAGTA**A**TTC**AA**CGCTA 3' |
| 76 | 5' GACAGTAATTCAACGCTAG 3' |
| 77 | 5' GGGGACAGTA**A**TTCAACGC 3' |
| 78 | 5' TGGGGACAGTA**ATT**CAACG 3' |
| 127 | 5' GGACAGT**AA**GTCAACGCTA 3' |
| 128 | 5' GAGATGGGGACAGTAATTC 3' |
| 129 | 5' GGGGACAGT**AA**TTAAACGC 3' |
| 15 | 5' TCCCTCATCTACTGTGTGC 3' |
| 79 | 5' CTCCCTCAT**CTA**CTGTGTG 3' |
| 80 | 5' GCTCCCTCATCTACTGTGT 3' |
| 81 | 5' CCCTCATCTACTGTGTGCA 3' |
| 82 | 5' CCTCATCTACTGTGTGCAC 3' |
| 130 | 5' GCCTGCTCCCTCATCTACT 3' |
| 131 | 5' TCCCTCATCTACTGGGTGC 3' |
| 132 | 5' TCCCTCATCTACGGTGTGC 3' |
| 16 | 5' TCCCTCATCCACTGTGTGC 3' |
| 83 | 5' CTCCCTCATCCACTGTGTG 3' |
| 84 | 5' GCTCCCTCATCCACTGTGT 3' |
| 85 | 5' CCCTCATCCACTGTGTGCA 3' |
| 86 | 5' CCTCATCCACTGTGTGCAC 3' |
| 133 | 5' GCCTGCTCCCTCATCCACT 3' |
| 134 | 5'TCCCTCATCCACTGGGTGC 3' |
| 135 | 5' TCCCTCATCCACGGTGTGC 3' |
| 17 | **5' -CCGCATCAACACACTAGGT- 3'** |
| 87 | 5' ACCGCATCAACACACTAGG 3' |
| 88 | 5' TACCGCATCAACACACTAG 3' |
| 89 | 5' CGCATCAACACACTAGGCT 3' |
| 90 | 5' GCATCAACACACTAGGCTG 3' |
| 18 | **5' -CCGCATCAATACACTAGGT- 3'** |
| 91 | 5' ACCGCATCAATACACTAGG 3' |
| 92 | 5' TACCGCATCAATACACTAG 3' |
| 93 | 5' CGCATCAATACACTAGGCT 3' |
| 94 | 5' GCATCAATACACTAGGCTG 3' |
| 19 | **5' -CTGGTGACGCAGCCCCTCG- 3'** |
| 95 | 5' CCTGGTGACGCAGCCCCTC 3' |
| 96 | 5' TCCTGGTGACGCAGCCCCT 3' |
| 97 | 5' TGGTGACGCAGCCCCTCGT 3' |
| 98 | 5' GTGACGCAGCCCCTCGTG 3' |
| 20 | **5' -CTGGTGACGTAGCCCCTCG- 3'** |
| 99 | 5' CCTGGTGACGTAGCCCCTC 3' |
| 100 | 5' TCCTGGTGACGTAGCCCCT 3' |
| 101 | 5' TGGTGACGTAGCCCCTCGT 3' |
| 102 | 5' GGTGACGTAGCCCCTCGTG 3' |
| 21 | **5' AAGCCCATATCACCGGCTG- 3'** |
| 103 | 5' GAAGCCCATATCACCGGCT 3' |
| 104 | 5' GGAAGCCCATATCACCGGC 3' |
| 105 | 5' AGCCCATATCACCGGCTGC 3' |
| 106 | 5' GCCCATATCACCGGCTGCT 3' |
| 22 | **5' -AAGCCCATACCACCGGCTG- 3'** |
| 107 | 5' GAAGCCCATACCACCGGCT 3' |
| 108 | 5' GGAAGCCCATACCACCGGC 3' |
| 109 | 5' AGCCCATACCACCGGCTGC 3' |
| 110 | 5' GCCCATACCACCGGCTGCT 3' |

| | |
|---|---|
| * extra mismatches underlined | |

Again, and as will be understood by one of skill in the art, the nucleic acid molecules of the present invention include the sequences in the preceding table, the reverse complement of these sequences and RNA based sequences including uracils in the place of the listed thymines. Thus, the sequences in the preceding table can be considered target sequences as well as sequences included in the nucleic acid molecules of the present invention.

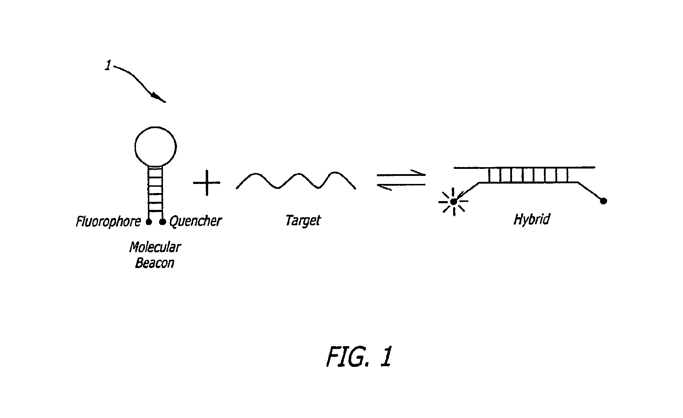

To identify SNPs present in only one allele of an individual's HD genes (in one embodiment a given HD patient's), in one method, the sequences in the preceding tables, among others, can be formatted into molecular beacons. Molecular beacons are single- stranded nucleic acid molecules with stem and loop structures that fluoresce on hybridization to their perfectly complementary targets. As shown in FIG. 1, the loop portion is complementary to a predetermined sequence in a target nucleic acid (in this invention, portions of the HD gene). The stem is formed by the binding of complementary arm sequences on each side of the loop sequence. A fluorophore moiety is covalently linked to the end of one arm and a quenching moiety is linked to the end of the other arm. When molecular beacons are free in solution, the stem keeps the two moieties of the molecular beacon in close proximity to each other, so that fluorescence is quenched. When molecular beacons hybridize to their perfectly complementary targets, however, they undergo a conformational change. The conformational change causes the fluorophore and the quencher to separate allowing the fluorophore to fluoresce. At temperatures between the dissociation temperature of perfectly complementary hybrids and mismatched hybrids, perfectly complementary targets can be distinguished from mismatched targets by higher fluorescence. By using different colors of fluorescence on different molecular beacon sequences, this technique can be used to detect multiple SNPs within HD genes in a given sample. This technique, which is described in, for example, Mhlanga & Malmberg, 2001, Methods, 25, 463-471, also can be used for testing the allele-specificity of the siNA molecules of the present invention by using molecular beacon-based real time RT-PCR reactions in which the molecular beacons used specifically target a SNP position on HD gene cDNA.

Primers for PCR using molecular beacons are designed to flank the loop sequence with the molecular beacon sequence approximately 20-30 bases 3' or 5' of the forward or reverse primer, respectively. For real time detection of SNPs, molecular beacons are designed to be complementary to a region of the amplicon where the SNP of interest occurs. The loop region of the molecular beacon generally is designed first and usually contains between approximately 15 and 30 nucleotides. This length gives a melting temperature that is slightly above the annealing temperature of the PCR which causes the molecular beacon/SNP of interest hybrid to be stable during annealing when signal detection takes place. The arm length sequences generally are designed next. These sequences often are approximately 5-7 nucleotides in length. This length allows the stems to melt at about 7- 10°C higher than the optimal annealing temperature of the involved primer set. As stated earlier, the sequences in the preceding tables as well as their reverse complements and corresponding RNA sequences can be formatted into molecular beacons.

To evaluate the effectiveness of nucleic acid sequences at suppressing expression of the mutated HD gene, appropriate cell lines for the studies were initially evaluated. First, fibroblast cells of 21 different HD patient families were purchased from the Coriel cell repository (www. coriell.org) and tested for heterozygosity of the 11 SNPs identified in the first table (SEQ ID NOS. 1-22). Of the 21 different fibroblast cell lines, ten were identified as heterozygous for one or more of the identified SNPs. Specifically, two cell lines (GM02155 and GM04022) were found to be heterozygous (C/G) for SNP 2 (SEQ ID NO. 3 and SEQ ID NO. 4); seven cell lines (GMO1 061, GM02147, GM02173, GM02191, GM01169, GM04196 and GM09197) were found to be heterozygous (A/G) for SNP 4 (SEQ ID NO. 7 and SEQ ID NO. 8); three cell lines (GM01171, GMO2155 and GM04022) were found to be heterozygous (G/T) for SNP 7 (SEQ ID NO. 13 and SEQ ID NO. 14) and 10 cell lines (GMO1061, GMO1171, GM02147, GM02155, GMO2173, GM02191, GM01169, GM04022, GM04196 and GM09197) were found to be heterozygous (A/C) for SNP 8 (SEQ ID NO. 15 and SEQ ID NO. 16). Stated another way, cell lines GMO1061, GM02147, GM02173, GM02191, GMO1169, GM04196 and GM09197 are heterozygous for SNPs 4 and 8 (SEQ ID NO. 7 and SEQ ID NO. 8 and SEQ ID NO. 15 and SEQ 1D NO. 16); cell line GMO1171 is heterozygous for SNPs 7 and 8 (SEQ ID NO. 13 and SEQ ID NO. 14 and SEQ ID NO. 15 and SEQ ID NO. 16.; and cell lines GM02155 and GM04022 are heterozygous for SNPs 2, 7 and 8 (SEQ ID NO. 3 and SEQ ID NO. 4 and SEQ ID NO. 13 and SEQ ID NO. 14 and SEQ ID NO. 15 and SEQ ID NO. 16). Thus, these cell lines that are heterozygous for the identified SNPs are appropriate cell lines to study the effectiveness of one or more of SEQ ID. NOS: 3, 4, 7, 8, 13-16, 31-38, 47-54, 71-86 and 114-135 at preferentially suppressing one selected HD allele. To study the effectiveness of nucleic acid sequences not identified as heterozygous in the studied cell lines, cell types that do not contain human HD genes can be cotransfected with a plasmid containing gene sequences of interest and a particular sequence directed against a gene sequence of interest (the mutated HD gene). For these studies, as non- limiting examples, appropriate canine or bovine cell lines could be chosen.

Once appropriate cell lines are identified as described above, in vitro transfection or co-transfection assay systems can be used to study the effectiveness of the identified sequences at suppressing expression of the mutated HD gene allele (and subsequent production of an expanded polyglutamine htt protein). In the human cell lines containing SNPs of interest, DNA sequences encoding for a siNA or shNA sequence directed against the mutated HD gene containing a particular SNP can be transfected into the cells through the use of an appropriate plasmid. Specifically, the sequence of interest (or a non- sense scrambled control, such as, in one embodiment, SEQ ID. NO. 111 (TAGCGACTAAACACATCAA)) can be subcloned into a plasmid that includes a GFP-Zeocin reporter gene for transfection efficiency normalization. The CMV promoter can direct constitutive expression of the DNA sequence encoding for the chosen siNA, shNA, or control sequence while the EFI promoter can direct constitutive expression of the GFP-Zeocin reporter gene. The generated recombinant plasmid can be used to facilitate screening of the particular nucleic acid sequence included in the recombinant plasmid by transfection into the appropriately-identified fibroblast cell lines. Forty-eight hours post-transfection, total cellular RNA can be harvested from the cells and used to make cDNA by standard methods. The cDNAs can be analyzed for normal and mutated HD gene as well as reporter (GFP) gene expression levels using real time PCR methods. The data can be normalized for GFP expression levels to control for variation in transfection efficiency. As will be recognized by one of skill in the art, screening also can be accomplished by Northern blot evaluation of transcripts harvested from transfected cells, by measuring siNA-mediated reduction of targeted protein levels and other techniques known to those of skill in the art.

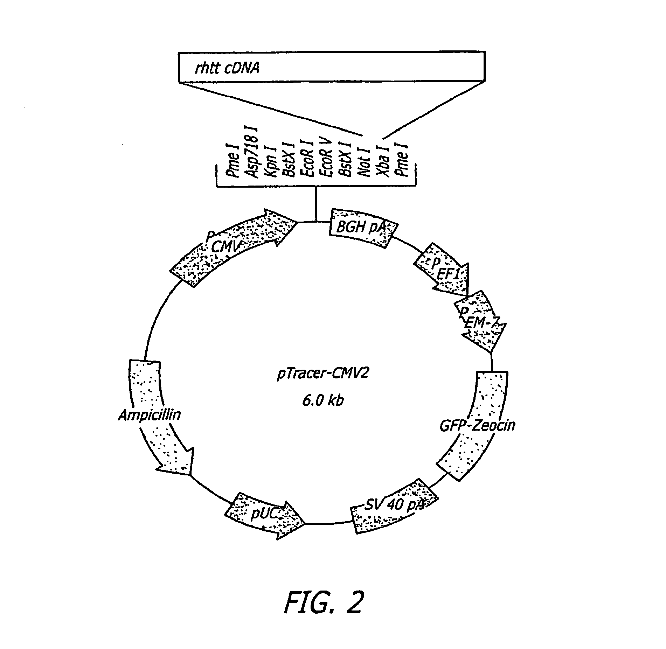

Alternatively, and referring to FIG. 2, HD gene sequences can be subcloned into pTracerTM-CMV2 (Invitrogen, Corp., Carlsbad, CA) to generate pTracer- rhtt. This recombinant plasmid also can include a GFP- Zeocin reporter gene for transfection efficiency normalization The CMV promoter can direct constitutive expression of the target genes (normal and mutated rhtt) while the EFI promoter can direct constitutive expression of the GFP-Zeocin reporter gene.

The generated recombinant plasmids can be used to facilitate screening of nucleic acid sequences by co-transfection into appropriately- identified cell lines. In one example, the cells can be cultured at 60- 70% confluency and can be co-transfected with the appropriate target plasmid and a test nucleic acid sequence (such as a DNA sequence encoding for a chosen siNA or shNA sequence) directed against the mutated HD gene sequence (or a non-sense scrambled control (in one embodiment, SEQ ID NO. 111)). Forty- eight hours post- transfection, total cellular RNA can be harvested from the cells and used to make cDNA by standard methods. The cDNAs can be analyzed for normal and mutated HD gene as well as reporter (GFP) gene expression levels using real time PCR methods. The data can be normalized for GFP expression levels to control for variation in transfection efficiency. Again, as will be recognized by one of ordinary skill in the art, screening also can be accomplished by Northern blot evaluation of transcripts harvested from transfected cells, by measuring siNA-mediated reduction of targeted protein levels and other techniques known to those of skill in the art.

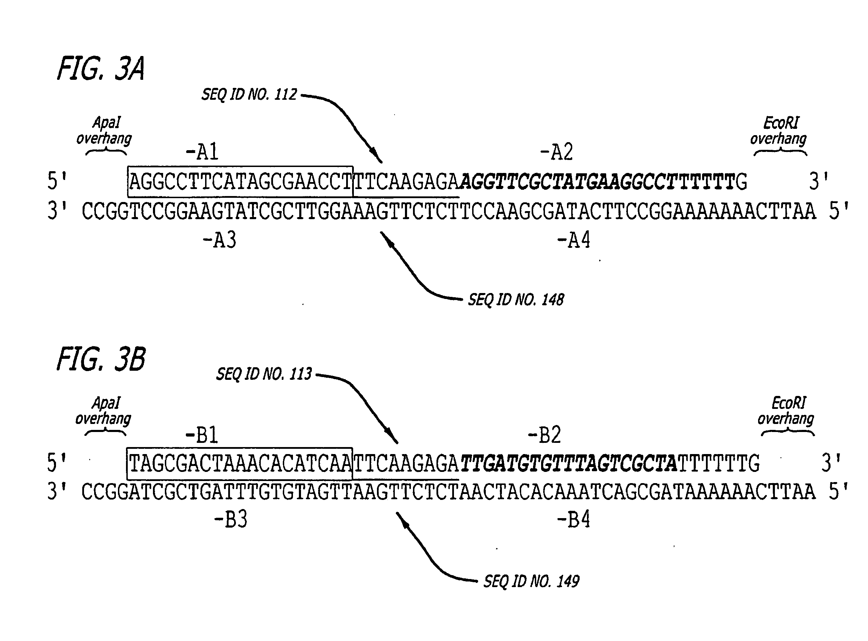

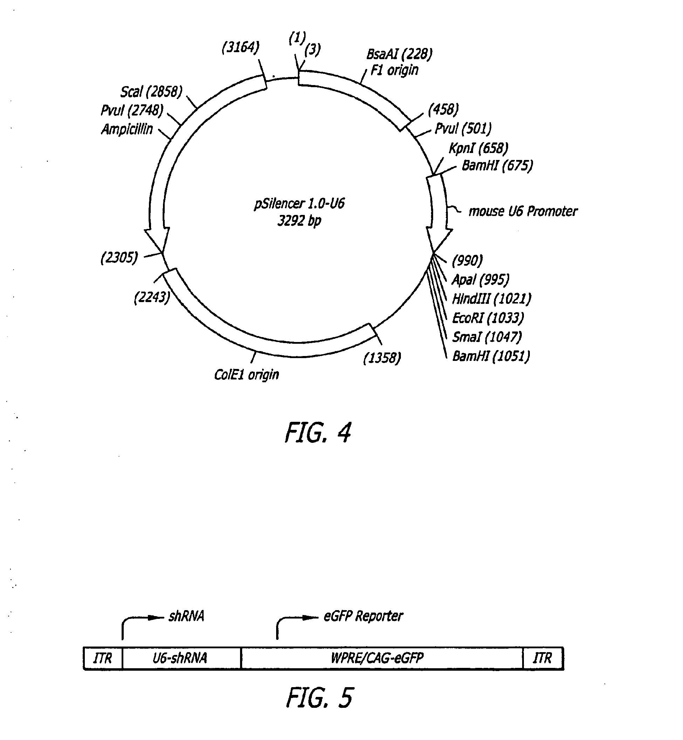

As suggested earlier, siNA nucleic acid sequences (and DNA sequences that encode for them) can be formatted into short hairpin nucleic acid ("shNA") structures. FIG. 3 shows a formatted structure and construction of an anti-mutated htt shNA sequence (SEQ ID NO: 112; FIG. 3A) and a control shNA sequence (SEQ ID NO: 113; FIG. 3B). The shNA sequences contain original nucleic acid sequences from Table I (boxed sequence shown in black), a nine nucleotide loop (underlined), the reverse complement of the boxed nucleic acid sequence (italicized), and a Pol III terminator sequence (TITTIT (bolded)). Four partially overlapping synthesized oligonucleotides (-Al, -A3, -A2, and -A4 in FIG. 3A and - B1, -B3, B2 and -B4 in FIG. 3B) can be used to assemble the shNAs. In two separate reactions, the 1/3 and 2/4 oligonucleotides for each shNA can be annealed. Referring to FIGS. 3A and 3B, the Al and Bl oligonucleotides include the boxed sequences. The A2 and B2 oligonucleotides include the underlined nine nucleotide loop, the reverse complement of oligonucleotides Al and Bl respectively, the bolded Pol III terminator sequence (TTTTTT) and the first G of the EcoRI site depicted in these FIGS. The A3 and B3 oligonucleotides of FIGS. 3A and 3B respectively include the Apal overhang region, the reverse complement of the boxed Al/Bl sequences, and the reverse complement of the underlined nine nucleotide loop sequence. The A4 and B4 oligonucleotides include the reverse complement of the italicized portion of A2 and B2 respectively and the reverse complement (AAAAAA) of the bolded Pot III terminator sequence. Finally, the A4 and B4 oligonucleotides contain the EcoRI overhang region. Apal and EcoRI restriction enzyme- compatible ends are included in the shNA structures for directional subcloning into a murine U6 promoter-containing shuttle vector (pSilencerl.O-U6; Ambion, Inc., Austin, TX; FIG. 4).
The full-length shNAs (SEQ ID NOS: 112 and 113) then can be cloned into a Apal/EcoRl- digested pSilencer vector using a three-way ligation reaction. The U6 promoter (murine or human) is used for constitutive high level expression of the nucleic acid sequences. In keeping with the present invention, a human I-II promoter can also be used.

While this example provides one embodiment of the nucleic acid sequences of the present invention in shNA format and a method for creating them, other configurations and methods also fall within the scope of the present invention. For example, in, one embodiment, the loop structure of the hairpin structure is 4 to 10 nucleotides in length. In another embodiment, the arms of the hairpin structures are less than approximately 30 nucleotides in length. In another embodiment, the arms of the hairpin structure are between 19 and 27 nucleotides in length. Thus, white a specific example is given, this example should not be interpreted to limit the scope of the present invention.

Next, a method to prepare viral production plasmids is described. This method wilt be based on the method for producing the nucleic acid sequences in shNA formats described above. As will be understood by one of skill in the art, however, various known modifications of the described technique also fall within the scope of the present invention.
First, the BamHI fragment (FIG. 5) containing the shNA expression cassette (murine U6 promoter, shNA sequence, and Pot III terminator sequence) from each of the pSilencer shuttle vectors (htt. and control) is recovered, blunted with T4 DNA polymerase, and subcloned into an AAVI/2 expression vector (deprAVE^{™}; GeneDetect.com Ltd, Bradenton, FL). As shown in FIG. 5, in the final viral expression vector (used for virus production), the U6 promoter wilt drive the expression of the shNA and the Woodchuck enhancer/chicken 3- actin promoter will drive the expression of the enhanced green fluorescent protein (WPRE/CAG-eGFP). These expression cassettes can be flanked by viral inverted terminal repeats (ITR). The Woodchuck post-transcriptional regulatory element (WPRE) and the presence of a bovine growth hormone (BGH) polyadenlyation sequence ensure high transcription following cellular transduction. In alternative methods of the present invention, the expression cassette additionally contains a polyadenylation signal, such as a synthetic minimal polyadenylation signal.

The siNA sequences and molecules of the present invention can be manipulated to enhance their uptake into the RISC complex. Specifically, manipulating the 5' terminal nucleotide of the guide strand can be highly advantageous. Preferential entry of the guide, or antisense, strand into RISC can be achieved by introducing 5' mismatches in the antisense strand while maintaining perfect base pairing at the 5' terminus of the sense strand. This maximizes entry of the antisense strand into the RISC complex, while also reducing potential off- target inhibition by the sense strand.

Physical methods to introduce nucleic acid- molecules and/or their carriers (i.e. vectors) into eukaryotic cells are known in the art. Some of these methods include, without limitation, calcium phosphate or calcium chloride co-precipitation, DEAE-dextran- mediated transfection, cationic lipid-mediated transfection, lipofection, protoplast fusion, particle bombardment, microinjection, liposome fusion, biolistics and other suitable methods found in, for example, Sambrook, et at. (Molecular Cloning: A Laboratory Manual, 2nd, ed, Cold Spring Harbor Laboratory, Cold Spring Harbor Laboratory Press, Cold Spring Harbor, N.Y., 1989), and other laboratory manuals. Further, biological methods to introduce nucleic acid molecules into a cell include the use of viral vectors. For mammalian gene therapy, viral vectors, and especially retroviral vectors, have become the most widely used method for inserting genes into mammalian, e.g., human cells. Other viral vectors can be derived from poxviruses, herpes simplex virus I, adenoviruses and adeno-associated viruses, and the like (see, for example, Boenisch et al., USPN 5,350,674 and Wilson et at., USPN 5,585,362). Embodiments of the present invention also can be delivered through the use of liposomes, polyethyleneimine, by iontophoresis, or by incorporation into other vehicles, such as hydrogels, cyclodextrins, biodegradable nanocapsules, and bioadhesive microspheres. Nucleic acid molecules also can be directly delivered to cells or tissues with or without the aforementioned vehicles. The nucleic acid molecules/vehicle combinations can be locally delivered by catheter and drug pump systems, direct local injection or through the use of polymers and/or drug-eluting stents. In one embodiment of the present invention, nucleic acid molecules can be administered as described in United States published patent application number 20040220132, Treatment of Neurodegenerative Disease Through Intracranial Delivery of sIRNA.

The nucleic acid sequences, molecules, expression cassettes and vectors of the present invention are administered to prevent or treat the symptoms of HD. The amount of these agents administered as well as the timing of their delivery will vary depending on various factors including, for example and without limitation, the composition chosen, the weight, physical condition, and age of the patient, and whether prevention or treatment is desired as well as other factors known to skilled practitioners. Administration of the therapeutic agents can be continuous or intermittent. The administration of the agents of the invention can be essentially continuous over a preselected period of time or can be in a series of spaced doses. Further, both local and systemic administration can be appropriate for use based on the present invention. Such factors can be determined by the researcher or treating physician.

### Example 1. Allele-Specific Suppression of the Huntingtin Gene

The following siRNA's were designed and used in the described study (synthesized by Ambion Inc., Austin, TX):

| | | | |
|---|---|---|---|
| SEQ ID NO: 136 | siRNA 363125_ C-16 | Sense sequence: 5' GAGAUGGGGACAGUACUUCtt 3' | position 5465on the Huntington mRNA (NM 002111.3) |
| SEQ ID NO:137 | siRNA 363125_ C-16 | Anti-sense sequence: 5' GAAGUACUGUCCCCAUCUCtt 3' | position 5465on the Huntington mRNA (NM 002111.3) |
| SEQ ID NO: 138 | siRNA 363125_ A-9*10 | Sense sequence: 5' GGACAGUAAGUCAACGCUAtt 3' | position 5472 on the Huntington mRNA (NM 002111.3) |
| SEQ ID NO: 139 | sIRNA 363125_ A-9*10 | Anti-sense sequence: 5' UAGCGUUGACUUACUGUCCct 3', | position 5472 on the Huntington mRNA (NM 002111.3) |

***Cell culture**.* Human fibroblasts from Huntington patients (GM04022, Coriell Institute) heterozygous for SNP363125 were cultured in Minimum Essential Medium with Earle's salts, L-glutamine, essential and non- essential amino acids, vitamins, and 15% fetal bovine serum (Gibco) at 37°C and 5% CO2.

***Transfection.* 4** x105 fibroblasts were plated in a 25cm2 culture flask containing 4.2m1 of growth medium without antibiotics one day before transfection to achieve about 50% confluency at the time of transfection. The cells were transfected with 26p.l s1 RNA (20pM) diluted in 5001 Optimem using 26d Lipofectamine 2000 transfection reagent diluted in 500d Optimem (Invitrogen) or with 2.6il siRNA diluted in 50pi Optimem using 2.6il Lipofectamine 2000 diluted in 50p.l Optimem. The final concentrations of siRNA in the culture dishes were 100nM and 10nM respectively. RNA was isolated after 36 hours of incubation.

***Semi-quantification.*** The cells were harvested from the culture flasks with I ml of Trypsin-EDTA (0.25% Trypsin, 1mM EDTA4Na, Gibco) and the cell numbers were estimated by a cell count using a viability counter (Beckman coulter). Total RNA was isolated from the cells using the RNeasy mini kit (Qiagen) and gDNA was removed using an on column DNase treatment (30mm). After isolation, the quantity and quality of the RNA was analyzed using the Experion automated electrophoresis system (Bio-Rad) and the absence of gDNA was confirmed by PCR. Reverse transcription took place using the iScript cDNA synthesis kit (Bio-Rad). Subsequently, three Real Time PCR's were carried out, one for GAPDH using iQ SYBR Green supermix (Bio-Rad) and one for each allele of SNP363125 using molecular beacons. In all reactions, standard curves were generated by amplifying the following numbers of DNA control molecules (in triple) in a 25p1 reaction: 1 x i09, 1 x 108....to 1x 102. The DNA control molecules were chemically synthesized and had the same sequence as the PCR products. Primer sequences used included: GAPDH upper primer SEQ ID NO: 140 (5' - ACTCCTCCACCT TTGACG C- 3'); GAPDH lower primer SEQ ID NO: 141 (5' - GTTGCTGTAGCCAAATTCGTT- 3') (optimal temperature and product length 56.7°C and 95 bp respectively); molecular beacon 363125 C: SEQ ID NO: 142 (5'-(6- FAM) cgcgatc GATGGGGACAGTA+CTTCAACGCTAGA gatcgcg (BHQ1)- 3; Huntington upper primer SEQ ID NO: 143 (5' -AGATATTGTTCTTTCTCGTATTCAGG- 3'); Huntington lower primer SEQ ID NO: 144(5' -TGCTCACTCATTTCCACCTTC- 3') (optimal temperature and product length 59-62°C and 234 bp respectively); molecular beacon 363125_A: SEQ ID NO: 145 (5'-(6 FAM) agtgcgt GGGACAGTA+ ATTCAACGCTAG acgcact (BHQ1)-3); Huntington upper primer SEQ ID NO: 146 (5' -CCTTCTCTCCGTATTTAATCTCCTGTA- 3'); Huntington lower primer SEQ ID NO: 147 (5' -TCATTTCCACCTTCAGCTGTTTTGTAA- 3') (optimal temperature and product length 57°C and 195 bp respectively).

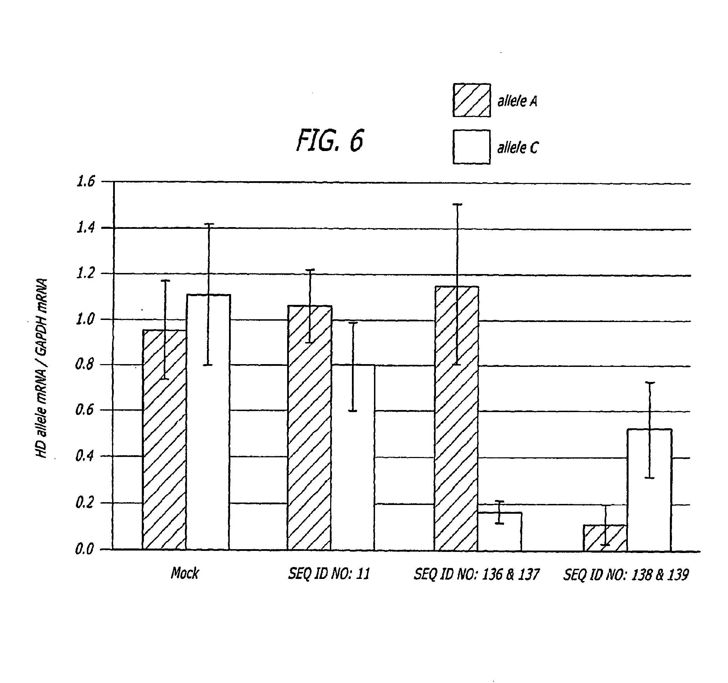

As shown in FIG. 6, SEQ ID NOs: 136 and 137 suppressed only the C- allele of Huntington mRNA while having little to no effect on the A- allele of Huntington mRNA at SNP363125 (aka SNPO7) when applied in vitro to a fibroblast cell line from a Huntington's disease patient heterozygous at this SNP site. More specifically, SEQ ID NOs: 136 and 137 suppressed the corresponding mRNA from the C allele of the Huntington's disease patient by about 83%, while showing little suppression if any of this patient's A-allele. Further siRNA with the SNP at position 9 and a further intentional mismatch at position 10 (SEQ ID NOs: 138 and 139) suppressed the A allele by about 85%, while suppressing the C allele by only about 47%. These results demonstrate the effectiveness of embodiments according to the present invention at allele-specific Huntingtin gene suppression.

### Example 2. Effectiveness of siNA and shNA Sequences in Treating HD Patients

A double-blind, placebo controlled study is conducted over a 1-year period. A total of 200 subjects, all presenting for treatment of HD are chosen for the study. The patients range in age from 35-65 years old.

An initial assessment of the symptoms of each patient is conducted when the patients initially present for treatment. The treating physician rates the severity of symptoms associated with HD on a 4-point scale (1: mild; 2: moderate; 3: strong; 4: severe). Patients then are screened for the presence of heterozygous SNPs within their HD genes. Patients that are heterozygous for a specific SNP within their HD genes are the 200 chosen for the study.

The 200 subjects chosen for the study are separated evenly into two main groups, treatment and control. The severity of the symptoms between the groups is comparable. No other medications are taken by the patients during the assessment period.

According to group assignment, patients receive intracranial administration of 10 mg of stabilized nucleic acid sequences in an appropriate vector every 7 days. Specifically, in this example, AAV vectors encoding for siNA molecules are delivered through the use of implanted, indwelling, intraparenchymal catheters as described in published U.S. Patent Application No. 2004/0220132. The treatment and control groups each are divided into two subgroups. One subgroup of the treatment group receives a siNA molecule directed against a SNP found in an individual patient's mutated HD gene. The second subgroup of the treatment group receives the same treatment except that the siNA molecules are in shNA formats. The control groups receive control sequences. Half of the control group receives control sequences in siNA formats while the second half receives control sequences in shNA formats.

During the 1 year evaluation period, patients self-evaluate the severity of their HD symptoms using the same 4-point scale (1: mild; 2: moderate; 3: strong; 4: severe) each morning and evening. Patients also note the presence and severity of adverse effects of the drug administration on the 4-point scale. In addition to the initial assessment by the treating physician, patients are evaluated at the end of each week by the treating physician.

A significant improvement in HD symptoms is observed in all of the treated subjects over the controls upon completion of the study. There is no significant difference in the effectiveness of siNA sequences as compared to shNA sequences. This study demonstrates the efficacy of the nucleic acid sequences of the present invention in treating the symptoms of HD. Regarding potential adverse effects, there are no significant differences between the therapeutic groups. Thus, this study demonstrates that the intracranial administration of nucleic acid sequences directed against mutated htt provides an effective treatment for the symptoms of HD. The study also demonstrates that the treatment is well-tolerated by patients.

It is to be understood that the present invention is not limited to the particular embodiments, materials, and examples described herein, as these can vary. For example, the nucleic acid molecules of the present invention can be created in a variety of formats and lengths. Indeed, those skilled in the art will recognize that the most important attribute of the nucleic acid molecules of the present invention is their ability to complementarily bind to the mRNA sequences of interest to reduce the mRNA stability and/or the translation rate of these sequences. The phrase "complementarily bind" as used herein, refers to the abilities of the nucleic acid molecules to form hydrogen bond(s) with mRNA sequences by either traditional Watson-Crick pairing or other non-traditional types.

### Further described herein are the following items:

1. A method comprising: the use of nucleic acid molecules comprising nucleotide sequences that preferentially suppress the expression of amino acid sequences encoding for mutated huntingtin ("htt") over suppressing the expression of amino acid sequences encoding for normal htt by targeting an area of a Huntington's disease gene that is heterozygous for the presence of one or more single nucleotide polymorphisms wherein an individual has been screened for the heterozygous presence of one or more single nucleotide polymorphisms within said individual's Huntington's genes.
2. A method according to item 1, wherein said nucleic acid molecules comprise nucleotide sequences selected from the group consisting of SEQ ID NO: 1; SEQ ID NO: 2; SEQ ID NO: 3; SEQ ID NO: 4; SEQ ID NO: 5; SEQ ID NO: 6; SEQ ID NO: 7; SEQ ID NO: 8; SEQ ID NO: 9; SEQ ID NO: 10; SEQ ID NO: 11 ; SEQ ID NO: 12; SEQ ID NO: 13; SEQ ID NO: 14; SEQ ID NO: 15; SEQ ID NO: 16; SEQ ID NO: 17; SEQ ID NO: 18; SEQ ID NO: 19; SEQ ID NO: 20; SEQ ID NO: 21 ; SEQ ID NO: 22; SEQ ID NO: 23; SEQ ID NO: 24; SEQ ID NO: 25; SEQ ID NO: 26; SEQ ID NO: 27; SEQ ID NO: 28; SEQ ID NO: 29; SEQ ID NO: 30; SEQ ID NO: 31; SEQ ID NO: 32; SEQ ID NO: 33; SEQ ID NO: 34; SEQ ID NO: 35; SEQ ID NO: 36; SEQ ID NO: 37; SEQ ID NO: 38; SEQ ID NO: 39; SEQ ID NO: 40; SEQ ID NO: 41; SEQ ID NO: 42; SEQ ID NO: 43; SEQ ID NO: 44; SEQ ID NO: 45; SEQ ID NO: 46; SEQ ID NO: 47; SEQ ID NO: 48; SEQ ID NO: 49; SEQ ID NO: 50; SEQ ID NO: 51 ; SEQ ID NO: 52; SEQ ID NO: 53; SEQ ID NO: 54; SEQ ID NO: 55; SEQ ID NO: 56; SEQ ID NO: 57; SEQ ID NO: 58; SEQ ID NO: 59; SEQ ID NO: 60; SEQ ID NO: 61 ; SEQ ID NO: 62; SEQ ID NO: 63; SEQ ID NO: 64; SEQ ID NO: 65; SEQ ID NO: 66; SEQ ID NO: 67; SEQ ID NO: 68; SEQ ID NO: 69; SEQ ID NO: 70; SEQ ID NO: 71; SEQ ID NO: 72; SEQ ID NO: 73; SEQ ID NO: 74; SEQ ID NO: 75; SEQ ID NO: 76; SEQ ID NO: 77; SEQ ID NO: 78; SEQ ID NO: 79; SEQ ID NO: 80; SEQ ID NO: 81; SEQ ID NO: 82; SEQ ID NO: 83; SEQ ID NO: 84; SEQ ID NO: 85; SEQ ID NO: 86; SEQ ID NO: 87; SEQ ID NO: 88; SEQ ID NO: 89; SEQ ID NO: 90; SEQ ID NO: 91 ; SEQ ID NO: 92; SEQ ID NO: 93; SEQ ID NO: 94; SEQ ID NO: 95; SEQ ID NO: 96; SEQ ID NO: 97; SEQ ID NO: 98; SEQ ID NO: 99; SEQ ID NO: 100; SEQ ID NO: 101 ; SEQ ID NO: 102; SEQ ID NO: 103; SEQ ID NO: 104; SEQ ID NO: 105; SEQ ID NO: 106; SEQ ID NO: 107; SEQ ID NO: 108; SEQ ID NO: 109; SEQ ID NO: 110; SEQ ID NO: 114; SEQ ID NO: 115; SEQ ID NO: 116; SEQ ID NO: 1 17; SEQ ID NO: 118; SEQ ID NO: 119; SEQ ID NO: 120; SEQ ID NO: 121; SEQ ID NO: 122; SEQ ID NO: 123; SEQ ID NO: 124; SEQ ID NO: 125; SEQ ID NO: 126; SEQ ID NO: 127; SEQ ID NO: 128; SEQ ID NO: 129; SEQ ID NO: 130; SEQ ID NO: 131; SEQ ID NO: 132; SEQ ID NO: 133; SEQ ID NO: 134; SEQ ID NO: 135; and combinations thereof.
3. A method according to item 1, wherein said nucleic acid molecules comprise nucleotide sequences selected from the group consisting of SEQ ID NO: 1 ; SEQ ID NO: 2; SEQ ID NO: 3; SEQ ID NO: 4; SEQ ID NO: 5; SEQ ID NO: 6; SEQ ID NO: 7; SEQ ID NO: 8; SEQ ID NO: 9; SEQ ID NO: 10; SEQ ID NO: 11; SEQ ID NO: 12; SEQ ID NO: 13; SEQ ID NO: 14; SEQ ID NO: 15; SEQ ID NO: 16; SEQ ID NO: 17; SEQ ID NO: 18; SEQ ID NO: 19; SEQ ID NO: 20; SEQ ID NO: 21 ; SEQ ID NO: 22; SEQ ID NO: 23; SEQ ID NO: 24; SEQ ID NO: 25; SEQ ID NO: 26; SEQ ID NO: 27; SEQ ID NO: 28; SEQ ID NO: 29; SEQ ID NO: 30; SEQ ID NO: 31; SEQ ID NO: 32; SEQ ID NO: 33; SEQ ID NO: 34; SEQ ID NO: 35; SEQ ID NO: 36; SEQ ID NO: 37; SEQ ID NO: 38; SEQ ID NO: 39; SEQ ID NO: 40; SEQ ID NO: 41; SEQ ID NO: 42; SEQ ID NO: 43; SEQ ID NO: 44; SEQ ID NO: 45; SEQ ID NO: 46; SEQ ID NO: 47; SEQ ID NO: 48; SEQ ID NO: 49; SEQ ID NO: 50; SEQ ID NO: 51; SEQ ID NO: 52; SEQ ID NO: 53; SEQ ID NO: 54; SEQ ID NO: 55; SEQ ID NO: 56; SEQ ID NO: 57; SEQ ID NO: 58; SEQ ID NO: 59; SEQ ID NO: 60; SEQ ID NO: 61 ; SEQ ID NO: 62; SEQ ID NO: 63; SEQ ID NO: 64; SEQ ID NO: 65; SEQ ID NO: 66; SEQ ID NO: 67; SEQ ID NO: 68; SEQ ID NO: 69; SEQ ID NO: 70; SEQ ID NO: 71; SEQ ID NO: 72; SEQ ID NO: 73; SEQ ID NO: 74; SEQ ID NO: 75; SEQ ID NO: 76; SEQ ID NO: 77; SEQ ID NO: 78; SEQ ID NO: 79; SEQ ID NO: 80; SEQ ID NO: 81; SEQ ID NO: 82; SEQ ID NO: 83; SEQ ID NO: 84; SEQ ID NO: 85; SEQ ID NO: 86; SEQ ID NO: 87; SEQ ID NO: 88; SEQ ID NO: 89; SEQ ID NO: 90; SEQ ID NO: 91 ; SEQ ID NO: 92; SEQ ID NO: 93; SEQ ID NO: 94; SEQ ID NO: 95; SEQ ID NO: 96; SEQ ID NO: 97; SEQ ID NO: 98; SEQ ID NO: 99; SEQ ID NO: 100; SEQ ID NO: 101 ; SEQ ID NO: 102; SEQ ID NO: 103; SEQ ID NO: 104; SEQ ID NO: 105; SEQ ID NO: 106; SEQ ID NO: 107; SEQ ID NO: 108; SEQ ID NO: 109; SEQ ID NO: 110; SEQ ID NO: 114; SEQ ID NO: 115; SEQ ID NO: 116; SEQ ID NO: 117; SEQ ID NO: 118; SEQ ID NO: 119; SEQ ID NO: 120; SEQ ID NO: 121 ; SEQ ID NO: 122; SEQ ID NO: 123; SEQ ID NO: 124; SEQ ID NO: 125; SEQ ID NO: 126; SEQ ID NO: 127; SEQ ID NO: 128; SEQ ID NO: 129; SEQ ID NO: 130; SEQ ID NO: 131 ; SEQ ID NO: 132; SEQ ID NO: 133; SEQ ID NO: 134; SEQ ID NO: 135; and combinations thereof in shNA format.
4. A method according to item 1 , wherein said nucleic acid molecules are administered to an area selected from the group consisting of the intrathecal space of the spinal cord; the striatum; intracranially; and combinations thereof.
5. A method according to item 2, wherein said nucleic acid sequences are administered through an administration system selected from the group consisting of a depot, an infusion pump, an osmotic pump, an interbody pump, a catheter, and combinations thereof.
6. A method according to item 2, wherein said nucleic acid sequences are administered through an implanted pump that controls the delivery of said nucleic acid sequences to an area selected from the group consisting of the intrathecal space of the spinal cord; the striatum; intracranially; and combinations thereof wherein said nucleic acid sequences are delivered in a pharmaceutically effective amount to improve at least one symptom of Huntington's disease.
7. A nucleic acid molecule comprising a nucleotide sequence that preferentially suppresses the expression of amino acid sequences encoding for mutated huntingtin ("htt") over suppressing the expression of amino acid sequences encoding for normal htt wherein said nucleotide sequence is a sequence selected from the group consisting of SEQ ID NO: 1 ; SEQ ID NO: 2; SEQ ID NO: 3; SEQ ID NO: 4; SEQ ID NO: 5; SEQ ID NO: 6; SEQ ID NO: 7; SEQ ID NO: 8; SEQ ID NO: 9; SEQ ID NO: 10; SEQ ID NO: 11; SEQ ID NO: 12; SEQ ID NO: 13; SEQ ID NO: 14; SEQ ID NO: 15; SEQ ID NO: 16; SEQ ID NO: 17; SEQ ID NO: 18; SEQ ID NO: 19; SEQ ID NO: 20; SEQ ID NO: 21 ; SEQ ID NO: 22; SEQ ID NO: 23; SEQ ID NO: 24; SEQ ID NO: 25; SEQ ID NO: 26; SEQ ID NO: 27; SEQ ID NO: 28; SEQ ID NO: 29; SEQ ID NO: 30; SEQ ID NO: 31 ; SEQ ID NO: 32; SEQ ID NO: 33; SEQ ID NO: 34; SEQ ID NO: 35; SEQ ID NO: 36; SEQ ID NO: 37; SEQ ID NO: 38; SEQ ID NO: 39; SEQ ID NO: 40; SEQ ID NO: 41 ; SEQ ID NO: 42; SEQ ID NO: 43; SEQ ID NO: 44; SEQ ID NO: 45; SEQ ID NO: 46; SEQ ID NO: 47; SEQ ID NO: 48; SEQ ID NO: 49; SEQ ID NO: 50; SEQ ID NO: 51 ; SEQ ID NO: 52; SEQ ID NO: 53; SEQ ID NO: 54; SEQ ID NO: 55; SEQ ID NO: 56; SEQ ID NO: 57; SEQ ID NO: 58; SEQ ID NO: 59; SEQ ID NO: 60; SEQ ID NO: 61 ; SEQ ID NO: 62; SEQ ID NO: 63; SEQ ID NO: 64; SEQ ID NO: 65; SEQ ID NO: 66; SEQ ID NO: 67; SEQ ID NO: 68; SEQ ID NO: 69; SEQ ID NO: 70; SEQ ID NO: 71 ; SEQ ID NO: 72; SEQ ID NO: 73; SEQ ID NO: 74; SEQ ID NO: 75; SEQ ID NO: 76; SEQ ID NO: 77; SEQ ID NO: 78; SEQ ID NO: 79; SEQ ID NO: 80; SEQ ID NO: 81; SEQ ID NO: 82; SEQ ID NO: 83; SEQ ID NO: 84; SEQ ID NO: 85; SEQ ID NO: 86; SEQ ID NO: 87; SEQ ID NO: 88; SEQ ID NO: 89; SEQ ID NO: 90; SEQ ID NO: 91; SEQ ID NO: 92; SEQ ID NO: 93; SEQ ID NO: 94; SEQ ID NO: 95; SEQ ID NO: 96; SEQ ID NO: 97; SEQ ID NO: 98; SEQ ID NO: 99; SEQ ID NO: 100; SEQ ID NO: 101 ; SEQ ID NO: 102; SEQ ID NO: 103; SEQ ID NO: 104; SEQ ID NO: 105; SEQ ID NO: 106; SEQ ID NO: 107; SEQ ID NO: 108; SEQ ID NO: 109; SEQ ID NO: 110; SEQ ID NO: 114; SEQ ID NO: 115; SEQ ID NO: 116; SEQ ID NO: 117; SEQ ID NO: 118; SEQ ID NO: 119; SEQ ID NO: 120; SEQ ID NO: 121; SEQ ID NO: 122; SEQ ID NO: 123; SEQ ID NO: 124; SEQ ID NO: 125; SEQ ID NO: 126; SEQ ID NO: 127; SEQ ID NO: 128; SEQ ID NO: 129; SEQ ID NO: 130; SEQ ID NO: 131; SEQ ID NO: 132; SEQ ID NO: 133; SEQ ID NO: 134; and SEQ ID NO: 135.
8. A nucleic acid molecule according to item 7, wherein said nucleotide sequence is a sequence selected from the group consisting of SEQ ID NO: 1; SEQ ID NO: 2; SEQ ID NO: 3; SEQ ID NO: 4; SEQ ID NO: 5; SEPA ID NO: 6; SEQ ID NO: 7; SEQ ID NO: B; SbU ID NO: 9; SEQ ID NO: 10; SEQ ID NO: 11; SEQ ID NO: 12; SEQ ID NO: 13; SEQ ID NO: 14; SEQ ID NO: 15; SEQ ID NO: 16; SEQ ID NO: 17; SEQ ID NO: 18; SEQ ID NO: 19; SEQ ID NO: 20; SEQ ID NO: 21 ; SEQ ID NO: 22; SEQ ID NO: 23; SEQ ID NO: 24; SEQ ID NO: 25; SEQ ID NO: 26; SEQ ID NO: 27; SEQ ID NO: 28; SEQ ID NO: 29; SEQ ID NO: 30; SEQ ID NO: 31 ; SEQ ID NO: 32; SEQ ID NO: 33; SEQ ID NO: 34; SEQ ID NO: 35; SEQ ID NO: 36; SEQ ID NO: 37; SEQ ID NO: 38; SEQ ID NO: 39; SEQ ID NO: 40; SEQ ID NO: 41 ; SEQ ID NO: 42; SEQ ID NO: 43; SEQ ID NO: 44; SEQ ID NO: 45; SEQ ID NO: 46; SEQ ID NO: 47; SEQ ID NO: 48; SEQ ID NO: 49; SEQ ID NO: 50; SEQ ID NO: 51 ; SEQ ID NO: 52; SEQ ID NO: 53; SEQ ID NO: 54; SEQ ID NO: 55; SEQ ID NO: 56; SEQ ID NO: 57; SEQ ID NO: 58; SEQ ID NO: 59; SEQ ID NO: 60; SEQ ID NO: 61 ; SEQ ID NO: 62; SEQ ID NO: 63; SEQ ID NO: 64; SEQ ID NO: 65; SEQ ID NO: 66; SEQ ID NO: 67; SEQ ID NO: 68; SEQ ID NO: 69; SEQ ID NO: 70; SEQ ID NO: 71 ; SEQ ID NO: 72; SEQ ID NO: 73; SEQ ID NO: 74; SEQ ID NO: 75; SEQ ID NO: 76; SEQ ID NO: 77; SEQ ID NO: 78; SEQ ID NO: 79; SEQ ID NO: 80; SEQ ID NO: 81 ; SEQ ID NO: 82; SEQ ID NO: 83; SEQ ID NO: 84; SEQ ID NO: 85; SEQ ID NO: 86; SEQ ID NO: 87; SEQ ID NO: 88; SEQ ID NO: 89; SEQ ID NO: 90; SEQ ID NO: 91; SEQ ID NO: 92; SEQ ID NO: 93; SEQ ID NO: 94; SEPA ID NO: 95; SEQ ID NO: 96; SEQ ID NO: 97; SEQ ID NO: 98; SEQ ID NO: 99; SEQ ID NO: 100; SEQ ID NO: 101 ; SEQ ID NO: 102; SEQ ID NO: 103; SEQ ID NO: 104; SEQ ID NO: 105; SEQ ID NO: 106; SEQ ID NO: 107; SEQ ID NO: 108; SEQ ID NO: 109; SEQ ID NO: 110; SEQ ID NO: 114; SEQ ID NO: 115; SEQ ID NO: 116; SEQ ID NO: 117; SEQ ID NO: 118; SEQ ID NO: 119; SEQ ID NO: 120; SEQ ID NO: 121 ; SEQ ID NO: 122; SEQ ID NO: 123; SEQ ID NO: 124; SEQ ID NO: 125; SEQ ID NO: 126; SEQ ID NO: 127; SEQ ID NO: 128; SEQ ID NO: 129; SEQ ID NO: 130; SEQ ID NO: 131 ; SEQ ID NO: 132; SEQ ID NO: 133; SEQ ID NO: 134; and SEQ ID NO: 135; in shNA format.
9. An expression cassette comprising a nucleotide sequence wherein said sequence is a human sequence selected from the group consisting of SEQ ID NO: 1; SEQ ID NO: 2; SEQ ID NO: 3; SEQ ID NO: 4; SEQ ID NO: 5; SEQ ID NO: 6; SEQ ID NO: 7; SEQ ID NO: 8; SEQ ID NO: 9; SEQ ID NO: 10; SEQ ID NO: 11; SEQ ID NO: 12; SEQ ID NO: 13; SEQ ID NO: 14; SEQ ID NO: 15; SEQ ID NO: 16; SEQ ID NO: 17; SEQ ID NO: 18; SEQ ID NO: 19; SEQ ID NO: 20; SEQ ID NO: 21; SEQ ID NO: 22; SEQ ID NO: 23; SEQ ID NO: 24; SEQ ID NO: 25; SEQ ID NO: 26; SEQ ID NO: 27; SEQ ID NO: 28; SEQ ID NO: 29; SEQ ID NO: 30; SEQ ID NO: 31; SEQ ID NO: 32; SEQ ID NO: 33; SEQ ID NO: 34; SEQ ID NO: 35; SEQ ID NO: 36; SEQ ID NO: 37; SEQ ID NO: 38; SEQ ID NO: 39; SEQ ID NO: 40; SEQ ID NO: 41 ; SEQ ID NO: 42; SEQ ID NO: 43; SEQ ID NO: 44; SEQ ID NO: 45;<">SEQ ID NO: 46; SEQ ID NO: 47; SEQ ID NO: 48; SEQ ID NO: 49; SEQ ID NO: 50; SEQ ID NO: 51 ; SEQ ID NO: 52; SEQ ID NO: 53; SEQ ID NO: 54; SEQ ID NO: 55; SEQ ID NO: 56; SEQ ID NO: 57; SEQ ID NO: 58; SEQ ID NO: 59; SEQ ID NO: 60; SEQ ID NO: 61; SEQ ID NO: 62; SEQ ID NO: 63; SEQ ID NO: 64; SEQ ID NO: 65; SEQ ID NO: 66; SEQ ID NO: 67; SEQ ID NO: 68; SEQ ID NO: 69; SEQ ID NO: 70; SEQ ID NO: 71; SEQ ID NO: 72; SEQ ID NO: 73; SEQ ID NO: 74; SEQ ID NO: 75; SEQ ID NO: 76; SEQ ID NO: 77; SEQ ID NO: 78; SEQ ID NO: 79; SEQ ID NO: 80; SEQ ID NO: 81 ; SEQ ID NO: 82; SEQ ID NO: 83; SEQ ID NO: 84; SECT ID NO: 85; SEQ ID NO: 86; SEQ ID NO: 87; SEQ ID NO: 88; SEQ ID NO: 89; SEQ ID NO: 90; SEQ ID NO: 91; SEQ ID NO: 92; SEQ ID NO: 93; SEQ ID NO: 94; SEQ ID NO: 95; SEQ ID NO: 96; SEQ ID NO: 97; SEQ ID NO: 98; SEQ ID NO: 99; SEQ ID NO: 100; SEQ ID NO: 101 ; SEQ ID NO: 102; SEQ ID NO: 103; SEQ ID NO: 104; SEQ ID NO: 105; SEQ ID NO: 106; SEQ ID NO: 107; SEQ ID NO: 108; SEQ ID NO: 109; SEQ ID NO: 110; SEQ ID NO: 114; SEQ ID NO: 115; SEQ ID NO: 116; SEQ ID NO: 117; SEQ ID NO: 118; SEQ ID NO: 119; SEQ ID NO: 120; SEQ ID NO: 121 ; SEQ ID NO: 122; SEQ ID NO: 123; SEQ ID NO: 124; SEQ ID NO: 125; SEQ ID NO: 126; SEQ ID NO: 127; SEQ ID NO: 128; SEQ ID NO: 129; SEQ ID NO: 130; SEQ ID NO: 131 ; SEQ ID NO: 132; SEQ ID NO: 133; SEQ ID NO: 134; and SEQ ID NO: 135.
10. An expression cassette according to item 9, further comprising a regulatable or a constitutive promoter operably linked to said nucleic acid sequence.
11. A cell comprising the expression cassette of item 9.
12. A vector comprising the expression cassette of item 9.
13. A vector according to item 12, wherein said vector is a viral vector selected from the group consisting of an adenoviral virus vector, a retroviral virus vector, a parvoviral virus vector, a picornaviral virus vector, and a herpes virus vector.
14. A vector according to item 11, wherein said vector comprises a regulatable or a constitutive promoter operably linked to said nucleic acid sequence.
15. A kit for screening individuals for the heterozygous presence of one or more single nucleotide polymorphisms within said individual's Huntington's genes.
16. A kit according to item 15, wherein said kit comprises at least one molecular beacon that detects a single nucleotide polymorphism found in a sequence selected from the group consisting of SEQ ID NO: 1; SEQ ID NO: 2; SEQ ID NO: 3; SEQ ID NO: 4; SEQ ID NO: 5; SEQ ID NO: 6; SEQ ID NO: 7; SEQ ID NO: 8; SEQ ID NO: 9; SEQ ID NO: 10; SEQ ID NO: 11 ; SEQ ID NO: 12; SEQ ID NO: 13; SEQ ID NO: 14; SEQ ID NO: 15; SEQ ID NO: 16; SEQ ID NO: 17; SEQ ID NO: 18; SEQ ID NO: 19; SEQ ID NO: 20; SEQ ID NO: 21 ; and SEQ ID NO: 22 wherein said molecular beacon comprises at least a portion of the reverse complement of said sequence containing said detected single nucleotide polymorphism.
17. A kit according to item 15, wherein said kit further comprises nucleic acid molecules that preferentially suppress the expression of amino acid sequences encoding for mutated huntingtin ("htt") over suppressing the expression of amino acid sequences encoding for normal htt by targeting an area of a Huntington's disease gene that is heterozygous for the presence of one or more single nucleotide polymorphisms.
18. A kit according to item 17, wherein said nucleotide sequences are selected from the group consisting of SEQ ID NO: 1; SEQ ID NO: 2; SEQ ID NO: 3; SEQ ID NO: 4; SEQ ID NO: 5; SEQ ID NO: 6; SEQ ID NO: 7; SEQ ID NO: 8; SEQ ID NO: 9; SEQ ID NO: 10; SEQ ID NO: 11; SEQ ID NO: 12; SEQ ID NO: 13; SEQ ID NO: 14; SEQ ID NO: 15; SEQ ID NO: 16; SEQ ID NO: 17; SEQ ID NO: 18; SEQ ID NO: 19; SEQ ID NO: 20; SEQ ID NO: 21 ; SEQ ID NO: 22; SEQ ID NO: 23; SEQ ID NO: 24; SEQ ID NO: 25; SEQ ID NO: 26; SEQ ID NO: 27; SEQ ID NO: 28; SEQ ID NO: 29; SEQ ID NO: 30; SEQ ID NO: 31 ; SEQ ID NO: 32; SEQ ID NO: 33; SEQ ID NO: 34; SEQ ID NO: 35; SEQ ID NO: 36; SEQ ID NO: 37; SEQ ID NO: 38; SEQ ID NO: 39; SEQ ID NO: 40; SEQ ID NO: 41 ; SEQ ID NO: 42; SEQ ID NO: 43; SEQ ID NO: 44; SEQ ID NO: 45; SEQ ID NO: 46; SEQ ID NO: 47; SEQ ID NO: 48; SEQ ID NO: 49; SEQ ID NO: 50; SEQ ID NO: 51; SEQ ID NO: 52; SEQ ID NO: 53; SEQ ID NO: 54; SECT ID NO: 55; SEQ ID NO: 56; SEQ ID NO: 57; SEQ ID NO: 58; SEQ ID NO: 59; SEQ ID NO: 60; SEQ ID NO: 61 ; SEQ ID NO: 62; SEQ ID NO: 63; SEQ ID NO: 64; SEQ ID NO: 65; SEQ ID NO: 66; SEQ ID NO: 67; SEQ ID NO: 68; SEQ ID NO: 69; SEQ ID NO: 70; SEQ ID NO: 71 ; SEQ ID NO: 72; SEQ ID NO: 73; SEQ ID NO: 74; SEQ ID NO: 75; SEQ ID NO: 76; SEQ ID NO: 77; SEQ ID NO: 78; SEQ ID NO: 79; SEQ ID NO: 80; SEQ ID NO: 81 ; SEQ ID NO: 82; SEQ ID NO: 83; SEQ ID NO: 84; SEQ ID NO: 85; SEQ ID NO: 86; SEQ ID NO: 87; SEQ ID NO: 88; SEQ ID NO: 89; SEQ ID NO: 90; SEQ ID NO: 91; SEQ ID NO: 92; SEQ ID NO: 93; SEQ ID NO: 94; SEQ ID NO: 95; SEQ ID NO: 96; SEQ ID NO: 97; SEQ ID NO: 98; SEQ ID NO: 99; SEQ ID NO: 100; SEQ ID NO: 101 ; SEQ ID NO: 102; SEQ ID NO: 103; SEQ ID NO: 104; SEQ ID NO: 105; SEQ ID NO: 106; SEQ ID NO: 107; SEQ ID NO: 108; SEQ ID NO: 109; SEQ ID NO: 110; SEQ ID NO: 114; SEQ ID NO: 115; SEQ ID NO: 116; SEQ ID NO: 117; SEQ ID NO: 118; SEQ ID NO: 119; SEQ ID NO: 120; SEQ ID NO: 121; SEQ ID NO: 122; SEQ ID NO: 123; SEQ ID NO: 124; SEQ ID NO: 125; SEQ ID NO: 126; SEQ ID NO: 127; SEQ ID NO: 128; SEQ ID NO: 129; SEQ ID NO: 130; SEQ ID NO: 131 ; SEQ ID NO: 132; SEQ ID NO: 133; SEQ ID NO: 134; SEQ ID NO: 135; and combinations thereof.
19. A kit according to item 17, wherein said nucleotide sequences are selected from the group consisting of SEQ ID NO: 1; SEQ ID NO: 2; SEQ ID NO: 3; SEQ ID NO: 4; SEQ ID NO: 5; SEQ ID NO: 6; SEQ ID NO: 7; SEQ ID NO: 8; SEQ ID NO: 9; SEQ ID NO: 10; SEQ ID NO: 11 ; SEQ ID NO: 12; SEQ ID NO: 13; SEQ ID NO: 14; SEQ ID NO: 15; SEQ ID NO: 16; SEQ ID NO: 17; SEQ ID NO: 18; SEQ ID NO: 19; SEQ ID NO: 20; SEQ ID NO: 21 ; SEQ ID NO: 22; SEQ ID NO: 23; SEQ ID NO: 24; SEQ ID NO: 25; SEQ ID NO: 26; SEQ ID NO: 27; SEQ ID NO: 28; SEQ ID NO: 29; SEQ ID NO: 30; SEQ ID NO: 31 ; SEQ ID NO: 32; SEQ ID NO: 33; SEQ ID NO: 34; SEQ ID NO: 35; SEQ ID NO: 36; SEQ ID NO: 37; SEQ ID NO: 38; SEQ ID NO: 39; SEQ ID NO: 40; SEQ ID NO: 41 ; SEQ ID NO: 42; SEQ ID NO: 43; SEQ ID NO: 44; SEQ ID NO: 45; SEQ ID NO: 46; SEQ ID NO: 47; SEQ ID NO: 48; SEQ ID NO: 49; SEQ ID NO: 50; SEQ ID NO: 51; SEQ ID NO: 52; SEQ ID NO: 53; SEQ ID NO: 54; SEQ ID NO: 55; SEQ ID NO: 56; SEQ ID NO: 57; SEQ ID NO: 58; SEQ ID NO: 59; SEQ ID NO: 60; SEQ ID NO: 61; SEQ ID NO: 62; SEQ ID NO: 63; SEQ ID NO: 64; SEQ ID NO: 65; SEQ ID NO: 66; SEQ ID NO: 67; SEQ ID NO: 68; SEQ ID NO: 69; SEQ ID NO: 70; SEQ ID NO: 71 ; SEQ ID NO: 72; SEQ ID NO: 73; SEQ ID NO: 74; SEQ ID NO: 75; SEQ ID NO: 76; SEQ ID NO: 77; SEQ ID NO: 78; SEQ ID NO: 79; SEQ ID NO: 80; SEQ ID NO: 81 ; SEQ ID NO: 82; SEQ ID NO: 83; SEQ ID NO: 84; SEQ ID NO: 85; SEQ ID NO: 86; SEQ ID NO: 87; SEQ ID NO: 88; SEQ ID NO: 89; SEQ ID NO: 90; SEQ 10 NO: 91; SEQ ID NO: 92; SEQ ID NO: 93; SEQ ID NO: 94; SEQ ID NO: 95; SEQ ID NO: 96; SEQ ID NO: 97; SEQ ID NO: 98; SEQ ID NO: 99; SEQ ID NO: 100; SEQ ID NO: 101 ; SEQ ID NO: 102; SEQ ID NO: 103; SEQ ID NO: 104; SEQ ID NO: 105; SEQ ID NO: 106; SEQ ID NO: 107; SEQ ID NO: 108; SEQ ID NO: 109; SEQ ID NO: 110; SEQ ID NO: 114; SEQ ID NO: 115; SEQ ID NO: 116; SEQ ID NO: 117; SEQ ID NO: 118; SEQ ID NO: 119; SEQ ID NO: 120; SEQ ID NO: 121 ; SEQ ID NO: 122; SEQ ID NO: 123; SEQ ID NO: 124; SEQ ID NO: 125; SEQ ID NO: 126; SEQ ID NO: 127; SEQ ID NO: 128; SEQ ID NO: 129; SEQ ID NO: 130; SEQ ID NO: 131 ; SEQ ID NO: 132; SEQ ID NO: 133; SEQ ID NO: 134; SEQ ID NO: 135; and combinations thereof in shNA format.
20. A kit according to item 15, wherein said kit further comprises a vector comprising an expression cassette.
21. A kit according to item 20, wherein said vector is a viral vector selected from the group consisting of an adenoviral virus vector, a retroviral virus vector, a parvoviral virus vector, a picornaviral virus vector, and a herpes virus vector.

## Claims

1. A kit for screening individuals for the heterozygous presence of one or more single nucleotide polymorphisms within said individual's Huntington's genes, the kit comprising two pairs of molecular beacons selected from the group consisting of:
(a) a molecular beacon that detects a single polymorphism found in SEQ ID NO: 7, wherein said molecular beacon comprises at least a portion of a reverse complement of SEQ ID NO: 7 and a molecular beacon that detects a single polymorphism found in SEQ ID NO: 8, wherein said molecular beacon comprises at least a portion of a reverse complement of SEQ ID NO: 8;
(b) a molecular beacon that detects a single polymorphism found in SEQ ID NO: 15, wherein said molecular beacon comprises at least a portion of a reverse complement of SEQ ID NO: 15 and a molecular beacon that detects a single polymorphism found in SEQ ID NO: 16, wherein said molecular beacon comprises at least a portion of a reverse complement of SEQ ID NO: 16;
and optionally, including at least one pair of molecular beacons selected from the group (c) and (d), wherein (c) and (d) are as follows:
(c) a molecular beacon that detects a single polymorphism found in SEQ ID NO: 3, wherein said molecular beacon comprises at least a portion of a reverse complement of SEQ ID NO: 3; and a molecular beacon that detects a single polymorphism found in SEQ ID NO: 4, wherein said molecular beacon comprises at least a portion of a reverse complement of SEQ ID NO: 4;
(d) a molecular beacon that detects a single polymorphism found in SEQ ID NO: 13, wherein said molecular beacon comprises at least a portion of a reverse complement of SEQ ID NO: 13 and a molecular beacon that detects a single polymorphism found in SEQ ID NO: 14, wherein said molecular beacon comprises at least a portion of a reverse complement of SEQ ID NO: 14,
wherein the presence or the detection of the SNP allows for deciding on allele-specific Huntington's disease treatment of the individual.

2. The kit for screening according to claim 1, further comprising nucleic acid molecules that preferentially suppress the expression of amino acid sequences encoding for mutated huntingtin ("htt") over suppressing the expression of amino acid sequences encoding for normal htt by targeting an area of a Huntington's disease gene that is heterozygous for the presence of one or more single nucleotide polymorphisms found in SEQ ID NOs: 3, 4, 7, 8, 13, 14, 15 and 16.

3. *In vitro* use of the kit of any one of claims 1 or 2 for screening individuals for the heterozygous presence of one or more SNPs with the individual's Huntingtin genes, wherein said one more SNPs are selected from the group consisting of SNP4, and SNP8, which are **characterised by** SEQ ID NOs: 7, 8 and 15, 16, respectively.

4. The kit for screening according to claim 1 comprising:
(a) a molecular beacon that detects a single polymorphism found in SEQ ID NO: 3, wherein said molecular beacon comprises at least a portion of a reverse complement of SEQ ID NO: 3;
(b) a molecular beacon that detects a single polymorphism found in SEQ ID NO: 4, wherein said molecular beacon comprises at least a portion of a reverse complement of SEQ ID NO: 4;
(c) a molecular beacon that detects a single polymorphism found in SEQ ID NO: 7, wherein said molecular beacon comprises at least a portion of a reverse complement of SEQ ID NO: 7;
(d) a molecular beacon that detects a single polymorphism found in SEQ ID NO: 8, wherein said molecular beacon comprises at least a portion of a reverse complement of SEQ ID NO: 8;
(e) a molecular beacon that detects a single polymorphism found in SEQ ID NO: 13, wherein said molecular beacon comprises at least a portion of a reverse complement of SEQ ID NO: 13;
(f) a molecular beacon that detects a single polymorphism found in SEQ ID NO: 14, wherein said molecular beacon comprises at least a portion of a reverse complement of SEQ ID NO: 14;
(g) a molecular beacon that detects a single polymorphism found in SEQ ID NO: 15, wherein said molecular beacon comprises at least a portion of a reverse complement of SEQ ID NO: 15; and
(h) a molecular beacon that detects a single polymorphism found in SEQ ID NO: 16, wherein said molecular beacon comprises at least a portion of a reverse complement of SEQ ID NO: 16.

5. *In vitro* use of the kit of claim 1 for screening individuals for the heterozygous presence of one or more SNPs with the individual's Huntingtin genes, wherein said one or more SNPs are selected from SNP2, SNP4, SNP7 and SNP8, as **characterized by** present SEQ ID NOs: 3, 4, 7, 8, 13, 14, 15 and 16.

6. The kit of claim 1 for use in allele-specific treatment of individuals heterozygous at one or more SNPs with the individual's Huntingtin genes, wherein said one or more SNPs are selected from SNP2, SNP4, SNP7 and SNPB, as **characterized by** present SEQ ID NOs: 3, 4, 7, 8, 13, 14, 15 and 16.

## Patentansprüche

1. Kit zum Absuchen von Individuen auf das heterozygote Vorhandensein eines oder mehrerer einzelner Nukleotidpolymorphismen ("SNPs") innerhalb der Huntington-Gene des Individuums, wobei der Kit zwei Paare molekularer Leuchtsignale ("*Molecular Beacons*") umfasst, die ausgewählt sind aus der Gruppe bestehend aus:
(a) ein molekulares Leuchtsignal, das einen einzelnen Nukleotidpolymorphismus nachweist, der in SEQ ID NO: 7 gefunden wird, wobei das molekulare Leuchtsignal mindestens einen Teil eines reversen Komplements der SEQ ID NO: 7 umfasst, und ein molekulares Leuchtsignal, das einen einzelnen Nukleotidpolymorphismus nachweist, der in SEQ ID NO: 8 gefunden wird, wobei das molekulare Leuchtsignal mindestens einen Teil eines reversen Komplements der SEQ ID NO: 8 umfasst;
(b) ein molekulares Leuchtsignal, das einen einzelnen Nukleotidpolymorphismus nachweist, der in SEQ ID NO: 15 gefunden wird, wobei das molekulare Leuchtsignal mindestens einen Teil eines reversen Komplements der SEQ ID NO: 15 umfasst, und ein molekulares Leuchtsignal, das einen einzelnen Nukleotidpolymorphismus nachweist, der in SEQ ID NO: 16 gefunden wird, wobei das molekulare Leuchtsignal mindestens einen Teil eines reversen Komplements der SEQ ID NO: 16 umfasst;
und gegebenenfalls mindestens ein Paar molekularer Leuchtsignale einschließt, die ausgewählt sind aus der Gruppe bestehend aus der Gruppe (c) und (d), wobei (c) und (d) wie folgt sind:
(c) ein molekulares Leuchtsignal, das einen einzelnen Nukleotidpolymorphismus nachweist, der in SEQ ID NO: 3 gefunden wird, wobei das molekulare Leuchtsignal mindestens einen Teil eines reversen Komplements der SEQ ID NO: 3 umfasst; und ein molekulares Leuchtsignal, das einen einzelnen Nukleotidpolymorphismus nachweist, der in SEQ ID NO: 4, gefunden wird, wobei das molekulare Leuchtsignal mindestens einen Teil eines reversen Komplements der SEQ ID NO: 4 umfasst;
(d) ein molekulares Leuchtsignal, das einen einzelnen Nukleotidpolymorphismus nachweist, der in SEQ ID NO: 13 gefunden wird, wobei das molekulare Leuchtsignal mindestens einen Teil eines reversen Komplements der SEQ ID NO: 13 umfasst, und ein molekulares Leuchtsignal, das einen einzelnen Nukleotidpolymorphismus nachweist, der in SEQ ID NO: 14, gefunden wird, wobei das molekulare Leuchtsignal mindestens einen Teil eines reversen Komplements der SEQ ID NO: 14 umfasst,
wobei das Vorhandensein oder der Nachweis des SNPs eine Entscheidung über die Behandlung einer Allel-spezifischen Huntington Erkrankung eines Individuums erlaubt.

2. Kit zum Absuchen nach Anspruch 1, das weiterhin Nukleinsäuremoleküle umfasst, die bevorzugt die Expression einer Aminosäuresequenz supprimieren, die ein mutiertes Huntingtin ("htt") kodieren, über das Supprimieren der Expression von Aminosäuresequenzen hinaus, die normales htt kodieren, indem auf einen Bereich eines Gens der Huntingtonkrankheit gezielt wird, der heterozygot für das Vorhandensein eines oder mehrerer einzelner Nukleotidpolymorphismen ist, die in SEQ ID NR: 3, 4, 7, 8, 13, 14, 15 und 16 gefunden werden.

3. *In vitro* -Verwendung des Kits nach einem der Ansprüche 1 oder 2 zum Absuchen von Individuen auf das heterozygote Vorhandensein eines oder mehrerer SNPs mit den Huntingtin-Genen des Individuums, worin das eine oder die mehreren SNPs ausgewählt sind aus der Gruppe bestehend aus SNP4 und SNP8, die durch SEQ ID NR: 7, 8 beziehungsweise 15, 16 **gekennzeichnet** sind.

4. Kit zum Absuchen nach Anspruch 1 umfassend:
(a) a ein molekulares Leuchtsignal, das einen einzelnen Polymorphismus nachweist, der in SEQ ID NO: 3 gefunden wird, wobei das molekulare Leuchtsignal mindestens einen Teil eines reversen Komplements der SEQ ID NO: 3 umfasst;
(b) a ein molekulares Leuchtsignal, das einen einzelnen Polymorphismus nachweist, der in SEQ ID NO: 4 gefunden wird, wobei das molekulare Leuchtsignal mindestens einen Teil eines reversen Komplements der SEQ ID NO: 4 umfasst;
(c) a ein molekulares Leuchtsignal, das einen einzelnen Polymorphismus nachweist, der in SEQ ID NO: 7 gefunden wird, wobei das molekulare Leuchtsignal mindestens einen Teil eines reversen Komplements der SEQ ID NO: 7 umfasst;
(d) a ein molekulares Leuchtsignal, das einen einzelnen Polymorphismus nachweist, der in SEQ ID NO: 8 gefunden wird, wobei das molekulare Leuchtsignal mindestens einen Teil eines reversen Komplements der SEQ ID NO: 8 umfasst;
(e) a ein molekulares Leuchtsignal, das einen einzelnen Polymorphismus nachweist, der in SEQ ID NO: 13 gefunden wird, wobei das molekulare Leuchtsignal mindestens einen Teil eines reversen Komplements der SEQ ID NO: 13 umfasst;
(f) a ein molekulares Leuchtsignal, das einen einzelnen Polymorphismus nachweist, der in SEQ ID NO: 14 gefunden wird, wobei das molekulare Leuchtsignal mindestens einen Teil eines reversen Komplements der SEQ ID NO: 14 umfasst;
(g) a ein molekulares Leuchtsignal, das einen einzelnen Polymorphismus nachweist, der in SEQ ID NO: 15 gefunden wird, wobei das molekulare Leuchtsignal mindestens einen Teil eines reversen Komplements der SEQ ID NO: 15 umfasst und
(h) a ein molekulares Leuchtsignal, das einen einzelnen Polymorphismus nachweist, der in SEQ ID NO: 16 gefunden wird, wobei das molekulare Leuchtsignal mindestens einen Teil eines reversen Komplements der SEQ ID NO: 16 umfasst.

5. *In vitro* -Verwendung des Kits nach Anspruch 1 zum Absuchen von Individuen auf das heterozygote Vorhandensein eines oder mehrerer SNPs mit den Huntingtin-Genen des Individuums, worin das eine oder die mehreren SNPs ausgewählt sind aus SNP2, SNP4, SNP7 und SNP8 die durch SEQ ID NR: 3, 4, 7, 8, 13, 14, 15 und 16 **gekennzeichnet** sind.

6. Kit nach Anspruch 1 zur Verwendung in einer Allel-spezifischen Behandlung von Individuen, die heterozygot sind bei einem oder mehreren SNPs mit den Huntingtin-Genen des individuums, worin das eine oder die mehreren SNPs ausgewählt sind aus SNP2, SNP4, SNP7 und SNP8 die durch SEQ ID NR: 3, 4, 7, 8, 13, 14, 15 und 16 **gekennzeichnet** sind.

## Revendications

1. Kit de dépistage chez des individus de la présence hétérozygote d'un ou de plusieurs polymorphismes nucléotidiques simples au sein desdits gènes Huntington des individus, le kit comprenant deux paires de balises moléculaires choisies dans le groupe comprenant :
(a) une balise moléculaire qui détecte un polymorphisme simple trouvé dans SEQ ID NO : 7, ladite balise moléculaire comprenant au moins une partie d'un complément inversé de SEQ ID NO : 7 et une balise moléculaire qui détecte un polymorphisme simple trouvé dans SEQ ID NO : 8, ladite balise moléculaire comprenant au moins une partie d'un complément inversé de SEQ ID NO : 8 ;
(b) une balise moléculaire qui détecte un polymorphisme simple trouvé dans SEQ ID NO: 15, ladite balise moléculaire comprenant au moins une partie d'un complément inversé de SEQ ID NO : 15 et une balise moléculaire qui détecte un polymorphisme simple trouvé dans SEQ ID NO : 16, ladite balise moléculaire comprenant au moins une partie d'un complément inversé de SEQ ID NO : 16 ;
et comprenant facultativement au moins une paire de balises moléculaires choisies dans le groupe (c) et (d), (c) et (d) étant les suivantes :
(c) une balise moléculaire qui détecte un polymorphisme simple trouvé dans SEQ ID NO : 3, ladite balise moléculaire comprenant au moins une partie d'un complément inversé de SEQ ID NO : 3 et une balise moléculaire qui détecte un polymorphisme simple trouvé dans SEQ ID NO : 4, ladite balise moléculaire comprenant au moins une partie d'un complément inversé de SEQ ID NO : 4 ;
(d) une balise moléculaire qui détecte un polymorphisme simple trouvé dans SEQ ID NO: 13, ladite balise moléculaire comprenant au moins une partie d'un complément inversé de SEQ ID NO : 13 et une balise moléculaire qui détecte un polymorphisme simple trouvé dans SEQ ID NO : 14, ladite balise moléculaire comprenant au moins une partie d'un complément inversé de SEQ ID NO : 14 ;
dans lequel la présence ou la détection du SNP (polymorphisme nucléotidique simple) permet de prendre une décision sur le traitement de la maladie de Huntington spécifique d'un allèle de l'individu.

2. Kit de dépistage selon la revendication 1, comprenant en outre des molécules d'acides nucléiques qui suppriment préférentiellement l'expression de séquences d'acides aminés codant une huntingtine mutée (« htt ») par rapport à la suppression de l'expression de séquences d'acides aminés codant une htt normale en ciblant une zone d'un gène de la maladie de Huntington qui est hétérozygote pour la présence d'un ou de plusieurs polymorphismes nucléotidiques simples trouvés dans SEQ ID NO : 3, 4, 7, 8, 13, 14, 15 et 16.

3. Utilisation *in vitro* du kit selon la revendication 1 ou 2, pour le dépistage chez des individus de la présence hétérozygote d'un ou de plusieurs SNP avec les gènes Huntingtin des individus, dans laquelle le ou les SNP sont choisis dans le groupe constitué par SNP4 et SNP8, qui sont **caractérisées par** SEQ ID NO: 7, 8 et 15 et 16, respectivement.

4. Kit de dépistage selon la revendication 1, comprenant :
(a) une balise moléculaire qui détecte un polymorphisme simple dans SEQ ID NO: 3, ladite balise moléculaire comprenant au moins une partie d'un complément inversé de SEQ ID NO : 3 ;
(b) une balise moléculaire qui détecte un polymorphisme simple dans SEQ ID NO : 4, ladite balise moléculaire comprenant au moins une partie d'un complément inversé de SEQ ID NO : 4 ;
(c) une balise moléculaire qui détecte un polymorphisme simple dans SEQ ID NO : 7, ladite balise moléculaire comprenant au moins une partie d'un complément inversé de SEQ ID NO : 7 ;
(d) une balise moléculaire qui détecte un polymorphisme simple dans SEQ ID NO : 8, ladite balise moléculaire comprenant au moins une partie d'un complément inversé de SEQ ID NO : 8 ;
(e) une balise moléculaire qui détecte un polymorphisme simple dans SEQ ID NO: 13, ladite balise moléculaire comprenant au moins une partie d'un complément inversé de SEQ ID NO : 13 ;
(f) une balise moléculaire qui détecte un polymorphisme simple dans SEQ ID NO: 14, ladite balise moléculaire comprenant au moins une partie d'un complément inversé de SEQ ID NO : 14 ;
(g) une balise moléculaire qui détecte un polymorphisme simple dans SEQ ID NO: 15, ladite balise moléculaire comprenant au moins une partie d'un complément inversé de SEQ ID NO : 15 ; et
(h) une balise moléculaire qui détecte un polymorphisme simple dans SEQ ID NO: 16, ladite balise moléculaire comprenant au moins une partie d'un complément inversé de SEQ ID NO : 16.

5. Utilisation *in vitro* du kit selon la revendication 1, pour le dépistage chez des individus de la présence hétérozygote d'un ou de plusieurs SNP avec les gènes Huntingtin des individus, dans laquelle le ou les SNP sont choisis parmi SNP2, SNP4, SNP7 et SNP8, qui sont **caractérisées par** les présentes SEQ ID NO : 3, 4, 7, 8, 13, 14, 15 et 16.

6. Kit selon la revendication 1, utilisable pour le traitement spécifique d'un allèle d'individus hétérozygotes à un ou plusieurs SNP avec les gènes Huntingtin des individus, dans lequel le ou les SNP sont choisis parmi SNP2, SNP4, SNP7 et SNP8, qui sont **caractérisées par** les présentes SEQ ID NO : 3, 4, 7, 8, 13, 14, 15 et 16.
